(19) Europäisches Patentamt / European Patent Office / Office européen des brevets

(11) **EP 1 765 759 B1**

(12) **EUROPÄISCHE PATENTSCHRIFT**

(45) Veröffentlichungstag und Bekanntmachung des Hinweises auf die Patenterteilung:
**17.08.2011 Patentblatt 2011/33**

(21) Anmeldenummer: **05743080.3**

(22) Anmeldetag: **21.04.2005**

(51) Int Cl.:
***C07C 57/05*** *(2006.01)*

(86) Internationale Anmeldenummer:
**PCT/EP2005/004282**

(87) Internationale Veröffentlichungsnummer:
**WO 2006/002703 (12.01.2006 Gazette 2006/02)**

(54) **VERFAHREN ZUR HERSTELLUNG VON ACRYLSÄURE DURCH HETEROGEN KATALYSIERTE PARTIELLE GASPHASEN OXIDATION VON PROPYLEN**

METHOD FOR THE PRODUCTION OF ACRYLIC ACID BY MEANS OF HETEROGENEOUSLY CATALYZED PARTIAL GAS PHASE OXIDATION OF PROPYLENE

PROCEDE DE PRODUCTION D'ACIDE ACRYLIQUE PAR OXYDATION PARTIELLE EN PHASE GAZEUSE A CATALYSE HETEROGENE DE PROPYLENE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **01.07.2004 US 584469 P
01.07.2004 DE 102004032129
01.03.2005 US 656875 P
01.03.2005 DE 102005009891
02.03.2005 US 657407 P
02.03.2005 DE 102005010111**

(43) Veröffentlichungstag der Anmeldung:
**28.03.2007 Patentblatt 2007/13**

(73) Patentinhaber: **BASF SE
67056 Ludwigshafen (DE)**

(72) Erfinder:
• DIETERLE, Martin
  **68167 Mannheim (DE)**
• MÜLLER-ENGEL, Klaus, Joachim
  **76297 Stutensee (DE)**
• HECHLER, Claus
  **67063 Ludwigshafen (DE)**
• HAMMON, Ulrich
  **68163 Mannheim (DE)**
• DIEFENBACHER, Armin
  **76726 Germersheim (DE)**

(56) Entgegenhaltungen:
**DE-A1- 10 313 209**

## Beschreibung

Vorliegende Erfindung betrifft ein Verfahren zur Herstellung von Acrylsäure durch heterogen katalysierte partielle Gasphasenoxidation von Propylen, bei dem man

a) in einer ersten Reaktionsstufe Propan im Beisein und/oder unter Ausschluß von Sauerstoff einer homogenen und/oder einer heterogen katalysierten Dehydrierung und/oder Oxidehydrierung unterwirft, wobei ein Propan und Propylen enthaltendes Produktgasgemisch 1 erhalten wird, und

b) aus in der ersten Reaktionsstufe gebildetem Produktgasgemisch 1, von den darin enthaltenen, von Propan und Propylen verschiedenen Bestandteilen gegebenenfalls eine Teilmenge abtrennt und/oder in andere Verbindungen wandelt, wobei aus dem Produktgasgemisch 1 ein Produktgasgemisch 1' erzeugt wird, und

c) Produktgasgemisch 1 und/öder Produktgasgemisch 1' als Bestandteil eines Reaktionsgasausgangsgemischs 2, das molekularen Sauerstoff und Propylen in einem molaren Verhältnis $O_2:C_3H_6 \geq 1$ enthält, in einer mit einem Katalysatorfestbett 2, dessen Katalysatoren als Aktivmasse wenigstens ein die Elemente Mo, Fe und Bi enthaltendes Multimetalloxid aufweisen, beschickten zweiten Reaktionsstufe einer heterogen katalysierten partiellen Gasphasenoxidation von im Produktgasgemisch 1 und/oder Produktgasgemisch 1' enthaltenem Propylen zu Acrolein unterwirft, wobei ein Produktgasgemisch 2 erhalten wird, und

d) die Temperatur des die zweite Reaktionsstufe verlassenden Produktgasgemischs 2 durch indirekte und/oder direkte Kühlung gegebenenfalls verringert und dem Produktgasgemisch 2 gegebenenfalls molekularen Sauerstoff und/oder Inertgas zugibt, und

e) danach als Acrolein, molekularen Sauerstoff und wenigstens ein Inertgas enthaltendes Reaktionsgasausgangsgemisch 3, das molekularen Sauerstoff und Acrolein in einem molaren Verhältnis $O_2:C_3H_4O \geq 0{,}5$ enthält, in einer mit einem Katalysatorfestbett 3, dessen Katalysatoren als Aktivmasse wenigstens ein die Elemente Mo und V enthaltendes Multimetalloxid aufweisen, beschickten dritten Reaktionsstufe einer heterogen katalysierten partiellen Gasphasenoxidation von im Reaktionsgasausgangsgemisch 3 enthaltenem Acrolein zu Acrylsäure unterwirft, wobei ein Produktgasgemisch 3 erhalten wird, und

f) aus dem Produktgasgemisch 3 in einer Trennzone A Acrylsäure abtrennt und wenigstens das im Produktgasgemisch 3 enthaltene nicht umgesetzte Propan und Propylen zu jeweils wenigstens 80 mol-%, bezogen auf die im Produktgasgemisch 3 enthaltene jeweilige Menge, in wenigstens die erste der drei Reaktionsstufen zurückführt.

Acrylsäure ist ein bedeutendes Monomeres, das als solches oder in Form seiner Alkylester zur Erzeugung von z. B. als Klebstoffen geeigneten Polymerisaten Verwendung findet.

Es ist bekannt, Acrylsäure durch zweistufige heterogen katalysierte partielle Gasphasenoxidation von Propylen herzustellen (vgl. z.B. EP-A 990 636, US-A 5,198,578, EP-A 10 15 410, EP-A 14 84 303, EP-A 14 84 308, EP-A 14 84 309 und US-A 2004/0242826).

Charakteristisch an den vorgenannten Verfahren ist, dass die Acrylsäure dabei nicht als solche, sondern als Bestandteil eines Produktgasgemisches erhalten wird, aus welchem sie nachfolgend abgetrennt werden muss.

Im wesentlichen allen diesbezüglich bekannten Trennverfahren ist gemein, dass, gegebenenfalls nach direktem und/oder indirektem Abkühlen des vorgenannten Produktgasgemischs, im Produktgasgemisch enthaltene Acrylsäure in einem Grundabtrennschritt in die kondensierte Phase überführt wird. Dies kann z.B. durch Absorption in ein geeignetes Lösungsmittel (z.B. Wasser, hochsiedende organische Lösungsmittel, wässrige Lösungen) und/oder durch partielle oder im wesentlichen vollständige Kondensation (z.B. durch fraktionierende Kondensation) erfolgen (vgl. dazu z.B. die oben genannten Schriften, sowie die Schriften EP-A 13 88 533, EP-A 13 88 532, DE-A 102 35 847, EP-A 79 28 67, WO 98/01415, EP-A 10 15 411, EP-A 10 15 410, WO 99/50219, WO 00/53560, WO 02/09839, DE-A 102 35 847, WO 03/041833, DE-A 102 23 058, DE-A 102 43 625, DE-A 103 36 386, EP-A 85 41 29, US-A 4,317,926, DE-A 198 37 520, DE-A 196 06 877, DE-A 190 50 1325, DE-A 102 47 240, DE-A 197 40 253, EP-A 69 57 36, EP-A 98 22 87, EP-A 10 41 062, EP-A 11 71 46, DE-A 43 08 087, DE-A 43 35 172, DE-A 44 36 243, DE-A 19 924 532, DE-A 103 32 758 sowie DE-A 19 924 533). Eine Acrylsäureabtrennung kann auch wie in der EP-A 98 22 87, der EP-A 98 22 89, der DE-A 103 36 386, der DE-A 101 15 277, der DE-A 196 06 877, der DE-A 197 40 252, der DE-A 196 27 847, der EP-A 92 04 08, der EP-A 10 68 174, der EP-A 10 66 239, der EP-A 10 66 240, der WO 00/53560, der WO 00/53561, der DE-A 100 53 086 und der EP-A 98 22 88 beschrieben vorgenommen werden. Günstige Abtrennweisen sind auch die in den Schriften WO 2004/063138, WO 2004/035514, DE-A 102 43 625 und DE-A 102 35 847 beschriebenen Verfahren.

Die Weiterreinigung der im Rahmen der beschriebenen Grundabtrennung anfallenden, Acrylsäure enthaltenden kondensierten Phase, kann dann z. B. extraktiv, rektifikativ, desorptiv und/oder kristallisativ bis zum gewünschten Reinheitsgrad der Acrylsäure erfolgen. Beispielsweise kann die Weiterverarbeitung wie in den Schriften WO 01/77056, WO 03/041832, WO 02/055469, WO 03/078378 und WO 03/041833 beschrieben erfolgen.

Gemeinsames Merkmal der beschriebenen Grundabtrennungen ist, dass normalerweise ein Restgasstrom (vgl. auch EP-A 11 80 508) verbleibt (in typischer Weise am Kopf der für die Grundabtrennung verwendeten trennwirksame Einbauten enthaltenden Trennkolonne), der im wesentlichen diejenigen Bestandteile des Produktgasgemischs enthält, deren Siedepunkt bei Normaldruck (1 bar) = -30˚C beträgt (d. h., die schwer kondensierbaren oder auch leicht flüchtigen Bestandteile). Die Restgasbestandteile sind in erster Linie bei der Partialoxidation nicht verbrauchte Reaktanden, d. h. molekularer Sauerstoff (wird in der Regel relativ zur Stöchiometrie der Partialoxidation im Überschuss eingesetzt, um die Katalysatoraktivität vorteilhaft zu gestalten) sowie gegebenenfalls Propylen, und vor allem in der Partialoxidation mitverwendete inerte Verdünnungsgase wie z. B. Stickstoff, Edelgase, Kohlenoxide und gesättigte Kohlenwasserstoffe. Wasserdampf kann je nach angewandtem Trennverfahren im Restgas nur noch in Spuren oder in Mengen von bis zu 20 Vol.-%, oder bis zu 25 Vol.-% oder mehr enthalten sein. In Geringmengen kann das Restgas auch noch Acrylsäure und/oder Acrolein enthalten, die normalerweise jedoch überwiegend wie beschrieben grundabgetrennt werden.

Die DE 10313209 A1 betrifft eine partielle Gasphasenoxidation von Propylen zu Acrylsäure ohne vorgeschaltete Propandehydrierung.

Nachteilig am verbliebenen Restgas ist, dass es nur in begrenzter Menge in die Partialoxidation rückgeführt werden kann. Eine weitergehende Rückführung ist deshalb nicht möglich, weil sich ansonsten im Rahmen einer solchen Kreisgasführung die Kreisgasbestandteile aufpegeln würden. Dies hätte nicht mehr bewältigbare Reaktionsgasströme in der Partialoxidation und schließlich deren Erlöschen zur Folge. In der Regel wird daher allenfalls die Hälfte des Restgases als Oxidationskreisgas in die Partialoxidation rückgeführt und die verbleibende Menge als Auslassgas der Verbrennung zugeführt (vgl. z. B. EP-A 925 272).

Vorgenanntes ist unter anderem deshalb von Nachteil, weil somit In der Partialoxidation nicht umgesetztes, im Restgas verbliebenes Propylen in notwendiger Weise verloren geht (eine Propylenabtrennung vom Restgas und nachfolgende separate Rückführung des abgetrennten Propylen in die Partialoxidation ist infolge der geringen Mengenanteile des Propylens wenig wirtschaftlich).

Die Nachteilhaftigkeit ist dabei vor allem deshalb so groß, weil als Ausgangspropylen für die Acrylsäureherstellung normalerweise ein eine vergleichsweise hohe Reinheit aufweisendes Propylen (z. B. "polymer grade" oder "chemical grade" Propylen; vgl. DE-A 101 31 297) verwendet wird, das normalerweise eine Trennung von den herstellungsbedingt begleitenden anderen Kohlenwasserstoffen wie z. B. Propan durchlaufen hat.

Unter anderem deshalb werden daher möglichst hohe Propylenumsätze in der ersten Reaktionsstufe angestrebt (vgl. WO 03/029177). Das gleiche gilt auch für den Acroleinumsatz in der zweiten Reaktionsstufe. Dies zusätzlich auch deshalb, weil sich in der zweiten Reaktionsstufe nicht umgesetztes Acrolein z. B. im Rahmen der Abtrennung der Acrylsäure aus dem Produktgasgemisch der Partialoxidation nachteilig bemerkbar macht (fördert in unerwünschter Weise die Polymerisationsneigung von Acrylsäure (vgl. z. B. DE-A 10 2004 021 764 und DE-A 10 2004 021 706)). Um vorgenannte hohe Umsätze zu erreichen, wird im Stand der Technik sogar die Anwendung von Nachreaktoren empfohlen (vgl. z. B. DE-A 10 2004 021 764 und DE-A 10 2004 021 706).

Vor allem im großtechnischen Betrieb der zweistufigen Partialoxidation von Propylen zu Acrylsäure werden vielfach in der ersten Reaktionsstufe Propylenumsätze von > 99,5 mol.-% angestrebt, um auf eine Oxidationskreisgasrückführung in die Partialoxidation völlig verzichten zu können. Eine solche Fahrweise beinhaltet zusätzlich den Vorteil, dass der für die Oxidationskreisgasrückführung erforderliche Energieaufwand (das Oxidationskreisgas muss vor der Rückführung mittels eines Turboverdichters auf Partialoxidationsdruck rückverdichtet werden) und das nicht unerhebliche Investment in den Verdichter (z. B. einen solchen vom Typ 12 MH4B der Fa. Mannesmann DEMAG, DE). entfällt.

Hohe Umsätze erfordern üblicherweise jedoch hinsichtlich ihrer Aktivität hochgeziichtete Katalysatoren und/oder hohe Temperaturen bei der Partialoxidation. Beides wirkt sich auf die Katalysatorlebensdauer nachteilig aus (vgl. DE-A 103 51 269 und DE-A 10 2004 025 445). Dies vor allem deshalb, weil das Reaktionsgasgemisch in jeder der beiden Partialoxidationsstufen beim Durchströmen durch das Katalysatorfestbett einen Höchstwert, den sogenannten Heißpunktwert durchläuft.

Aus Gründen der Zweckmäßigkeit werden daher die Temperatur des Katalysatorfestbetts und die effektive Temperatur des Katalysatorfestbetts voneinander unterschieden. Dabei wird unter der Temperatur des Katalysatorfestbetts die Temperatur des Katalysatorfestbetts bei Ausübung des Partialoxidationsverfahrens, jedoch in fiktiver Abwesenheit einer chemischen Reaktion (d. h., ohne den Einfluss der Reaktionswärme) verstanden. Unter effektiver Temperatur des Katalysatorfestbetts wird dagegen die tatsächliche Temperatur des Katalysatorfestbetts unter Einbezug der Reaktionswärme der Partialoxidation verstanden. Ist die Temperatur des Katalysatorfestbetts längs des Katalysatorfestbetts nicht konstant (z. B. im Fall von mehreren Temperaturzonen), so meint der Begriff Temperatur des Katalysatorfestbetts den (Zahlen) Mittelwert der Temperatur entlang des Katalysatorfestbetts. Selbstverständlich kann die Temperatur des Katalysatorfestbetts über seine Länge auch so gestaltet sein, dass die Temperatur über einen gewissen Längenabschnitt konstant ist, sich dann sprunghaft ändert und über einen weiteren Längenabschnitt auf diesem neuen Wert verharrt u.s.w.. Man spricht dann von einem mehr als eine Temperaturzone (oder auch Reaktionszone) aufweisenden oder sich in mehr als einer Temperaturzone (oder auch Reaktionszone) befindlichen Katalysatorfestbett (Festbettkatalysatorschüttung). Derartige Temperaturzonen (oder auch Reaktionszonen) realisierende, mit Katalysator beschickte Reaktoren werden in entsprechender Weise als "Ein-" oder "Mehr-Zonen-Reaktoren" bezeichnet (vgl. z. B. WO 04/085369). Mit der Temperatur des Reaktionsgasgemischs durchläuft die effektive Temperatur des Katalysatorfestbetts in Strömungsrichtung des Reaktionsgasgemischs gleichfalls den Heißpunktwert.

Von der Möglichkeit, die Heißpunkttemperatur dadurch zu mindern, dass die Partialoxidationsverfahren bei vermindertem Reaktandenumsatz betrieben werden, kann bei der konventionellen, vorstehend beschriebenen, Propylenpartialoxidation zu Acrylsäure aus den vorstehend beschriebenen Gründen allenfalls mit erheblichen Nachteilen Gebrauch gemacht werden.

Die Aufgabe der vorliegenden Erfindung bestand daher darin, ein Verfahren zur Herstellung von Acrylsäure durch heterogen katalysierte partielle Gasphasenoxidation von Propylen zur Verfügung zu stellen, das die beschriebenen Nachteile wenigstens in der ersten Reaktionsstufe (Propylen -> Acrolein) höchstens noch in verminderter Form aufweist.

Demgemäß wurde ein Verfahren zur Herstellung von Acrylsäure durch heterogen katalysierte partielle Gasphasenoxidation von Propylen, bei dem man

a) in einer ersten Reaktionsstufe Propan im Beisein und/oder unter Ausschluß von Sauerstoff einer homogenen und/oder einer heterogen katalysierten Dehydrierung und/oder Oxidehydrierung unterwirft, wobei ein Propan und Propylen enthaltendes Produktgasgemisch 1 erhalten wird, und

b) aus in der ersten Reaktionsstufe gebildetem Produktgasgemisch 1, von den darin enthaltenen, von Propan und Propylen verschiedenen Bestandteilen gegebenenfalls eine Teilmenge abtrennt und/oder in andere Verbindungen wandelt, wobei aus dem Produktgasgemisch 1 ein Produktgasgemisch 1' erzeugt wird, und

c) Produktgasgemisch 1 und/oder Produktgasgemisch 1' als Bestandteil eines Reaktionsgasausgangsgemischs 2, das molekularen Sauerstoff und Propylen in einem molaren Verhältnis $O_2:C_3H_6 \geq 1$ enthält, in einer mit einem Katalysatorfestbett 2, dessen Katalysatoren als Aktivmasse wenigstens ein die Elemente Mo, Fe und Bi enthaltendes Multimetalloxid aufweisen, beschickten zweiten Reaktionsstufe einer heterogen katalysierten partiellen Gasphasenoxidation von im Produktgasgemisch 1 und/oder Produktgasgemisch 1' enthaltenem Propylen zu Acrolein unterwirft, wobei ein Produktgasgemisch 2 erhalten wird, und

d) die Temperatur des die zweite Reaktionsstufe verlassenden Produktgasgemischs 2 durch indirekte und/oder direkte Kühlung gegebenenfalls verringert und dem Produktgasgemisch 2 gegebenenfalls molekularen Sauerstoff und/oder Inertgas zugibt, und

e) danach als Acrolein, molekularen Sauerstoff und wenigstens ein Inertgas enthaltendes Reaktionsgasausgangsgemisch 3, das molekularen Sauerstoff und Acrolein in einem molaren Verhältnis $O_Z:C_3H_4O \geq 0,5$ enthält, in einer mit einem Katalysatorfestbett 3, dessen Katalysatoren als Aktivmasse wenigstens ein die Elemente Mo und V enthaltendes Multimetalloxid aufweisen, beschickten dritten Reaktionsstufe einer heterogen katalysierten partiellen Gasphasenoxidation von im Reaktionsgasausgangsgemisch 3 enthaltenem Acrolein zu Acrylsäure unterwirft, wobei ein Produktgasgemisch 3 erhalten wird, und

f) aus dem Produktgasgemisch 3 in einer Trennzone A Acrylsäure abtrennt und wenigstens das im Produktgasgemisch 3 enthaltene nicht umgesetzte Propan und Propylen zu jeweils wenigstens 80 mol-%, bezogen auf die im Produktgasgemisch 3 enthaltene jeweilige Menge, wenigstens in die erste der drei Reaktionsstufen zurückführt, gefunden, das dadurch gekennzeichnet ist, dass

- der Umsatz $U^P$ des Propylens in der zweiten Reaktionsstufe, bezogen auf einmaligen Durchgang durch selbige, bei Werten $\leq 99,5.$ mol-%, und
- der Umsatz des Acroleins $U^A$ in der dritten Reaktionsstufe, bezogen auf einmaligen Durchgang durch selbige, bei Werten $\geq 96$ mol-% gehalten wird, und das Verfahren wenigstens einen, vorzugsweise separaten, Auslass für von Propan und Propen verschiedene Bestandteile aufweist. Separat heißt hier, dass der wenigstens eine Auslaß vorzugsweise ohne Begleitung von Propan und Propen erfolgt. D.h., der Gehalt von Propan und Propen im wenigstens einen Auslassgas liegt vorzugsweise bei Werten $\leq 5$, besser $\leq 1$ Vol.-%, bzw. besser bei $\leq 0,5$ Vol.-%, oder $\leq 0,25$ Vol.-%, oder $\leq 0,1$ Vol.-%.

Zusätzlich zu den bereits genannten Vorteilen des erfindungsgemäßen Verfahrens ist anzuführen, dass mit einem geringeren $U^P$ eine erhöhte Selektivität der Acrylsäurebildung einhergeht. Darüber hinaus fällt die für das Katalysatorfestbett 2 erforderlich Katalysatormenge mit abnehmenden $U^P$ überproportional, da der mit zunehmendem $U^P$ einhergehende Katalysatormengenbedarf exponentiell zunimmt.

Die Grundidee des erfindungsgemäßen Verfahrens, als Propylenquelle für die Acrylsäureherstellung eine partiellle Propandehydrierung und/oder eine Propanoxidehydrierung zu verwenden und im Gesamtverfahren nicht umgesetztes Propan und Propylen wenigstens teilweise in die Propylenquelle rückzuführen, ist bekannt (vgl. z. B. DE-A 102 45 585, WO 01 /96270, DE-A 10 2004 032 129, WO 03/076370, WO 01/96271, EP-A 117 146, WO 03/0118804, US-A 3,161,670, DE-A 33 13 573, WO 04/031106, DE-A 103 16 039, DE-A 195 08 558, WO 03/11804, DE-A 198 37 520, DE-A 198 37 519, DE-A 198 37 517, WO 97/36849 und EP-A 1 106 598 und EP-A 274 681).

Das sich durch den Rohstoffwechsel zum wesentlich kostengünstigeren Propan und die damit einhergehende Möglichkeit wenigstens eines, vorzugsweise separaten, wirtschaftlichen Auslass für von Propan (und Propylen) verschiedene Bestandteile, bei gleichzeitig möglichst quantitativer Rückführung von nicht umgesetztem Propan aus der Partialoxidation in die Propylenquelle, eröffnende Potential eines verminderten Propylenumsatzes in der ersten Reaktionsstufe, begleitet von einer nachfolgend ohne Mehraufwand mit Propan im Verbund erfolgenden möglichst umfassenden Propylenrückführung ins Gesamtverfahren, wurde bisher jedoch noch nicht gesehen.

Erfindungsgemäß ist es daher vorteilhaft, das erfindungsgemäße Verfahren so durchzuführen, dass

|  | |
|---|---|
|  | $U^P \leq 99{,}0$ mol-% und $U^A \geq 96$ mol-%, oder |
| besser | $U^P \leq 98{,}5$ mol-% und $U^A \geq 96$ mol-%, oder |
| besser | $U^P \leq 98{,}0$ mol-% und $U^A \geq 96$ mol-%, oder |
| besser | $U^P \leq 97{,}5$ mol-% und $U^A \geq 96$ mol-%, oder |
| besser | $U^P \leq 97{,}0$ mol-% und $U^A \geq 96$ mol-%, oder |
| besser | $U^P \leq 96{,}5$ mol-% und $U^A \geq 96$ mol-%, oder |
| besser | $U^P \leq 96{,}0$ mol-% und $U^A \geq 96$ mol-%, oder |
| besser | $U^P \leq 95{,}5$ mol-% und $U^A \geq 96$ mol-%, oder |
| besser | $U^P \leq 95{,}0$ mol-% und $U^A \geq 96$ mol-%, oder |
| besser | $U^P \leq 94{,}5$ mol% und $U^A \geq 96$ mol-%, oder |
| besser | $U^P \leq 94{,}0$ mol-% und $U^A \geq 96$ mol-%, oder |
| besser | $U^P \leq 93{,}5$ mol-% und $U^A \geq 96$ mol-%, oder |
| besser | $U^P \leq 93{,}0$ mol-% und $U^A \geq 96$ mol-%, oder |
| besser | $U^P \leq 92{,}0$ mol-% und $U^A \geq 96$ mol-%, oder |
| besser | $U^P \leq 91{,}0$ mol-% und $U^A \geq 96$ mol-%, oder |
| besser | $U^P \leq 90{,}0$ mol-% und $U^A \geq 96$ mol-%, oder |
| besser | $U^P \leq 85{,}0$ mol-% und $U^A \geq 96$ mol-%, oder |
| besser | $U^P \leq 80{,}0$ mol-% und $U^A \geq 96$ mol-%, oder |
| besser | $U^P \leq 75{,}0$ mol-% und $U^A \geq 96$ mol-%. |

In der Regel wird $U^P$ in allen vorgenannten Paarungen $\geq 50$ mol-%, bevorzugt $\geq 60$ mol-%, vorteilhaft $\geq 70$ mol-% betragen.

Selbstverständlich kann $U^P$ auch $\geq 75$ mol-%, oder $\geq 80$ mol-%, oder $\geq 85$ mol-%, oder $\geq 90$ mol-% betragen. D. h., möglicher erfindungsgemäßer $U^P$ Bereich ist jede mögliche Kombination einer vorstehend genannten $U^P$-Untergrenze mit einer vorstehend genannten $U^P$-Obergrenze.

Weiterhin ist es erfindungsgemäß von Vorteil, wenn in jeder der vorgenannten $U^P$, $U^A$-Paarungen $U^A \geq 96{,}5$ mol-%, besser $\geq 97$ mol-%, besser $\geq 97{,}5$ mol-%, besser $\geq 98$ mol-%, besser $\geq 98{,}5$-mol-%; besser $\geq 99$ mol-%, besser $\geq 99{,}5$ moi-%, besser $\geq 99{,}6$ mol-%, besser $\geq 99{,}7$ mol-%, besser $\geq 99{,}8$ mol-%, besser $\geq 99{,}9$ mol-%, besser $\geq 99{,}91$ mol-%, besser $\geq 99{,}92$ mol-%, besser $\geq 99{,}93$ mol-%, besser $\geq 99{,}94$ mol-%, besser $\geq 99{,}95$ mol-%, besser $\geq 99{,}96$ mol-%, besser $\geq 99{,}97$ mol-%, besser $\geq 99{,}98$ mol-%, besser $\geq 99{,}99$ mol-% beträgt.

Vorgenannte Höchstwerte sind z: B. dann erreichbar, wenn die dritte Reaktionsstufe wenigstens einen Nachreaktor gemäß der DE-A 10 2004 021 764 bzw. DE-A 10 2004 021 7063 umfasst. Alternativ können Verfahrensvarianten gemäß dem in diesen Schriften genannten Stand der Technik angewendet werden.

In der Regel wird $U^A < 100$ moI%, bzw. nicht größer als 99,995 mol% betragen. Vielfach wird $U^A \leq 99{,}99$, oder $\leq 99{,}98$, oder $\leq 99{,}96$, oder $\leq 99{,}95$ mol-% betragen. D. h., möglicher erfindungsgemäßer $U^A$ Bereich ist jede mögliche Kombination einer vorstehend genannten $U^A$-Untergrenze mit einer vorstehend genannten $U^A$-Obergrenze. Da $U^P$ und $U^A$ beim erfindungsgemäßen Verfahren voneinander unabhängig eingestellt werden können, können auch die Bereiche für $U^P$ mit den Bereichen für $U^A$ erfindungsgemäß frei kombiniert werden. Vorteilhaft wird ein bevorzugter Bereich für $U^P$ mit einem bevorzugten Bereich für $U^A$ kombiniert.

Erfindungsgemäß besonders vorteilhaft ist ein $U^P$ von 80 bis 98 mol-%, bei einem gleichzeitigen $U^A$ von 99,0 bis 99,9 mol-%. Eine andere erfindungsgemäß bevorzugte Kombination ist ein $U^P$ von 90 bis 98 mol-%, bei einem gleichzeitigen $U^A$ von 99,0 bis 99,9 mol-% oder mehr, bzw. von 99,3 bis 99,6 mol-%.

Ferner ist es für das erfindungsgemäße Verfahren unabhängig von $U^A$ und $U^P$ günstig, wenn nach der Abtrennung der Acrylsäure aus dem Produktgasgemisch 3 in der Trennzone A, das im Produktgasgemisch 3 enthaltene nicht umgesetzte Propan und Propylen zu jeweils wenigstens 85 mol-%, besser zu jeweils wenigstens 90 mol-%, besser zu jeweils wenigstens 92 mol-%, besser zu jeweils wenigstens 94 mol-%, besser zu jeweils wenigstens 95 mol-%, besser zu jeweils wenigstens 95,5 mol-%, besser zu jeweils wenigstens 96 mol-%, besser zu jeweils wenigstens 96,5 mol-%, besser zu jeweils wenigstens 97 mol-%, besser zu jeweils wenigstens 97,5 mol-%, besser zu jeweils wenigstens 98 mol-%, besser zu jeweils wenigstens 98,5 mol-%, besser zu jeweils wenigstens 99 mol-%, besser zu jeweils wenigstens 99,5 mol-%, besser zu jeweils wenigstens 99,75 oder wenigstens 99,9 mol-% und am besten quantitativ (jeweils bezogen auf die im Produktgasgemisch 3 enthaltene jeweilige Menge an Propan bzw. Propylen) wenigstens in die erste der drei Reaktionsstufen rückgeführt wird. Erfindungsgemäß vorteilhaft erfolgt die vorgenannte Propan- und Propenrückführung ausschließlich in die erste Reaktionsstufe. Sie kann jedoch auch teilweise (z. B. zu bis zu 50 Gew.-%, oder zu bis zu 30 Gew.-%, oder zu bis zu 20 Gew.-%, oder zu bis zu 10 Gew.-%, oder zu bis zu 5 Gew.-% oder weniger) in die zweite und/oder dritte Reaktionsstufe hinein erfolgten.

Im Fall der zweiten Reaktionsstufe kann die Zugabestelle dabei in eine zwischen der ersten und der zweiten Reaktionsstufe befindliche $C_3$-Abtrennung hinein erfolgen (z.B. als Stripgas).

Erfindungsgemäß zweckmäßig wird man die vorgenannte Rückführung von Propan und Propylen im Verbund, d.h., im selben Rückführgas befindlich, durchführen. Im folgenden werden solche Rückführgase als $C_3$-Kreisgase bezeichnet.

Im einfachsten Fall kann es sich bei $C_3$-Kreisgas um Oxidationskreisgas handeln. D. h., in der Trennzone A wird man die im Produktgasgemisch 3 enthaltene Acrylsäure als Zielprodukt z. B. durch absorptive und/oder kondensative Maßnahmen aus der gasförmigen in die kondensierte Phase überführen. Als Absorptionsmittel kommen dabei z. B. Wasser und/oder organische Lösungsmittel (insbesondere unter Normalbedingungen (25°C, 1 bar) höher als Acrylsäure siedende hydrophobe organische Lösungsmittel) in Betracht (prinzipiell kann die Abtrennung (Kondensation) wie in der EP-A 117146, der DE-A 4308087, der DE-A 4335172, der DE-A 4436243, der DE-A 19924533, der EP-A 982287, der EP-A 982289, der DE-A 19924532, der DE-A 10115277, der DE-A 19606877, der DE-A 19740252, der DE-A 19627847, der DE-A 10053086 und der EP-A 982288 beschrieben vorgenommen werden; vorzugsweise wird wie in Figur 7 der WO 0196271 sowie wie in der DE-A 102004032129 und deren Äquivalente beschrieben abgetrennt). Im Rahmen dieser "Kondensation" der Acrylsäure verbleibt, wie bereits eingangs beschrieben, normalerweise ein nicht in die kondensierte Phase übergehendes Restgas (Oxidationskreisgas), das die (verglichen mit Acrylsäure) vergleichsweise schwierig zu "kondensierenden" Bestandteile des Produktgasgemischs 3 umfasst. Dies sind, wie bereits gesagt, in der Regel diejenigen Komponenten, deren Siedepunkt bei Normaldruck (1 bar) ≤ -30°C beträgt (ihr Gesamtteil am Oxidationsrestgas beträgt in der Regel ≥ 70 Vol.-%, häufig ≥ 80 Vol.-% und vielfach ≥ 90 Vol.-%).

Das Produktgasgemisch 3 ist beim erfindungsgemäßen Verfahren in der Regel im wesentlichen zusammengesetzt aus dem Zielprodukt Acrylsäure, nicht umgesetztem molekularem Sauerstoff, Propan, nicht umgesetztem Propylen, molekularem Stickstoff, als Nebenprodukt entstandenem und/oder als Verdünnungsgas mitverwendetem Wasserdampf, als Nebenprodukt entstandenen und/oder als Verdünnungsgas mitverwendeten Kohlenoxiden, Restmengen an Acrolein, sowie geringen Mengen sonstiger niederer Aldehyde, niederer Alkancarbonsäuren (z. B. Essigsäure, Ameisensäure und Propionsäure) sowie Maleinsäureanhydrid, Benzaldehyd, aromatische Carbonsäuren und aromatische Carbonsäureanhydride (z.B. Phthalsäureanhydrid und Benzoesäure), sowie gegebenenfalls weiteren Kohlenwasserstoffen und sonstigen inerten Verdünnungsgasen.

Dem vorstehend beschriebenen entsprechend, gehören zum Oxidationskreisgas erfindungsgemäß in erster Linie nicht umgesetztes Propan, nicht umgesetztes Propylen sowie in der Regel $O_2$ und inerte Verdünnungsgase wie z.B. andere gesättigte Kohlenwasserstoffe wie Methan, Ethan, aber auch $N_2$, $CO_2$, Edelgase (He, Ne, Ar etc.), CO, sowie in geringem Umfang auch Acrylsäure und Acrolein sowie gegebenenfalls Essigsäure, Formaldehyd und Ameisensäure sowie Ethylen. Sein Wasserdampfgehalt kann bis zu 25 Vol.-%, häufig nur bis zu 20 Vol.-%, oder nur bis zu 10 Vol.-%, vielfach aber auch unter 10 Vol.-% oder unter 5 Vol.-% betragen. Sonstige niedere Aldehyde und Alkancarbonsäuren können gleichfalls in geringen Mengen enthalten sein.

D.h., im Regelfall besteht Oxidationskreisgas überwiegend aus den für die beiden Partialoxidationsstufen verwendeten inerten Verdünnungsgasen sowie aus bei der Partialoxidation üblicherweise als Nebenprodukt gebildetem oder als Verdünnungsgas zugesetztem Wasserdampf und durch unerwünschte vollständige Oxidation gebildeten Kohlenoxiden.

Erfindungsgemäß kann nun das Oxidationskreisgas als solches (und mit ihm das darin enthaltene Propan und Propylen) in die erste Reaktionsstufe (in die Reaktionsstufe 1) rückgeführt werden. Selbstverständlich können aber auch das darin enthaltene Propan und Propylen zuvor von anderen Bestandteilen abgetrennt und so für sich oder im Beisein weniger Nebenkomponenten in die erste Reaktionsstufe rückgeführt werden. Im letzteren Fall befände sich an dieser Stelle ein erster Auslaß für von Propan und Propylen verschiedene Bestandteile aus dem erfindungsgemäßen Verfahren.

Eine solche Abtrennung von Propan und Propylen kann z. B. durch Absorption mit nachfolgender Desorption und/oder Strippung (sowie Absorptionsmittelwiederverwendung) in einem hochsiedenden hydrophoben organischen Lösungsmittel (z. B. Tetradekan) erfolgen. Weitere Trennmöglichkeiten sind Adsorption, Rektifikation, Membranverfahren und partielle Kondensation. Bevorzugt werden die genannten Trennverfahren bei erhöhtem Druck ausgeführt.

Bei Verwendung von Dehydrierkatalysatoren, die gegenüber Sauerstoff oder Sauerstoff enthaltenden Verbindungen empfindlich sind, wird man diese Oxygenate vor einer Rückführung des Propan und Propylen von selbigen zweckmäßig abtrennen. Eine solche Sauerstoffabtrennung kann auch sinnvoll sein, um eine Verbrennung von Propan und/oder Propylen an der Rückführstelle in die Dehydrierstufe zu vermeiden. Die Dehydrierkatalysatoren der DE-A 19 937 107 sind nicht empfindlich gegen Oxygenate (insbesondere jene gemäß Beispiel 1 bis 4 der DE-A).

Eine andere Abtrennmöglichkeit bietet die fraktionierte Destillation. Vorzugsweise wird eine fraktionierte Druckdestillation bei tiefen Temperaturen durchgeführt. Der anzuwendende Druck kann z.B. 10 bis 100 bar betragen. Als Rektifikationskolonnen können Füllkörperkolonnen, Bodenkolonnen oder Packungskolonnen eingesetzt werden. Als Bodenkolonnen eignen sich Osolche mit Dual-Flow-Böden, Glockenböden oder Ventilböden. Das Rücklaufverhältnis kann z.B. 1 bis 10 betragen. Andere Trennmöglichkeiten bilden z.B. Druckextraktion, Druckwechseladsorption, Druckwäsche, partielle Kondensation und Druckextraktion.

Falls das $C_3$-Kreisgas vor seiner Rückführung in die erste Reaktionsstufe noch Kohlenmonoxid enthält kann dieses vorab der Rückführung katalytisch zu $CO_2$ verbrannt werden. Das gebildete $CO_2$ lässt sich dann durch Wäsche mit einer basischen Flüssigkeit vergleichsweise einfach abtrennen.

Natürlich kann auch so verfahren werden, dass man nur einen Teil des Oxidationskreisgases in seiner Zusammensetzung unverändert in die erste Reaktionsstufe rückführt und nur aus dem verbliebenen Teil Propan und Propylen im Gemisch (im Verbund) abtrennt und ebenfalls in die erste Reaktionsstufe rückführt (wie bereits früher erwähnt, können alle beschriebenen Propan/Propylen-Rückführungen teilweise auch in die zweite und/oder in die dritte Reaktionsstufe hinein erfolgen).

Im Rahmen einer Abtrennung von Propan und Propylen durch fraktionierte Destillation des Oxidationskreisgases kann eine Trennlinie z.B. so gelegt werden, dass im Auftriebsteil der Rektifikationskolonne im wesentlichen alle diejenigen Bestandteile abgetrennt und am Kopf der Kolonne abgezogen werden, deren Siedepunkt tiefer als der Siedepunkt von Propylen liegt. Diese Bestandteile werden in erster Linie die Kohlenoxide CO und $CO_2$ sowie nicht umge-setzter Sauerstoff und Ethylen sowie Methan, Ethan und $N_2$ sein. Im Sumpf können schwerer als Propylen und Propan siedende Bestandteile abgetrennt werden.

Wird in der ersten Reaktionsstufe des erfindungsgemäßen Verfahrens eine heterogen katalysierte Oxidehydrierung des Propans angewendet, kann ein Auslaß an von Propan und Propylen verschiedenen Nebenkomponenten auch immer dann mit vorgenommen werden, wenn wie in den Schriften DE-A 19837520, DE-A 19837517, DE-A 19837519 und DE-A 19837518 Abtrennungen von molekularem Stickstoff vorgenommen werden.

Das vorstehend beschriebene Oxidationskreisgas bildet (bezogen auf die darin enthaltene Menge an Propan und Propylen) die Hauptmenge (normalerweise wenigstens 80 Gew.-%, bzw. wenigstens 90 Gew.-%, oder wenigstens 95 Gew.-% oder mehr) aller $C_3$-Kreisgase eines erfindungsgemäßen Verfahrens, weshalb es und die daraus durch Nebenkomponentenabtrennung gegebenenfalls erzeugten Propan und Propylen enthaltenden Kreisgasströme auch als Haupt-$C_3$-Kreisgas bezeichnet werden.

Insbesondere dann, wenn die Kondensation der Acrylsäure durch Absorption mittels eines organischen Lösungsmittels erfolgt, fällt in der Trennzone A in der Regel wenigstens ein zweites, nicht umgesetztes Propan sowie nicht umgesetztes Propylen enthaltendes, restliches Gas an, das erfindungsgemäß vorteilhaft ebenfalls als $C_3$-Kreisgas behandelt wird. Bezogen auf darin enthaltenes Propan und Propylen ist seine Menge im Vergleich zum Haupt-$C_3$-Kreis-gas normalerweise wesentlich geringer. Dies ist darauf zurückzuführen, dass das sich bildende Absorbat neben Acrylsäure in gewissem Umfang auch Propan und Propylen aufnimmt. Im weiteren Verlauf der extraktiven, destillativen, kristallisativen und/oder desorptiven Abtrennung der Acrylsäure aus der kondensierten Phase bzw. us dem Absorbat, werden dieses nicht umgesetzte Propan und Propylen erfindungsgemäß vorteilhaft als Bestandteil wenigstens einer weiteren Gasphase rückgewonnen und diese als weiteres $C_3$-Kreisgas rückgeführt. Vorab der Rückführung kann wie beim Haupt-$C_3$-Kreisgas beschrieben, als weiterer möglicher Nebenkomponentenauslass, eine Nebenkomponentenabtrennung vorgenommen werden (unabhängig davon, ob solch eine Nebenkomponentenabtrennung vorgenommen wird oder nicht, wird für diese rückgeführten $C_3$-Kreisgase der Begriff Neben-$C_3$-Kreisgas verwendet).

Die Rückführung der Neben-$C_3$-Kreisgase kann eigenständig oder im Gemisch mit Haupt-$C_3$-Kreisgas erfolgen. Für letzteres wird der Begriff Gesamt-$C_3$-Kreisgas verwendet. Neben-$C_3$-Kreisgase können an Sauerstoff frei, oder auch Sauerstoff enthaltend sein. Letzteres ist beispielsweise dann der Fall, wenn sie durch Strippen mittels Luft oder am Kopf einer mittels Luft als Polymerisationsinhibitor gespülten Rektifikationskolonne anfallen. Mit Vorteil können die Neben-$C_3$-Kreisgase infolge ihrer Mengenbeschränkung auch unmittelbar dem Reaktionsgasausgangsgemisch 2 (bevorzugt) und/oder dem Reaktionsgasausgangsgemisch 3 zugeführt werden.

Erfindungsgemäß bevorzugt erfolgt die Rückführung der $C_3$-Kreisgase in die Reaktionsstufe 1 in das Reaktionsgasausgangsgemisch 1 hinein, das zu deren Beschickung verwendet wird und auch für das erfindungsgemäße Verfahren benötigtes Frischpropan enthält. Sie kann aber auch längs des Dehydrierumsatzes in die Reaktionsstufe 1 hinein erfolgen, wie es die DE-A 102004032129 empfiehlt.

Unter Frischpropan wird in dieser Schrift Propan verstanden, das noch an keiner chemischen Reaktion teilgenommen hat. In der Regel wird es Roh-Propan (das vorzugsweise die Spezifikation gemäß DE-A 102 46 119 und DE-A 102 45 585 erfüllt) sein, das in geringen Mengen auch von Propan verschiedene Komponenten enthält.

Das Reaktionsgasausgangsgemisch 2 für die Propylenpartialoxidation zu Acrolein erfüllt in dieser Schrift in zweckmäßiger Weise gleichfalls die in der DE-A 102 46 119 und DE-A 102 45 585 empfohlenen Spezifikationen.

Im Unterschied zur exotherm verlaufenden homogenen oder heterogen katalysierten Oxidehydrierung, die durch anwesenden Sauerstoff erzwungen und bei der intermediär kein freier Wasserstoff gebildet wird (der dem zu dehydrierenden Propan entrissene Wasserstoff wird unmittelbar als Wasser ($H_2O$) entrissen) bzw. nachweisbar ist, soll unter einer heterogen katalysierten Dehydrierung eine ("konventionelle") Dehydrierung verstanden werden, deren Wärmetönung im Unterschied zur Oxidehydrierung endotherm ist (als Folgeschritt kann eine exotherme Wasserstoffverbrennung in die heterogen katalysierte Dehydrierung einbezogen sein) und bei der wenigstens intermediär freier molekularer Wasserstoff gebildet wird. Dazu bedarf es in der Regel anderer Reaktionsbedingungen und anderer Katalysatoren als zur Oxidehydrierung.

Unter der Belastung eines einen Reaktionsschritt katalysierenden Katalysatorbetts mit Reaktionsgas(ausgangs)gemisch wird in dieser Schrift die Menge an Reaktionsgas(ausgangs)gemisch in Normlitern (=Nl; das Volumen in Litern, das die entsprechende Reaktionsgas(ausgangs)gemischmenge bei Normalbedingungen (0˚C, 1 bar) einnehmen würde) verstanden, die pro Stunde durch einen Liter Katalysatorfestbett geführt wird.

Die Belastung kann auch nur auf einen Bestandteil des Reaktionsgas(ausgangs)gemischs bezogen sein. Dann ist es die Menge dieses Bestandteils in Nl/l•h, die pro Stunde durch einen Liter des Katalysatorfestbetts geführt wird (reine Intertmaterialschüttungen werden nicht zum Katalysatorfestbett gerechnet).

Als ein Inertgas soll in dieser Schrift ein Reaktionsgasbestandteil verstanden werden, der sich unter den Bedingungen der Reaktion im wesentlich als inert verhält und -jeder inerte Reaktionsgasbestandteil für sich betrachtet - zu mehr als 95 mol-%, vorzugsweise zu mehr als 99 mol-% chemisch unverändert erhalten bleibt.

Prinzipiell wird der Propylengehalt des Reaktionsgasausgangsgemischs 2 unter dem Aspekt befriedigender Raum-Zeit-Ausbeuten ≥ 4 Vol.-% betragen. Die erfindungsgemäße Verfahrensweise ist jedoch insbesondere dann vorteilhaft, wenn der Propylengehalt des Reaktionsgasausgangsgemischs 2 ≥ 7 Vol.-% beträgt.

Im Normalfall wird der vorgenannte Propylengehalt ≤ 15 Vol.-% betragen. Erfindungsgemäß bevorzugt sind Propylengehalte des Reaktionsgasausgangsgemischs 2 von 7 bis 12 Vol.-%, besonders bevorzugt von 7 bis 11 Vol.-% und ganz besonders bevorzugt von 7 bis 10 Vol.-% bzw. von 7 bis 9 Vol.-%. Der erfindungsgemäß bevorzugte Propylengehalt des Reaktionsgasausgangsgemischs 2 liegt bei 8 Vol.-%.

Weiterhin ist es für das erfindungsgemäße Verfahren von generellem Vorteil, wenn das molare Verhältnis $V_1$ von im Reaktionsgasausgangsgemisch 2 enthaltenem Propan zu im Reaktionsgasausgangsgemisch 2 enthaltenem Propylen 1 bis 4 beträgt. Bevorzugt ist $V_1$ = 1 bis 3,5, besonders bevorzugt 1 bis 3,0 oder 1 bis 2,5 und ganz besonders bevorzugt beträgt $V_1$ = 1,5 bis 2,2.

Außerdem ist es für das erfindungsgemäße Verfahren von generellem Vorteil, wenn das molare Verhältnis von im Reaktionsgasausgangsgemisch 2 enthaltenem molekularem Stickstoff zu im Reaktionsgasausgangsgemisch 2 enthaltenem molekularem Sauerstoff 2 bis 6, bevorzugt 3 bis 4,5, besonders bevorzugt 3,5 bis 4,5 und ganz besonders bevorzugt 3,5 bis 4 bzw. 3,73 beträgt.

Desweiteren ist es für das erfindungsgemäße Verfahren von allgemeinem Vorteil, wenn das molare Verhältnis $V_3$ von im Reaktionsgasausgangsgemisch 2 enthaltenem molekularem Sauerstoff zu im Reaktionsgasausgangsgemisch 2 enthaltenem Propylen 1,3 bis 2,4, bevorzugt 1,4 bis 2,2, besonders bevorzugt 1,4 bis 2,1 und ganz besonders bevorzugt 1,5 bis 2,1 bzw. 1,7 bis 2,1 oder 1,9 beträgt.

Mögliche erfindungsgemäße Reaktionsgasausgangsgemische 2 enthalten

6 bis 9 Vol.-% Propylen,
8 bis 18 Vol.-% molekularer Sauerstoff,
6 bis 30 Vol.-% Propan und
32 bis 72 Vol.-% molekularer Stickstoff

mit

$V_1$ = 1 bis 4,
$V_2$ = 2 bis 6 und
$V_3$ = 1,3 bis 2,4.

Bevorzugte erfindungsgemäße Reaktionsgasausgangsgemische 2 enthalten

7 bis 9 Vol.-% Propylen,
9,8 bis 16 Vol.-% molekularer Sauerstoff,
9 bis 25 Vol.-% Propan und
35 bis 65 Vol.-% molekularer Stickstoff,

mit

$V_1$ = 1 bis 3,5,
$V_2$ = 3 bis 4,5 und
$V_3$ = 1,4 bis 2,2.

Ganz besonders bevorzugte Reaktionsgasausgangsgemische 2 enthalten

7 bis 9 Vol.-% Propylen,
9,8 bis 15 Vol.-% molekularer Sauerstoff,
10,5 bis 20 Vol.-% Propan und
40 bis 60 Vol.% molekularer Stickstoff,

mit

$V_1$ = 1,5 bis 2,5,
$V_2$ = 3,5 bis 4,5 und
$V_3$ = 1,4 bis 2,14.

Das erfindungsgemäß bevorzugteste Reaktionsgasausgangsgemisch 2 enthält

7 bis 8 Vol.-% Propylen,
11,9 bis 15,5 Vol.-% molekularer Sauerstoff,
11,9 bis 15,5 Vol.-% Propan und
50 bis 60 Vol.-% molekularer Stickstoff

mit

$V_1$ = 1,7 bis 2,1
$V_2$ = 3,5 bis 4,5 und
$V_3$ = 1,7 bis 2,1.

Prinzipiell wird der Acroleingehalt des Reaktionsgasausgangsgemischs 3 unter dem Aspekt befriedigender Raum-Zeit-Ausbeuten ≥ 3 Vol.-% betragen.
Die erfindungsgemäße Verfahrensweise ist jedoch insbesondere dann vorteilhaft, wenn der Acroleingehalt des Reaktionsgasausgangsgemischs 3 ≥ 4, oder ≥ 5, oder ≥ 6 Vol.-% beträgt. Im Normalfall wird der vorgenannte Acroleingehalt ≤ 15 Vol.-% betragen. Erfindungsgemäß bevorzugt sind Acroleingehalte des Reaktionsgasausgangsgemischs 3 von 6 bis 11 Vol.-%, besonders bevorzugt von 6 bis 10 Vol.-% und ganz besonders bevorzugt von 6 bis 9 bzw. 6 bis 8 Vol.-%. Anstelle der Ziffer 6 kann in den vorgenannten Acroleingehaltsgrenzen auch eine 5,5 stehen. Der erfindungsgemäß bevorzugte Acroleingehalt des Reaktionsgasausgangsgemischs 3 liegt bei 7 Vol.-%.
Weiterhin ist es für das erfindungsgemäße Verfahren von generellem Vorteil, wenn das molare Verhältnis $V_4$ von im Reaktionsgasausgangsgemisch 3 enthaltenem molekularem Sauerstoff zu im Reaktionsgasausgangsgemisch 3 enthaltenem Acrolein ≥ 0,5 und ≤ 2, mit Vorteil ≥ 1 und ≤ 1,75, besonders vorteilhaft ≥ 1 und ≤ 1,5 und ganz besonders vorteilhaft ≥ 1 und ≤ 1,25 beträgt.
Zusätzlich ist es für das erfindungsgemäße Verfahren von Vorteil, wenn das molare Verhältnis $V_5$ von im Reaktionsgasausgangsgemisch 3 enthaltenem Propan zu in ihm enthaltenem Acrolein 1 bis 4, mit Vorzug 1,5 bis 3,5, besonders bevorzugt 1,5 bis 3 und ganz besonders bevorzugt 1,5 bzw. 2 bis 2,5 beträgt.
Mögliche erfindungsgemäße Reaktionsgasausgangsgemische 3 enthalten

4,5 bis 8 Vol.-% Acrolein,
2,25 bzw 4,5 bis 9 Vol.-% molekularer Sauerstoff,
6 bis 30 Vol.-% Propan,
32 bis 72 Vol.-% molekularer Stickstoff und
5 bis 15 Vol.-% Wasserdampf.

Bevorzugte erfindungsgemäße Reaktionsgasausgangsgemische 3 enthalten

5,5 bis 8 Vol.-% Acrolein,
2,75 bzw. 5,5 bis 9 Vol.-% molekularer Sauerstoff,
10 bis 25 Vol.-% Propan,

40 bis 70 Vol.-% molekularer Stickstoff und

5 bis 15 Vol.-% Wasserdampf.

Ganz besonders bevorzugte erfindungsgemäße Reaktionsgasausgangsgemische 3 enthalten

6 bis 8 Vol.-% Acrolein,

3 bzw. 6 bis 9 Vol.-% molekularer Sauerstoff,

10 bis 20 Vol.-% Propan,

50 bis 65 Vol.-% molekularer Stickstoff und

7 bis 13 Vol.-% Wasserdampf.

Erfindungsgemäß ist es günstig, wenn ein mögliches Reaktionsgasausgangsgemisch 2 und ein mögliches Reaktionsgasausgangsgemisch 3, oder ein bevorzugtes Reaktionsgasausgangsgemisch 2 und ein bevorzugtes Reaktionsgasausgangsgemisch 3, oder ein ganz besonders bevorzugtes Reaktionsgasausgangsgemisch 2 und ein ganz besonders bevorzugtes Reaktionsgasausgangsgemisch 3 beim erfindungsgemäßen Verfahren in Kombination zur Anwendung kommen.

In der Regel enthalten sowohl das Reaktionsgasausgangsgemisch 2 als auch das Reaktionsgasausgangsgemisch 3 neben den in den vorstehenden Rastern aufgelisteten Bestandteilen ≤ 10 Vol.-%, vorzugsweise ≤ 8 Vol.-%, besonders bevorzugt ≤ 5 Vol.-% und ganz besonders bevorzugt ≤ 3 Vol.-% sonstige Bestandteile.

Der Gehalt sowohl des Reaktionsgasausgangsgemischs 2 als auch des Reaktionsgasausgangsgemischs 3 an Methan und/oder Ethan wird in der Regel ≤ 8 Vol.-%, meist ≤ 5 Vol.-% und üblicherweise ≤ 3 Vol.-% bzw ≤ 2 Vol.-% betragen. Häufig wird er jedoch mit Vorteil (beide Bestandteile sind im wesentlichen inert und von günstiger Wärmeleitfähigkeit) ≤ 0,5 Vol.-% betragen.

Besonders vorteilhaft ist der Gesamtgehalt des Reaktionsgasausgangsgemischs 2 und des Reaktionsgasausgangsgemisch 3 an Kohlenoxiden ($CO_2$, CO) beim erfindungsgemäßen Verfahren s 5 Vol.-%, besonders vorteilhaft ≤ 3 Vol.-% bzw. 2 Vol.-%.

In entsprechender Weise enthält das Reaktionsgasausgangsgemisch 2 erfindungsgemäß mit Vorteil ≤ 5 Vol.-% Wasser, vorteilhaft ≤ 3 Vol.-% Wasser, besonders vorteilhaft ≤ 2 Vol.-% Wasser und in der Regel ≥ 0,5 Vol.-% Wasser.

Die Belastung sowohl des Katalysatorfestbetts 2 als auch des Katalysatorfestbetts 3 mit dem jeweiligen Reaktionsgasausgangsgemisch liegt erfindungsgemäß vorzugsweise bei 1500 bis 4000 bzw. 6000 Nl/l•h oder mehr.

Die Belastung sowohl des Katalysatorfestbetts 2 als auch des Katalysatorfestbetts 3 mit dem jeweiligen Reaktanden (Propylen bzw. Acrolein) beträgt normalerweise ≥ 70 Nl/l•h, vorteilhaft ≥ 90 Nl/l•h oder ≥ 120 Nl/l•h, besonders vorteilhaft ≥ 140 Nl/l•h und ganz besonders vorteilhaft ≥ 160 Nl/l•h. In der Regel liegen die vorgenannten Reaktandenbelastungen bei ≤ 600 Nl/l•h, vielfach ≤ 300 Nl/l•h, und oft ≤ 250 Nl/l•h.

Sowohl in der zweiten Reaktionsstufe als auch in der dritten Reaktionsstufe liegt der Reaktionsdruck in der Regel im Bereich von 0,5 bis 3 bar, wobei der Bereich 1 bis 3 bar bevorzugt ist.

Die Temperatur des Katalysatorfestbetts 2 in der zweiten Reaktionsstufe liegt in der Regel bei 300 bis 400˚C. Die Temperatur des Katalysatorfestbetts 3 in der dritten Reaktionsstufe liegt in der Regel bei 200 bis 350˚C.

Handelt es sich bei der ersten Reaktionsstufe um eine Oxidehydrierung des Propan kann diese als homogene und/oder heterogen katalysierte Oxidehydrierung von Propan zu Propylen mit molekularem Sauerstoff in der Gasphase durchgeführt werden. Als Quelle des molekularen Sauerstoff kann dabei Luft, reiner molekularer Sauerstoff oder an molekularem Sauerstoff angereicherte Luft eingesetzt werden. Als alternative Sauerstoffquelle kommen auch Stickoxide wie $NO_2$, $N_2O_4$ etc. in Betracht.

Gestaltet man die erste Reaktionsstufe als eine homogene Oxidehydrierung, so läßt sich diese prinzipiell so durchführen, wie es z.B. in den Schriften US-A 3,798,283, CN-A 1 105 352, Applied Catalysis, 70(2)1991, S. 175-187, Catalysis Today 13, 1992, S. 673-678 und in der Anmeldung DE-A 19 622 331 beschrieben ist. Eine zweckmäßige Sauerstoffquelle ist Luft. Die Temperatur der homogenen Oxidehydrierung wird zweckmäßigerweise im Bereich von 300 bis 700˚C, vorzugsweise im Bereich von 400 bis 600˚C, besonders bevorzugt im Bereich von 400 bis 500˚C liegend gewählt. Der Arbeitsdruck kann 0,5 bis 100 bar, insbesondere 1 bis 10 bar betragen. Die Verweilzeit liegt üblicherweise bei 0,1 beziehungsweise 0,5 bis 20 Sekunden, vorzugsweise bei 0,1 beziehungsweise 0,5 bis 5 Sekunden.

Als Reaktor kann zum Beispiel ein Rohrofen oder ein Rohrbündelreaktor verwendet werden, wie zum Beispiel ein Gegenstrom-Rohrofen mit Rauchgas als Wärmeträger oder ein Rohrbündelreaktor mit Salzschmelze als Wärmeträger. Das Propan zu Sauerstoff-Verhältnis im entsprechenden Reaktionsgasausgangsgemisch 1 beträgt vorzugsweise 0,5:1 bis 40:1, insbesondere zwischen 1:1 bis 6:1 und stärker bevorzugt zwischen 2:1 bis 5:1. Das Reaktionsgasausgangsgemisch 1 kann auch weitere, bevorzugt inerte (unter inerten Bestandteilen sollen in dieser Schrift, wie bereits gesagt, ganz generell vorzugsweise solche Bestandteile verstanden werden, die sich beim relevanten Reaktionsschritt zu weniger als 5 mol%, bevorzugt zu weniger als 3 mol.-% und besonders bevorzugt zu weniger als 1 mol.-% umsetzen; ganz besonders bevorzugt setzten sie sich überhaupt nicht um), Bestandteile, wie Wasser, Kohlendioxid, Kohlenmonoxid, Stickstoff, Edelgase, andere Kohlenwasserstoffe (z.B. im Roh-Propan enthaltene Nebenbestandteile), und/oder Propylen etc. umfassen, wobei es sich hierbei auch um zurückgeführte (Kreisgas) Bestandteile handeln kann.

Gestaltet man die Propandehydrierung als eine heterogen katalysierte Oxidehydrierung, so läßt sich diese prinzipiell durchführen wie beschrieben zum Beispiel in den Schriften US-A 4 788 371, CN-A 1073893, Catalysis Letters 23 (1994), 103-106, W. Zhang, Gaodeng Xuexiao Huaxue Xuebao, 14 (1993) 566, Z. Huang, Shiyou Huagong, 21 (1992) 592, WO 97/36849, DE-A 197 53 817, US-A 3 862 256, US-A 3 887 631, DE-A 195 30 454, US-A 4 341 664, J. of Catalysis 167, 560-569 (1997), J. of Catalysis 167, 550-559 (1997), Topics in Catalysis 3 (1996) 265-275, US-A 5 086 032, Catalysis Letters 10 (1991), 181-192, Ind. Eng. Chem. Res. 1996, 35, 14-18, US-A 4 255 284, Applied Catalysis A: General, 100 (1993), 111-130, J. of Catalysis 148, 56-67 (1994), V. Cortés Corberán und S. Vic Bellón (Ed.), New Developments in Selective Oxidation II, 1994, Elsevier Science B.V., S. 305-313, 3rd World Congress on Oxidation Catalysis, R.K. Grasselli, S.T. Oyama, A.M. Gaffney and J.E. Lyons (Ed.), 1997, Elsevier Science B.V., S. 375ff oder in DE-A 19837520, DE-A 19837517, DE-A 19837519 und DE-A 19837518. Dabei kann als Sauerstoffquelle auch Luft eingesetzt werden. Häufig weist jedoch die Sauerstoffquelle hier zu mindestens 90 mol-% an molekularem Sauerstoff, und vielfach wenigstens 95 mol-% an molekularem Sauerstoff auf.

Die für die heterogen katalysierte Oxidehydrierung geeigneten Katalysatoren unterliegen keinen besonderen Beschränkungen. Es eignen sich alle dem Fachmann auf diesem Gebiet bekannten Oxidehydrierungskatalysatoren, die in der Lage sind, Propan zu Propylen zu oxidieren. Insbesondere können alle in den zuvor genannten Schriften genannten Oxidehydrierungskatalysatoren eingesetzt werden. Geeignete Katalysatoren sind beispielsweise Oxidehydrierungskatalysatoren, die MoVNb-Oxide oder Vanadylpyrophosphat, gegebenenfalls mit Promotor, umfassen. Ein Beispiel für einen günstigen Oxidehydrierkatalysator ist ein Katalysator, der ein Mischmetalloxid mit Mo, V, Te, O und X als wesentliche Bestandteile enthält, wobei X mindestens ein Element ist, ausgewählt aus Niob, Tantal, Wolfram, Titan, Aluminium, Zirkonium, Chrom, Mangan, Gallium, Eisen, Ruthenium, Kobalt, Rhodium, Nickel, Palladium, Platin, Antimon, Bismut, Bor, Indium, Silizium, Lanthan, Natrium, Lithium, Kalium, Magnesium, Silber, Gold und Cer (vgl. dazu auch EP-A 938463 und EP-A 167109). Weiterhin besonders geeignete Oxidehydrierungskatalysatoren sind die Multimetalloxidmassen beziehungsweise -katalysatoren A der DE-A-197 53 817 und die Katalysatoren der DE-A 19838312, wobei die in der erstgenannten Schrift als bevorzugt genannten Multimetalloxidmassen beziehungsweise -katalysatoren A ganz besonders günstig sind. Das heißt, als Aktivmassen kommen für die Oxidehydrierung insbesondere Multimetalloxidmassen der nachfolgenden allgemeinen Formel

$$M^1_a Mo_{1-b} M^2_b O_x,$$

wobei

M' = Co, Ni, Mg, Zn, Mn und/oder Cu,

$M^2$ = W, V, Te, Nb, P, Cr, Fe, Sb, Ce, Sn und/oder La,

a = 0,5-1,5

b = 0-0,5

sowie

x = eine Zahl, die durch die Wertigkeit und Häufigkeit der von Sauerstoff verschiedenen Elemente in der allgemeinen Formel bestimmt wird,

in Betracht.

Prinzipiell können geeignete Aktivmassen der vorgenannten allgemeinen Formel in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 450 bis 1000˚C calciniert. Als Quellen für die elementaren Konstituenten der allgemeinen Formel kommen solche Verbindungen in Betracht, bei denen es sich bereits um Oxide handelt und/oder solche Verbindungen, die durch Erhitzen, wenigstens in Anwesenheit von Sauerstoff, in Oxide überführbar sind. Hierbei handelt es sich vom allem um Halogenide, Nitrate, Formiate, Oxalate, Citrate, Acetate, Carbonate, Amminkomplexsalze, Ammoniumsalze und/oder Hydroxide. Das innige Vermischen der Ausgangsverbindungen zur Herstellung der Multimetalloxidmasse kann in trok-kener Form, zum Beispiel als feinteiliges Pulver, oder in nasser Form, zum Beispiel mit Wasser als Lösungmittel, erfolgen. Die resultierenden Multimetalloxidmassen können sowohl in Pulverform als auch zu bestimmten Katalysatorgeometrien geformt, zur Oxidehydrierung eingesetzt werden, wobei die Formgebung vor oder nach der abschließenden Calcinierung erfolgen kann. Sie können als Vollkatalysatoren eingesetzt werden. Die Formgebung einer pulverförmigen Aktivmasse beziehungsweise Vorläufermasse kann aber auch durch Aufbringung auf vorgeformte inerte Katalysatorträger erfolgen. Als Trägermaterialien können dabei übliche, poröse oder unporöse Aluminiumoxide, Siliciumdioxid, Thoriumdioxid, Zirkondioxid, Siliciumcarbid oder Silikate verwendet werden, wobei die Trägerkörper regelmäßig oder unregelmäßig geformt sein können.

Für die heterogen katalysierte Oxidehydrierung des Propans liegt die Reaktionstemperatur vorzugsweise im Bereich von 200 bis 600˚C, insbesondere im Bereich von 250 bis 500˚C, stärker bevorzugt im Bereich von 350 bis 440˚C. Der Arbeitsdruck liegt vorzugsweise im Bereich von 0,5 bis 10 bar, insbesondere von 1 bis 10 bar, stärker bevorzugt von 1 bis 5 bar. Arbeitsdrucke oberhalb von 1 bar, zum Beispiel von 1,5 bis 10 bar, haben sich als besonders vorteilhaft erwiesen. In der Regel erfolgt die heterogen katalysierte Oxidehydrierung des Propans an einem Katalysatorfestbett. Letzteres wird zweckmäßigerweise in den Rohren eines Rohrbündelreaktors aufgeschüttet, wie sie zum Beispiel in der EP-A-0 700 893 und in der EP-A-0 700 714 sowie der in diesen Schriften zitierten Literatur beschrieben sind. Die mittlere Verweilzeit des Reaktionsgasgemisches 1 in der Katalysatorschüttung liegt zweckmäßigerweise bei 0,5 bis 20 Sekunden. Das Verhältnis von Propan zu Sauerstoff variiert mit dem gewünschten Umsatz und der Selektivität des Katalysators. Zweckmäßigerweise liegt es im Bereich von 0,5:1 bis 40:1, insbesondere von 1:1 bis 6:1, stärker bevorzugt von 2:1 bis 5:1. In der Regel nimmt die Propylenselektivität mit steigendem Propanumsatz ab. Vorzugsweise wird deshalb die Propan zu Propylen-Reaktion so durchgeführt, daß relativ niedrige Umsätze mit Propan bei hohen Selektivitäten zu Propylen erreicht werden. Besonders bevorzugt liegt der Umsatz an Propan im Bereich von 5 bis 40 mol-%, häufig im Bereich von 10 bis 30 mol-%. Hierbei bedeutet der Begriff "Propanumsatz den Anteil an zugeführtem Propan (Summe aus im Roh-Propan und rückgeführtem $C_3$-Kreisgas enthaltenem Propan) der beim einfachen Durchgang umgesetzt wird. In der Regel beträgt die Selektivität der Propylenbildung 50 bis 98 mol-%, stärker bevorzugt 80 bis 98 mol%, wobei der Begriff "Selektivität" die Mole an Propylen bezeichnet, die pro Mol umgesetztem Propan erzeugt werden, ausgedrückt als molarer Prozentsatz.

In der Regel enthält das bei der oxidativen Propandehydrierung eingesetzte Ausgangsgemisch 5 bis 95 mol-% Propan (bezogen auf 100 mol-% Ausgangsgemisch). Neben Propan und Sauerstoff kann das Ausgangsgemisch für die heterogen katalysierte Oxidehydrierung auch weitere, insbesondere inerte, Bestandteile wie Kohlendioxid, Kohlenmonoxid, Stickstoff, Edelgase, andere Kohlenwasserstoffe, z.B. im Roh-Propan enthaltene Nebenbestandteile, und/oder Propylen umfassen. Die heterogen katalysierte Oxidehydrierung kann auch in Anwesenheit von Verdünnungsmitteln, wie zum Beispiel Wasserdampf, durchgeführt werden.

Jede beliebige Reaktorsequenz kann zur Durchführung der homogenen Oxidehydrierung oder der heterogen katalysierten Oxidehydrierung des Propans eingesetzt werden, die dem Fachmann bekannt ist. Zum Beispiel kann die Oxidehydrierung in einem einzigen Reakor oder in einer Kaskade aus zwei oder mehreren Reaktoren, zwischen denen gegebenenfalls Sauerstoff eingeführt wird, durchgeführt werden. Es besteht auch die Möglichkeit, die homogene und die heterogen katalysierte Oxidehydrierung miteinander kombiniert zu praktizieren.

Als mögliche Bestandteile kann das Produktgasgemisch 1 einer erfindungsgemäßen Propanoxidehydrierung zum Beispiel folgende Komponenten enthalten: Propylen, Propan, Kohlendioxid, Kohlenmonoxid, Wasser, Stickstoff, Sauerstoff, Ethan, Ethen, Methan, Acrolein, Acrylsäure, Ethylenoxid, Butan (z.B. n-Butan oder iso-Butan), Essigsäure, Formaldehyd, Ameisensäure, Propylenoxid und Butene (z.B. Buten-1). In typischer Weise enthält ein bei der erfindungsgemäßen Propanoxidehydrierung erhaltenes Produktgasgemisch: 5 bis 10 mol-% Propylen, 0,1 bis 2 mol-% Kohlenmonoxid, 1 bis 3 mol-% Kohlendioxid, 4 bis 10 mol-% Wasser, 0 bis 1 mol-% Stickstoff, 0,1 bis 0,5 mol-% Acrolein, 0 bis 1 mol-% Acrylsäure, 0,05 bis 0,2 mol-% Essigsäure, 0,01 bis 0,05 mol-% Formaldehyd, 1 bis 5 mol-% Sauerstoff, 0,1 bis 1,0 mol-% weitere oben genannte Komponenten, sowie als Rest im wesentlichen Propan, jeweils bezogen auf 100 mol-% Produktgasgemisch.

Erfindungsgemäß vorteilhaft handelt es sich bei der ersten Reaktionsstufe jedoch um eine konventionelle heterogen katalysierte partielle Dehydrierung des Propans. Prinzipiell kommen dazu alle bekannten heterogen katalysierten partiellen Dehydrierungen des Propans in Betracht, wie sie z. B. aus den Schriften WO 03/076370, WO 01/96271, EP-A 117 146, WO 03/011804, US-A 3,161,670, WO 01/96270, DE-A 33 13 573, DE-A 102 45 585, DE-A 103 16 039 sowie aus der deutschen Anmeldung DE-A 10 2004 032 129 vorbekannt sind.

Ebenso kommen alle im Stand der Technik dafür bekannten Dehydrierkatalysatoren in Betracht. Mit Vorteil wird man im Katalysatorfestbett dehydrieren.

Die Dehydrierkatalysatoren lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen, Träger abgeschiedenen, in der Regel vergleichsweise edlen, Metall (zum Beispiel Platin) bestehen. Unter anderem können damit sowohl alle Dehydrierkatalysatoren eingesetzt werden, die in der DE-A 102 19 879, WO 01/96270, der EP-A 731 077, der DE-A 10211275, der DE-A 10131297, der WO 99/46039, der US-A 4 788 371, der EP-A-0 705 136, der WO 99/29420, der US-A 4 220 091, der US-A 5 430 220, der US-A 5 877 369, der EP-A-0 117 146, der DE-A 199 37 196, der DE-A 199 37 105 sowie der DE-A 199 37 107 empfohlen werden, als auch der Katalysator gemäß Beispiel 4 der DE-A 102 19 879. Im besonderen können sowohl der Katalysator gemäß Beispiel 1, Beispiel 2, Beispiel 3, und Beispiel 4 der DE-A 199 37 107 als auch der Katalysator gemäß Beispiel 4 der DE-A 102 19 879 sowie die Katalysatoren der WO 02/51547, WO 02/51540 und der DE-A 10 2005 002 127 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

Besonders geeignet ist auch der im Beispiel dieser Schrift eingesetzte Dehydrierkatalysator.

Generell kann es sich bei den Dehydrierkatalysatoren um Katalysatorstränge (Durchmesser typisch 1 bis 10 mm, bevorzugt 1,5 bis 5 mm; Länge typisch 1 bis 20 mm, bevorzugt 3 bis 10 mm), Tabletten (vorzugsweise gleiche Abmessungen wie bei den Strängen) und/oder Katalysatorringe (Außendurchmesser und Länge jeweils typisch 2 bis 30 mm oder bis 10 mm, Wandstärke zweckmäßig 1 bis 10 mm, oder bis 5 mm, oder bis 3 mm) handeln.

In der Regel sind die Dehydrierkatalysatoren (insbesondere die in dieser Schrift beispielhaft verwendeten sowie die in der DE-A 199 37 107 empfohlenen (insbesondere die beispielhaften Katalysatoren dieser DE-A) so beschaffen, dass sie sowohl die Dehydrierung von Propan als auch die Verbrennung von molekularem Wasserstoff zu katalysieren vermögen. Die Wasserstoffverbrennung verläuft dabei im Vergleich zur Dehydrierung des Propans im Fall einer Konkurrenzsituation an den Katalysatoren sehr viel schneller.

Zur Durchführung der heterogen katalysierten Propandehydrierung kommen prinzipiell alle im Stand der Technik bekannten Reaktortypen und Verfahrensvarianten in Betracht. Beschreibungen solcher Verfahrensvarianten enthalten zum Beispiel alle bezüglich der konventionellen Dehydrierungen und zugehöriger Dehydrierkatalysatoren zitierten Schriften des Standes der Technik.

Charakteristisch für die partielle heterogen katalysierte Dehydrierung von Propan ist, dass sie endotherm verläuft. Das heißt, die für die Einstellung der erforderlichen Reaktionstemperatur sowie die für die Reaktion benötigte Wärme (Energie) muss entweder dem Reaktionsgasausgangsgemisch 1 vorab und/oder im Verlauf der heterogen katalysierten Dehydrierung zugeführt werden. Gegebenenfalls muss das Reaktionsgasgemisch 1 die benötigte Reaktionswärme sich selbst entziehen.

Ferner ist es für heterogen katalysierte Dehydrierungen von Propan aufgrund der hohen benötigten Reaktionstemperaturen typisch, dass in geringen Mengen schwersiedende hochmolekulare organische Verbindungen, bis hin zum Kohlenstoff, gebildet werden, die sich auf der Katalysatoroberfläche abscheiden und selbige dadurch deaktivieren. Um diese nachteilige Begleiterscheinung zu minimieren, kann das zur heterogen katalysierten Dehydrierung bei erhöhter Temperatur über die Katalysatoroberfläche zu leitende, Propan haltige Reaktionsgasgemisch 1 mit Wasserdampf verdünnt werden. Sich abscheidender Kohlenstoff wird unter den so gegebenen Bedingungen nach dem Prinzip der Kohlevergasung teilweise oder vollständig eliminiert.

Eine andere Möglichkeit, abgeschiedene Kohlenstoffverbindungen zu beseitigen, besteht darin, den Dehydrierkatalysator von Zeit zu Zeit (bei Bedarf täglich oder stündlich) bei erhöhter Temperatur mit einem Sauerstoff enthaltenden Gas (zweckmäßig in Abwesenheit von Kohlenwasserstoffen) zu durchströmen und damit den abgeschiedenen Kohlenstoff quasi abzubrennen. Eine weitgehende Unterdrückung der Bildung von Kohlenstoffablagerungen ist aber auch dadurch möglich, dass man dem heterogen katalysiert zu dehydrierenden Propan, bevor es bei erhöhter Temperatur über den Dehydrierkatalysator geführt wird, molekularen Wasserstoff zusetzt. Hohe Dehydrierumsätze bedingen in der Regel kurze Regenerierabstände.

Selbstverständlich besteht auch die Möglichkeit, dem heterogen katalysiert zu dehydrierenden Propan ein Gemisch aus Wasserdampf und molekularem Wasserstoff zuzusetzen. Ein Zusatz von molekularem Wasserstoff zur heterogen katalysierten Dehydrierung von Propan mindert auch die unerwünschte Bildung von Allen (Propadien), Propin und Acetylen als Nebenprodukten. Teiloxidation von solchermaßen zugesetztem Wasserstoff vermag gleichfalls benötigte Reaktionswärme zu liefern.

Erfindungsgemäß wesentlich ist, dass als Reaktionsstufe 1 auch heterogen katalysierte partielle Propandehydrierungen in Betracht kommen, bei denen der Propanumsatz 20 bis 30 mol-% beträgt (auf den einmaligen Durchgang von Frischpropan durch die Dehydrierung bezogen), Besonders günstig für das erfindungsgemäße Verfahren sind als Reaktionsstufe 1 jedoch heterogen katalysierte partielle Propandehydrierungen, bei denen der vorgenannte Propanumsatz 30 bis 60 mol-%, vorzugsweise 35 bis 55 mol-% und besonders bevorzugt 35 bis 45 mol-% beträgt. Ein anderer geeigneter Umsatzbereich beträgt 25 bzw. 30 bis 40 mol-%.

Für die Realisierung der vorgenannten Propanumsätze ist es günstig, die heterogen katalysierte Propandehydrierung bei einem Arbeitsdruck von 0,3 bis 10 bar, bzw. vorteilhaft bis 3 bar durchzuführen. Ferner ist es günstig, das heterogen katalytisch zu dehydrierende Propan mit Wasserdampf zu verdünnen. So ermöglicht die Wärmekapazität des Wassers einerseits, einen Teil der Auswirkung der Endothermie der Dehydrierung auszugleichen und andererseits reduziert die Verdünnung mit Wasserdampf den Edukt- und Produktpartialdruck, was sich günstig auf die Gleichgewichtslage der Dehydrierung auswirkt. Ferner wirkt sich die Wasserdampfmitverwendung, wie bereits erwähnt, vorteilhaft auf die Standzeit von Edelmetall enthaltenden Dehydrierkatalysatoren aus, insbesondere im Fall von angestrebten hohen Propanumsätzen. Bei Bedarf kann als weiterer Bestandteil auch molekularer Wasserstoff zugegeben werden. Das molare Verhältnis von molekularem Wasserstoff zu Propan ist dabei im Reaktionsgasausgangsgemisch 1 in der Regel ≤ 5. Das molare Verhältnis von Wasserdampf zu Propan wird im Reaktionsgasausgangsgemisch 1 zweckmäßig ≥ 0,05 bis 2 bzw. bis 1 betragen.

Generell werden möglichst kleine Wasserdampfgehalte im Reaktionsgasausgangsgemisch 1 bevorzugt und angestrebt. Bei auf Frischpropan bezogenen Propanumsätzen im Bereich von 20 bis 30 mol.-% ist üblicherweise die in erfindungsgemäß gegebenenfalls ins Reaktionsgasausgangsgemisch 1 rückgeführtem Oxidationskreisgas üblicherweise enthaltene Wasserdampfmenge als Wasserdampfversorgung für die heterogen katalysierte Dehydrierung ausreichend. Für höhere auf Frischpropan bezogene Propanumsätze wird normalerweise darüber hinaus Wasserdampf zugegeben, bei welchem es sich z. B. um abgeschiedenes Prozesswasser handeln kann.

Grundsätzlich kann eine als Reaktionsstufe 1 fungierende heterogen katalysierte partielle Propandehydrierung (quasi) adiabat und dabei endotherm durchgeführt werden. Dabei wird das Reaktionsgasausgangsgemisch 1 in der Regel zunächst auf eine Temperatur von 500 bis 700°C (beziehungsweise von 550 bis 650°C) erhitzt (zum Beispiel durch Direktbefeuerung der es umgebenden Wandung). Beim adiabaten Durchgang durch das wenigstens eine Katalysatorbett wird sich das Reaktionsgasgemisch 1 dann je nach Umsatz und Verdünnung um etwa 30°C bis 200°C abkühlen. Ein Beisein von Wasserdampf als Wärmeträger macht sich auch unter dem Gesichtspunkt einer adiabaten Fahrweise vorteilhaft bemerkbar. Niedrigere Reaktionstemperaturen ermöglichen längere Standzeiten des verwendeten Katalysatorbetts. Höhere Reaktionstemperaturen fördern erhöhte Umsätze.

Anwendungstechnisch zweckmäßig wird man eine heterogen katalysierte Propandehydrierung als Reaktionsstufe 1 für das erfindungsgemäße Verfahren als Hordenreaktor verwirklichen.

Dieser enthält räumlich aufeinanderfolgend zweckmäßig mehr als ein die Dehydrierung katalysierendes Katalysatorbett. Die Katalysatorbettenanzahl kann 1 bis 20, zweckmäßig 2 bis 8, aber auch 3 bis 6 betragen. Mit zunehmender Hordenzahl lassen sich zunehmend leichter erhöhte Propanumsätze erreichen. Die Katalysatorbetten sind vorzugsweise radial oder axial hintereinander angeordnet. Anwendungstechnisch zweckmäßig wird in einem solchen Hordenreaktor das Katalysatorbett als Festbett gestaltet.

Im einfachsten Fall sind die Katalysatorfestbetten in einem Schachtofenreaktor axial oder in den Ringspalten von zentrisch ineinandergestellten zylindrischen Gitterrosten angeordnet. Es ist jedoch auch möglich, die Ringspalte in Segmenten übereinander anzuordnen und das Gas nach radialem Durchtritt in einem Segment in das nächste darüber- oder darunterliegende Segment zu führen.

In zweckmäßiger Weise wird das Reaktionsgasgemisch 1 auf seinem Weg von einem Katalysatorbett zum nächsten Katalysatorbett, zum Beispiel durch Überleiten über mit heißen Gasen erhitzte Wärmetauscheroberflächen (z. B. Rippen) oder durch Leiten durch mit heißen Brenngasen erhitzte Rohre, im Hordenreaktor einer Zwischenerhitzung unterworfen.

Wird der Hordenreaktor im übrigen adiabat betrieben, ist es für auf Frischpropan bezogen Propanumsätze $\leq$ 30 mol-%, vor allem bei Verwendung der in der DE-A 199 37 107 beschriebenen Katalysatoren, insbesondere der beispielhaften Ausführungsformen, ausreichend, das Reaktionsgasgemisch 1 auf eine Temperatur von 450 bis 550°C vorerhitzt in den Dehydrierreaktor zu führen und innerhalb des Hordenreaktors in diesem Temperaturbereich zu halten. Das heißt, die gesamte Propandehydrierung ist so bei äußerst niederen Temperaturen zu verwirklichen, was sich für die Standzeit der Katalysatorfestbetten zwischen zwei Regenerierungen als besonders günstig erweist. Für höhere Propanumsätze wird das Reaktionsgasgemisch 1 zweckmäßig auf höhere Temperaturen vorerhitzt in den Dehydrierreaktor geführt (diese können bis zu 700°C betragen) und innerhalb des Hordenreaktors in diesem erhöhten Temperaturbereich gehalten.

Noch geschickter ist es, die vorstehend geschilderte Zwischenerhitzung auf direktem Weg durchzuführen (autotherme Fahrweise). Dazu wird dem Reaktionsgasgemisch 1 entweder bereits vor Durchströmung des ersten Katalysatorbettes (dann sollte das Reaktionsgasausgangsgemisch 1 molekularen Wasserstoff zugesetzt enthalten) und/ oder zwischen den nachfolgenden Katalysatorbetten in begrenztem Umfang molekularer Sauerstoff zugesetzt. So kann (in der Regel durch die Dehydrierkatalysatoren selbst katalysiert) eine begrenzte Verbrennung von im Reaktionsgasgemisch 1 enthaltenem, im Verlauf der heterogen katalysierten Propandehydrierung gebildetem und/oder dem Reaktionsgasgemisch 1 zugesetztem molekularem Wasserstoff (gegebenenfalls begleitet von einer in geringem Umfang erfolgenden Propanverbrennung) bewirkt werden (es kann auch anwendungstechnisch zweckmäßig sein, im Hordenreaktor Katalysatorbetten einzufügen, die mit Katalysator beschickt sind, der besonders spezifisch (selektiv) die Verbrennung von Wasserstoff katalysiert (als solche Katalysatoren kommen zum Beispiel jene der Schriften US 4,788,371, US 4,886,928, US 5,430,209, US 5,530,171, US 5,527,979 und US 5,563,314 in Betracht; beispielsweise können solche Katalysatorbetten in alternierender Weise zu den Dehydrierkatalysator enthaltenden Betten im Hordenreaktor untergebracht sein). Die dabei freigesetzte Reaktionswärme ermöglicht so auf quasi autotherme (die Bruttowärmetönung ist im wesentlichen Null) Weise eine nahezu isotherme Betriebsweise der heterogen katalysierten Propandehydrierung. Mit zunehmender gewählter Verweilzeit des Reaktionsgases im Katalysatorbett ist so eine Propandehydrierung bei abnehmender oder im wesentlichen konstanter Temperatur möglich, was besonders lange Standzeiten zwischen zwei Regenerierungen ermöglicht (im Extremfall kann zur Gewährleistung der Autothermie auch nur Propan verbrannt werden).

Generell sollte erfindungsgemäß eine wie vorstehend beschriebene Sauerstoffeinspeisung so vorgenommen werden, dass der Sauerstoffgehalt des Reaktionsgasgemisches 1, bezogen auf die darin enthaltene Menge an molekularem Wasserstoff, 0,5 bis 50 bzw. bis 30, vorzugsweise 10 bis 25 Vol.-% beträgt. Als Sauerstoffquelle kommen dabei sowohl reiner molekularer Sauerstoff oder mit Inertgas, zum Beispiel CO, $CO_2$, $N_2$ und/oder Edelgase, verdünnter Sauerstoff, insbesondere aber auch Luft (abgesehen von Oxidationskreisgas wird bevorzugt ausschließlich Luft als Sauerstoffquelle verwendet), in Betracht. Die resultierenden Verbrennungsgase wirken in der Regel zusätzlich verdünnend und fördern dadurch die heterogen katalysierte Propandehydrierung. Dies gilt insbesondere für den im Rahmen der Verbrennung gebildeten Wasserdampf.

Die Isothermie der für das erfindungsgemäße Verfahren in zweckmäßiger Weise als Reaktionsstufe 1 fungierenden heterogen katalysierten Propandehydrierung lässt sich dadurch weiter verbessern, dass man im Hordenreaktor in den Räumen zwischen den Katalysatorbetten geschlossene, vor ihrer Füllung günstigerweise aber nicht notwendigerweise evakuierte, Einbauten (zum Beispiel rohrförmige) anbringt. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Selbstredend lässt sich eine die Reaktionsstufe 1 bildende heterogen katalysierte Propandehydrierung auch wie in der DE-A 102 11 275 beschrieben (als "Schlaufenvariante") realisierten, die einen integralen Bestandteil dieser Patentanmeldung bildet.

D. h., vorteilhaft für das erfindungsgemäße Verfahren ist, dass als Reaktionsstufe 1 ein Verfahren der kontinuierlichen heterogen katalysierten partiellen Dehydrierung von Propan in der Gasphase fungieren kann, bei dem

- einer Dehydrierzone ein das zu dehydrierende Propan enthaltende Reaktionsgasausgangsgemisch 1 kontinuierlich zugeführt wird,
- das Reaktionsgasausgangsgemisch 1 in der Dehydrierzone durch wenigstens ein Katalysatorfestbett geführt wird, an welchem durch katalytische Dehydrierung molekularer Wasserstoff und (partiell) Propylen gebildet werden,
- dem Reaktionsgasausgangsgemisch 1 vor und/oder nach Eintritt in die Dehydrierzone wenigstens ein molekularen Sauerstoff enthaltendes Gas zugesetzt wird,
- der molekulare Sauerstoff in der Dehydrierzone im Reaktionsgasgemisch 1 enthaltenen molekularen Wasserstoff teilweise zu Wasserdampf oxidiert
und
- der Dehydrierzone ein Produktgas entnommen wird, das molekularen Wasserstoff, Wasserdampf, Propylen und nicht umgesetztes Propan enthält und das dadurch gekennzeichnet ist, dass das der Dehydrierzone entnommene Produktgas in zwei Teilmengen identischer Zusammensetzung aufgeteilt und eine der beiden Teilmengen als Dehydrierkreisgas (vorzugsweise als Bestandteil des Reaktionsgasausgangsgemischs 1) in die Dehydrierzone zurückgeführt wird, wie es auch die WO 03/076370 empfiehlt.

Dabei kann Oxidationskreisgas Bestandteil des Reaktionsgasausgangsgemisch 1 sein und/oder gemäß der Lehre der deutschen Anmeldung DE-A 10 2004 032 129 erst nach bereits teilweise erfolgter Dehydrierung dem Reaktionsgasgemisch 1 zugeführt werden.

Ist Oxidationskreisgas Bestandteil des Reaktionsgasausgangsgemischs 1, enthält dieses zweckmäßig lediglich dem Oxidationskreisgas entstammenden molekularen Sauerstoff.

Für das erfindungsgemäße Verfahren ist es im Rahmen der beschriebenen Schlaufenfahrweise günstig, wenn die Menge des Dehydrierkreisgases, bezogen auf das in der Dehydrierzone gebildete Produktgas, 30 bis 70 Vol.-%, vorteilhaft 40 bis 60 Vol.-%, vorzugsweise 50 Vol.-% beträgt.

Mit Bezug auf die erfindungsgemäß nachfolgenden Reaktionsstufen 2 und 3 enthält das Reaktionsgasausgangsgemisch 1 für den Fall einer wie beschrieben z. B. im Hordenreaktor durchgeführten Schlaufenfahrweise (Hordenschlaufenreaktor dann = Dehydrierzone) im stationären Zustand in zweckmäßiger Weise:

| | |
|---|---|
| 15 bis 25 Vol.-% | Propan, |
| 2 bis 6 Vol.-% | Propylen, |
| 5 bis 20 Vol.-% | Wasserdampf, |
| 2 bis 10 Vol.-% | molekularer Wasserstoff, |
| 40 bis 75 Vol.-% | molekularer Stickstoff, und |
| > 0 bis 3 Vol.-% | molekularer Sauerstoff. |

Der Umsatz an Propan (bezogen auf den einmaligen Durchgang des vorgenannten Reaktionsgasausgangsgemisch 1 durch den in Schlaufenfahrweise betriebenen Hordenreaktor) und das Schlaufenkreisgasverhältnis (Menge des Dehydrierkreisgases bezogen auf die in der Dehydrierzone insgesamt anfallende Produktgasmenge) werden mit Blick auf die erfindungsgemäß nachfolgende zweistufige heterogen katalysierte Gasphasenpartiafoxidation des Propylens mit Vorteil (z. B. im Hordenschlaufenreaktor als Dehydrierzone) so gewählt, dass das in der Dehydrierzone gebildete Produktgas nicht umgesetztes Propan und gewünschtes Propylen im molaren Verhältnis Propen zu Propan von 0,25 bzw. 0,3 bis 0,5 (gegebenenfalls bis 0,66) enthält. Bei einem Schlaufenkreisgasverhältnis von 0,5 entspricht dem ein auf den einmaligen Durchgang des Reaktionsgasausgangsgemisch 1 durch die Dehydrierzone bezogener Umsatz des darin enthaltenen Propan von 15 bis 25 mol-%.

Typische Belastungen der Dehydrierkatalysatorbetten mit Reaktionsgasgemisch 1 betragen 250 bis 5000 $h^{-1}$ ( bei Hochlastfahrweise auch bis 40000 $h^{-1}$), vorzugsweise 10000 bis 25000 Nl/l•h, besonders bevorzugt 15000 bis 20000 Nl/l•h. Die entsprechenden Belastungen mit Propan liegen typisch bei 50 bis 1000 $h^{-1}$ (bei Hochlastfahrweise auch bis 40000 $h^{-1}$), vorzugsweise bei 2000 bis 5000 Nl/l·h, besonders bevorzugt 3000 bis 4000 Nl/l•h.

Das der Dehydrierzone (dem Dehydrierreaktor) als Propylenquelle (Reaktionsstufe 1) entnommene Dehydrierproduktgas befindet sich entsprechend den für die heterogen katalysierte Propandehydrierung gewählten Reaktionsbedingungen bei einem Druck von 0,3 bis 10 bar, vorzugsweise 1 bis 3 bar, und weist häufig eine Temperatur von 450 bis 650˚C, vielfach eine Temperatur von 500 bis 600˚C auf. In der Regel enthält es Propan, Propen, $H_2$, $N_2$, $H_2O$ Methan, Ethan (die letzteren beiden resultieren meist infolge thermischen Zerfalls einer geringen Propanmenge), Ethylen, Buten-1, sonstige Butene wie iso-Buten, andere $C_4$-Kohlenwasserstoffe wie n-Butan, iso-Butan, Butadien etc., CO und $CO_2$, im Regelfall aber auch Oxygenate wie Alkohole, Aldehyde und Carbonsäuren (normalerweise mit $\leq$ 9 C-Atomen). Wei-terhin können in geringen Mengen aus dem Oxidationskreisgas herrührende Bestandteile enthalten sein.

Während die EP-A 117 146, die DE-A 33 13 573 und die US-A 3,161,670 empfehlen, das in einer Propanoxidehydrierung und/oder Propandehydrierung gebildete Produktgas als solches zur Beschickung der erfindungsgemäßen Partialoxidation zu verwenden, ist es für die erfindungsgemäß zweistufige sich anschließende Partialoxidation des Propylens zu Acrylsäure vorteilhaft, aus dem das Propylen enthaltenden Produktgas der ersten Reaktionsstufe vor seiner Verwendung als Propenquelle für die erfindungsgemäße Propylenpartialoxidation wenigstens eine Teilmenge der darin enthaltenen, von Propan und Propylen verschiedenen, Bestandteile abzutrennen. Dabei sollten die Erfordernisse der DE-A 102 11 275 beachtet werden. Diese Abtrennung bietet eine weitere Auslassmöglichkeit für von Propan und Propylen verschiedene Nebenkomponenten beim ertindungsgemäßen Verfahren.

Erfindungsgemäß vorteilhaft wird man wenigstens 50 Vol.-%, vorzugsweise wenigstens 75 Vol.-%, besonders bevorzugt wenigstens 90 Vol.-% und ganz besonders bevorzugt wenigstens 95 Vol.-% der im Produktgas der ersten Reaktionsstufe enthaltenen, von Propan und Propylen verschiedenen, Bestandteile abtrennen, bevor selbiges als Propylenquelle für die erfindungsgemäße Partialoxidation eingesetzt wird. Dies insbesondere dann, wenn es sich bei der ersten Reaktionsstufe um eine heterogen katalysierte partielle Propandehydrierung handelt.

Eine für die erfindungsgemäßen Bedürfnisse zweckmäßige Möglichkeit dazu besteht z.B. darin, das vorzugsweise abgekühlte (vorzugsweise auf Temperaturen von 10 bis 100 bzw. 70˚C) Produktgasgemisch der Propandehydrierung und/oder Oxidehydrierung, z. B. bei einem Druck von 0,1 bis 50 bar, vorzugsweise 5 bis 15 bar und einer Temperatur von z. B. 0 bis 100˚C, vorzugsweise 20 bis 40˚C, mit einem (vorzugsweise hochsiedenden) organischen Lösungsmittel (vorzugsweise ein hydrophobes), in welchem Propan und Propylen (gegenüber den anderen Bestandteilen des Produktgasgemischs der Propandehydrierung und/oder Oxidehydrierung zweckmäßig bevorzugt) absorbiert werden, in Kontakt zu bringen (z. B. durch einfaches Durchleiten). Durch nachfolgende Desorption, Rektifikation und/oder Strippung mit einem bezüglich der erfindungsgemäßen Partialoxidation sich inert verhaltenden und/oder in dieser Partialoxidation als Reaktand benötigten Gas (z. B. Luft oder ein anderes Gemisch aus molekularem Sauerstoff und Inertgas) können das Propan und Propylen im Gemisch in gereinigter Form rückgewonnen und dieses Gemisch für die Partialoxidation als Propylenquelle verwendet werden (vorzugsweise wird wie im Vergleichsbeispiel 1 der deutschen Anmeldung DE-A 10 2004 032 129 beschrieben vorgegangen). Das gegebenenfalls molekularen Wasserstoff enthaltende Abgas einer solchen Absorption kann man z.B. wieder einer Druckwechseladsorptions- und/oder Membrantrennung (z.B. gemäß DE-A 10235419) unterwerfen und dann, bei Bedarf, den abgetrennten Wasserstoff mitverwenden.

Allerdings ist der C3-Kohlenwasserstoffe/C4-Kohlenwasserstoffe Trennfaktor beim vorstehenden Trennverfahren vergleichsweise begrenzt und für die in der DE-A 10245585 beschriebenen Bedürfnisse häufig nicht ausreichend.

Als Alternative für den beschriebenen Trennschritt via Absorption ist für die erfindungsgemäßen Zwecke daher häufig eine Druckwechseladsorption oder eine Druckrektifikation bevorzugt.

Als Absorptionsmittel für die vorstehend beschriebene absorptive Abtrennung eignen sich grundsätzlich alle Absorptionsmittel, die in der Lage sind, Propan und Propylen zu absorbieren (auf die im folgenden beschriebene Art und Weise können Propan und Propylen auch aus Oxidationskreisgas abgetrennt und dann als $C_3$-Kreisgas in die erste (und gegebenenfalls weitere) Reaktionsstufe rückgeführt werden). Bei dem Absorptionsmittel handelt es sich vorzugsweise um ein organisches Lösungsmittel, das vorzugsweise hydrophob und/oder hochsiedend ist. Vorteilhafterweise hat dieses Lösungsmittel einen Siedepunkt (bei einem Normaldruck von 1 atm) von mindestens 120˚C, bevorzugt von mindestens 180˚C, vorzugsweise von 200 bis 350˚C, insbesondere von 250 bis 300˚C, stärker bevorzugt von 260 bis 290˚C. Zweckmäßigerweise liegt der Flammpunkt (bei einem Normaldruck von 1 bar) über 110˚C. Allgemein eignen sich als Absorptionsmittel relativ unpolare organische Lösungsmittel, wie zum Beispiel aliphatische Kohlenwasserstoffe, die vorzugsweise keine nach außen wirkende polare Gruppe enthalten, aber auch aromatische Kohlenwasserstoffe. Allgemein ist es erwünscht, dass das Absorptionsmittel einen möglichst hohen Siedepunkt bei gleichzeitig möglichst hoher Löslichkeit für Propan und Propylen hat. Beispielhaft als Absorptionsmittel genannt seien aliphatische Kohlen-wasserstoffe, zum Beispiel $C_8$-$C_{20}$-Alkane oder -Alkene, oder aromatische Kohlenwasserstoffe, zum Beispiel Mittelölfraktionen aus der Paraffindestillation oder Ether mit sperrigen (sterisch anspruchvollen) Gruppen am O-Atom, oder Gemische davon, wobei diesen ein polares Lösungsmittel, wie zum Beispiel das in der DE-A 43 08 087 offenbarte 1,2-Dimethyl-phthalat zugesetzt sein kann. Weiterhin eignen sich Ester der Benzoesäure und Phthalsäure mit geradkettigen, 1 bis 8 Kohlenstoffatome enthaltenden Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkene, zum Beispiel 4-Methyl-4'-benzyl-diphenylmethan und dessen Isomere 2-Methyl-2'-benzyl-diphenyl-methan, 2-Methyl-4'-benzyldiphenyl-methan und 4-Methyl-2'-benzyldiphenylmethan und Gemische solcher Isomerer. Ein geeignetes Absorptionsmittel ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether, beispielsweise das im Handel erhältliche Diphyl® (z. B. von der Bayer Aktiengesellschaft bezogenes). Häufig enthält dieses Lösungsmittelgemisch ein Lösungsmittel wie Dimethylphthalat in einer Menge von 0,1 bis 25 Gew.-%, bezogen auf das gesamte Lösungsmittelgemisch, zugesetzt. Besonders geeignete Absorptionsmittel sind auch Octane, Nonane, Decane, Undecane, Dodecane, Tridecane, Tetradecane, Pentadecane, Hexadecane, Heptadecane und Octadecane, wobei sich insbesondere Tetradecane als besonders geeignet erwiesen haben. Günstig ist es, wenn das verwendete Absorptionsmittel einerseits den oben genannten Siedepunkt erfüllt, andererseits gleichzeitig aber ein nicht zu hohes Molekulargewicht aufweist. Mit Vorteil beträgt das Molekulargewicht des Absorptionsmittels $\leq$ 300 g/mol. Geeignet sind auch die in DE-A 33 13 573 beschriebenen Paraffinöle mit 8 bis 16 Kohlenstoffatomen. Beispiele für geeignete Handelsprodukte sind von der Firma Haltermann vertriebene Produkte wie Halpasole i, wie Halpasol 250/340 i und Halpasol 250/275 i, sowie Druckfarbenöle unter den Bezeichnungen PKWF und Printosol. Bevorzugt sind an Aromaten freie Handelsprodukte, z. B. solche des Typs PKWFaf. Falls sie einen geringen Restaromatengehalt enthalten, kann dieser vorab des beschriebenen Einsatzes vorteilhaft rektifikativ und/oder adsorptiv gemindert und auf Werte signifikant unter 1000 gew.ppm gedrückt werden.

Die Durchführung der Absorption unterliegt keinen besonderen Beschränkungen. Es können alle dem Fachmann gängigen Verfahren und Bedingungen eingesetzt werden. Vorzugsweise wird das Gasgemisch bei einem Druck von 1 bis 50 bar, bevorzugt 2 bis 20 bar, stärker bevorzugt 5 bis 15 bar, und einer Temperatur von 0 bis 100˚C, insbesondere von 20 bis 50 bzw. 40˚C, mit dem Absorptionsmittel in Kontakt gebracht. Die Absorption kann sowohl in Kolonnen als auch in Quenchapparaten vorgenommen werden. Dabei kann im Gleichstrom oder im Gegenstrom (ist bevorzugt) gearbeitet werden. Geeignete Absorptionskolonnen sind zum Beispiel Bodenkolonnen (mit Glocken- und/oder Siebböden), Kolonnen mit strukturierten Packungen (zum Beispiel Blechpackungen mit einer spezifischen Oberfläche von 100 bis 1000, oder bis 750 m$^2$/m$^3$, zum Beispiel Mellapak® 250 Y) und Füllkörperkolonnen (zum Beispiel mit Raschig-Füllkörpern gefüllte). Es können aber auch Riesel- und Sprühtürme, Graphitblockabsorber, Oberflächenabsorber wie Dickschicht- und Dünnschichtabsorber sowie Tellerwäscher, Kreuzschleierwäscher und Rotationswäscher verwendet werden. Zudem kann es günstig sein, die Absorption in einer Blasensäule mit und ohne Einbauten stattfinden zu lassen.

Die Abtrennung des Propans und Propylens von dem Absorptionsmittel kann durch Strippung, Entspannungsverdampfung (Flashen) und/oder Destillation erfolgen.

Die Abtrennung des Propans und Propylens von dem Absorptionsmittel erfolgt vorzugsweise durch Strippen und/oder Desorption. Die Desorption kann in üblicher Weise über eine Druck- und/oder Temperaturänderung durchgeführt werden, vorzugsweise bei einem Druck von 0,1 bis 10 bar, insbesondere 1 bis 5 bar, stärker bevorzugt 1 bis 3 bar, und einer Temperatur von 0 bis 200˚C, insbesondere 20 bis 100˚C, stärker bevorzugt 30 bis 70˚C, besonders bevorzugt 30 bis 50˚C. Ein für die Strippung geeignetes Gas ist beispielsweise Wasserdampf, bevorzugt sind jedoch insbesondere Sauerstoff-/Stickstoff-Mischungen, beispielsweise Luft. Bei der Verwendung von Luft beziehungsweise Sauerstoff-/Stickstoff-Mischungen, bei denen der Sauerstoffgehalt über 10 Vol-% liegt, kann es sinnvoll sein, vor und/oder während des Stripprozesses ein Gas zuzusetzen, das den Explosionsbereich reduziert. Besonders geeignet dafür sind Gase mit einer spezifischen Wärmekapazität $\geq$ 29 J/mol·K bei 20˚C, wie zum Beispiel Methan, Ethan, Propan (bevorzugt; es kommt Frischpropan und/oder C$_3$-Kreisgas in Betracht), Propen, Benzol, Methanol, Ethanol, sowie Ammoniak, Kohlendioxid, und Wasser C$_4$-Kohlenwasserstoffe sind erfindungsgemäß bevorzugt als solche Zusätze jedoch zu vermeiden. Besonders geeignet für die Strippung sind auch Blasensäulen mit und ohne Einbauten.

Die Abtrennung des Propans und Propylens von dem Absorptionsmittel kann auch über eine Destillation bzw. Rektifikation erfolgen, wobei die dem Fachmann geläufigen Kolonnen mit Packungen, Füllkörpern oder entsprechenden Einbauten verwendet werden können. Bevorzugte Bedingungen bei der Destillation bzw. Rektifikation sind ein Druck von 0,01 bis 5 bar, insbesondere 0,1 bis 4 bar, stärker bevorzugt 1 bis 3 bar, und eine Temperatur (im Sumpf) von 50 bis 300˚C, insbesondere 150 bis 250˚C.

Eine durch Strippen aus dem Absorptionsmittel gewonnene, für die nachfolgenden Partialoxidationsstufen geeignete Propylenquelle kann vor ihrer Verwendung zur Beschickung der Partialoxidation noch einer weiteren Verfahrensstufe zugeführt werden, um z. B. die Verluste an mitgestripptem Absorptionsmittel zu reduzieren (z. B. Abscheidung in Demistern und/oder Tiefenfiltern) und die Partialoxidationsstufen so gleichzeitig vor Absorptionsmittel zu schützen oder um die Trennwirkung zwischen C3-/C4-Kohlenwasserstoffen weiter zu verbessern. Eine solche Abtrennung des Absorptionsmittels kann nach allen dem Fachmann bekannten Verfahrensschritten erfolgen. Eine im Rahmen des erfindungsgemäßen Verfahrens bevorzugte Ausführungsform einer solchen Abtrennung ist z. B. das Quenchen des Ausgangsstromes aus der Strippvorrichtung mit Wasser. In diesem Fall wird das Absorptionsmittel aus diesem beladenen Ausgangsstrom mit Wasser herausgewaschen und der Ausgangsstrom gleichzeitig mit Wasser beladen (geringe Wassermengen wirken sich förderlich auf die Aktivität der Katalysatoren für die erfindungsgemäß nachfolgende Partialoxidation aus). Diese Wäsche beziehungsweise das Quenchen kann z. B. am Kopf einer Desorptionskolonne über einem Flüssigkeits-Fangboden durch Gegensprühen von Wasser oder in einem eigenen Apparat erfolgen.

Zur Unterstützung des Trenneffektes können im Quenchraum die Quenchoberfläche vergößernde Einbauten installiert werden, wie sie dem Fachmann von Rektifikationen, Absorptionen und Desorptionen her bekannt sind.

Wasser ist insofern ein bevorzugtes Waschmittel, da es in der nachfolgenden wenigstens einen Partialzone normalerweise nicht stört. Nachdem das Wasser das Absorptionsmittel aus dem mit Propan und Propylen beladenen Ausgangstrom herausgewaschen hat, kann das Wasser/Absorptionsmittel-Gemisch einer Phasentrennung zugeführt und der behandelte, volumenarme, Ausgangstrom, unmittelbar der erfindungsgemäß nachfolgenden Partialoxidation zugeführt werden.

In für das erfindungsgemäße Verfahren vorteilhafter Weise lassen sich insbesondere dann, wenn das Propylen/Propan-Gemisch aus dem Absorbat mittels Luft freigestrippt wird, in der Regel unmittelbar einsetzbare Reaktionsgasausgangsgemische 2 gewinnen. Für den Fall, dass deren Propangehalt erfindungsgemäß noch nicht befriedigen sollte, kann ihnen vor ihrer Verwendung für die erfindungsgemäße Partialoxidation des enthaltenen Propylens noch Frischpropan zugesetzt werden. Über $C_3$-Kreisgas (z. B. Oxidationskreisgas) wird selbiges dann erfindungsgemäß in die Reaktionsstufe 1 (z. B. in die heterogen katalysierte Dehydrierung (z. B. als Bestandteil des Reaktionsgasausgangsgemischs 1) rückgeführt werden. Um die entsprechende Propanmenge kann dann die Zufuhr an Frischpropan ins Reaktionsgasausgangsgemisch 1 (allgemein in die Reaktionsstufe1) verringert werden. Im Extremfall kann die in der Reaktionsstufe 1 (z.B. in der heterogen katalysierten Propandehydrierung) erforderliche Zufuhr an Frischpropan dann vollständig entfallen, wenn diese Zufuhr an Frischpropan vor der Durchführung der erfindungsgemäßen Partialoxidation des Propylens vollständig ins Reaktionsgasausgangsgemisch 2 und/oder 3 hinein erfolgt, von wo aus es dann als verbleibender Bestandteil im Oxidationskreisgas erst nach Durchlaufen der erfindungsgemäßen Partialoxidation dem Reaktionsgasausgangsgemisch 1 (bzw. allgemein der Reaktionsstufe 1) für z. B. die heterogen katalysierte Propandehydrierung zugeführt wird.

Ein Teil oder auch die vollständige Zufuhr an Frischpropan kann auch in das Reaktionsgasausgangsgemisch 3 hinein erfolgen (allerdings ist das Reaktionsgasausgangsgemisch 3 manchmal bereits dann nicht explosiv, wenn diese Qualifizierung schon auf das Reaktionsgasausgangsgemisch 2 zutraf). Dies ist vor allem deshalb vorteilhaft, weil eine unerwünschte Nebenreaktion von Propan zu Propionaldehyd und/oder Propionsäure vor allem von der ersten Reaktionsstufe unter deren Bedingungen ausgeht. Vorteilhaft ist es auch, eine Frischpropanzufuhr auf die zweite und auf die dritte Reaktionsstufe im wesentlichen gleichmäßig zu verteilen.

Durch diese Möglichkeit der Zufuhr von Frischpropan ins Reaktionsgasausgangsgemisch 2 und/oder 3 hinein, lässt sich die Zusammensetzung der Reaktionsgasausgangsgemische 2 und 3 zielsicher nicht explosiv gestalten. Entscheidend für die Beantwortung der Frage, ob das Reaktionsgasausgangsgemisch 2 bzw. 3 explosiv ist oder nicht, ist, ob sich in dem unter bestimmten Ausgangsbedingungen (Druck, Temperatur) befindlichen Reaktionsgasausgangsgemisch 2 bzw. 3 eine durch eine örtliche Zündquelle (z. B. glühender Platindraht) eingeleitete Verbrennung (Entzündung, Explosion) ausbreitet oder nicht (vgl. DIN51649 und die Untersuchungsbeschreibung in der WO 04/007405). Erfolgt eine Ausbreitung, soll das Gemisch hier als explosiv bezeichnet werden. Erfolgt keine Ausbreitung, wird das Gemisch in dieser Schrift als nicht explosiv eingeordnet. Ist das Reaktionsgasausgangsgemische 2 bzw. 3 nicht explosiv, gilt dies auch für die im Verlauf der erfindungsgemäßen Partialoxidation des Propylens gebildeten Reaktionsgasgemische 2 bzw. 3 (vgl. WO 04/007405).

Selbstverständlich kann man das für das erfindungsgemäße Verfahren benötigte Propan (Frischpropan) auch vollständig dem Reaktionsgasausgangsgemisch 1 zuführen. Die vorliegende Erfindung betrifft aber auch vorteilhafte Ausgestaltungen des erfindungsgemäßen Verfahrens, bei dem man das für das Verfahren benötigte Propan (Frischpropan) höchstens teilweise (z. B. nur zu 75 %, oder nur zu 50 %, oder nur zu 25 %) dem Reaktionsgasausgangsgemisch 1 und wenigstens teilweise (in der Regel die Restmenge, gegebenenfalls die Gesamtmenge) dem Reaktionsgasausgangsgemisch 2 (und/oder dem Reaktionsgasausgangsgemisch 3) zuführt. Im übrigen kann wie in der WO 01/96170 beschrieben verfahren werden, die einen integralen Bestandteil dieser Anmeldung bildet.

Die Zweistufigkeit der erfindungsgemäßen Propylenpartialoxidation zu Acrylsäure fußt auf dem Sachverhalt, dass die heterogen katalysierte Gasphase - Partialoxidation von Propylen zu Acrylsäure mit molekularem Sauerstoff grundsätzlich in zwei längs der Reaktionskoordinate aufeinanderfolgenden Schritten abläuft, von denen der erste zum Acrolein und der zweite vom Acrolein zur Acrylsäure führt.

Die Umsetzung dieses Sachverhalts in zwei Reaktionsstufen eröffnet die Möglichkeit, in jeder der beiden Oxidationsstufen das zu verwendende Katalysatorfestbett und vorzugsweise auch die sonstigen Reaktionsbedingungen, wie z. B. die Temperatur des Katalysatorfestbetts, in optimierender Weise anzupassen.

Für die jeweilige der beiden Reaktionsstufen 2, 3 besonders geeignete Multimetalloxidkatalysatoren sind vielfach vorbeschrieben und dem Fachmann wohlbekannt. Beispielsweise verweist die EP-A 25 34 09 auf Seite 5 auf entsprechende US-Patente.

Günstige Katalysatoren für die jeweilige Partialoxidationsstufe offenbaren auch die DE-A 4 431 957, die DE-A 10 2004 025 445 und die DE-A 4 431 949. Dieses gilt insbesondere für jene der allgemeinen Formel I in den beiden vorgenannten Schriften. Besonders vorteilhafte Katalysatoren für die jeweilige Oxidationsstufe offenbaren die Schriften DE-A 103 25 488, DE-A 103 25 487, DE-A 103 53 954, DE-A 103 44 149, DE-A 103 51 269, DE-A 103 50 812 und DE-A 103 50 822.

Für die erfindungsgemäße Reaktionsstufe 2, das ist die der heterogen katalysierten Gasphasen-Partialoxidation von Propylen zu Acrolein, kommen prinzipiell alle Mo, Bi und Fe enthaltenden Multimetalloxidmassen als Aktivmasse in Betracht.

Dies sind insbesondere die Multimetalloxidaktivmassen der allgemeinen Formel I der DE-A 199 55 176, die Multimetalloxidaktivmassen der allgemeinen Formel I der DE-A 199 48 523, die Multimetalloxidaktivmassen der allgemeinen Formeln I, II und III der DE-A 101 01 695, die Multimetalloxidaktivmassen der allgemeinen Formeln I, II und III der DE-A 199 48 248 und die Multimetalloxidaktivmassen der allgemeinen Formeln I, II und III der DE-A 199 55 168 sowie die in der EP-A 70 07 14 genannten Multimetalloxidaktivmassen.

Ferner eignen sich für diese Reaktionsstufe die Mo, Bi und Fe enthaltenden Multimetalloxidkatalysatoren die in den Schriften DE-A 100 46 957, DE-A 100 63 162, DE-C 3 338 380, DE-A 199 02 562, EP-A 15 565, DE-C 2 380 765, EP-A 8 074 65, EP-A 27 93 74, DE-A 330 00 44, EP-A 575897, US-A 4438217, DE-A 19855913, WO 98/24746, DE-A 197 46 210 (diejenigen der allgemeinen Formel II), JP-A 91/294239, EP-A 293 224 und EP-A 700 714 offenbart werden. Dies gilt insbesondere für die beispielhaften Ausführungsformen in diesen Schriften, unter denen jene der EP-A 15565, der EP-A 575897, der DE-A 197 46 210 und der DE-A 198 55 913 besonders bevorzugt werden. Besonders hervorzuheben sind in diesem Zusammenhang ein Katalysator gemäß Beispiel 1 c aus der EP-A 15 565 sowie ein in entsprechender Weise herzustellender Katalysator, dessen Aktivmasse jedoch die Zusammensetzung $Mo_{12}Ni_{6.5}Zn_2Fe_2Bi_1P_{0.0065}K_{0.06}O_x \cdot 10SiO_2$ aufweist. Ferner sind hervorzuheben das Beispiel mit der laufenden Nr. 3 aus der DE-A 198 55 913 (Stöchiometrie: $M0_{12}Co_7Fe_3Bi_{0.6}K_{0.06}Si_{1.6}O_x$) als Hohlzylindervollkatalysator der Geometrie 5 mm x 3 mm x 2 mm (Außendurchmesser x Höhe x Innendurchmesser) sowie der Multimetalloxid 11 - Vollkatalysator gemäß Beispiel 1 der DE-A 197 46 210. Ferner wären die Multimetalloxid-Katalysatoren der US-A 4,438,217 zu nennen. Letzteres gilt insbesondere dann, wenn diese Hohlzylinder eine Geometrie 5,5 mm x 3 mm x 3,5 mm, oder 5 mm x 2mm x 2mm, oder 5mm x 3mm x 2mm, oder 6mm x 3mm x 3mm, oder 7mm x 3 mm x 4 mm (jeweils Außendurchmesser x Höhe x Innendurchmesser) aufweisen. Weitere mögliche Katalysatorgeometrien sind in diesem Zusammenhang Stränge (z. B. 7,7 mm Länge und 7 mm Durchmesser; oder 6,4 mm Länge und 5,7 mm Durchmesser).

Eine Vielzahl der für den Schritt vom Propylen zu Acrolein geeigneten Multimetalloxidaktivmassen lässt sich unter der allgemeinen Formel IV,

$$Mo_{12}Bi_aFe_bX^1{}_cX^2{}_dX^3{}_eX^4{}_fO_n \qquad (IV),$$

in der die Variablen nachfolgende Bedeutung aufweisen:

$X^1 =$ Nickel und/oder Kobalt,

$X^2 =$ Thallium, ein Alkalimetall und/oder ein Erdalkalimetall,

$X^3 =$ Zink, Phosphor, Arsen, Bor, Antimon, Zinn, Cer, Blei und/oder Wolfram,

$X^4 =$ Silicium, Aluminium, Titan und/oder Zirkonium,

a= 0,5 bis 5,

b= 0,01 bis 5, vorzugsweise 2 bis 4,

c= 0 bis 10, vorzugsweise 3 bis 10,

d= 0 bis 2, vorzugsweise 0,02 bis 2,

e= 0 bis 8, vorzugsweise 0 bis 5,

f= 0 bis 10 und

n= eine Zahl, die durch die Wertigkeit und Häufigkeit der von Sauerstoff verschiede- nen Elemente in IV bestimmt wird,

subsumieren.

Sie sind in an sich bekannter Weise erhältlich (siehe z. B. die DE-A 4 023 239) und werden üblicherweise in Substanz zu Kugeln, Ringen oder Zylindern geformt oder auch in Gestalt von Schalenkatalysatoren, d. h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel IV in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemisch aus Inertgas, $NH_3$, CO und/oder $H_2$) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die Multimetalloxidaktivmassen der allgemeinen Formel IV können für den erfindungsgemäßen Schritt "Propylen → Acrolein" sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 2 909 671, der EP-A 293 859 oder aus der EP-A 714 700 bekannt ist. Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 $\mu$m, bevorzugt im Bereich 50 bis 500 $\mu$m und besonders bevorzugt im Bereich 150 bis 250 $\mu$m liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren unterliegenden Zielreaktion in der Regel im wesentlichen inert. Die Trägerkörper, können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für die zweite Reaktionsstufe zu verwendende Multimetalloxidaktivmassen sind ferner Massen der allgemeinen Formel V,

$$[Y^1{}_a \cdot Y^2{}_b \cdot O_x \cdot]_p [Y^3{}_c \cdot Y^4{}_d \cdot Y^5{}_e \cdot Y^6{}_f \, Y^7{}_g \cdot Y^2{}_h \cdot O_y \cdot]_q \qquad (V),$$

in der die Variablen folgende Bedeutung haben:

$Y^1$ = nur Wismut oder Wismut und wenigstens eines der Elemente Tellur, Antimon, Zinn und Kupfer,
$Y^2$ = Molybdän oder Molybdän und Wolfram,
$Y^3$ = ein Alkalimetall, Thallium und/oder Samarium,
$Y^4$ = ein Erdalkalimetall, Nickel, Kobalt, Kupfer, Mangan, Zink, Zinn, Cadmium und/oder Quecksilber,
$Y^5$ = Eisen oder Eisen und wenigstens eines der Elemente Chrom und Cer,
$Y^6$ = Phosphor, Arsen, Bor und/oder Antimon,
$Y^7$ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

a' = 0,01 bis 8,
b' = 0,1 bis 30,
c' = 0 bis 4,
d' = 0 bis 20,
e' > 0 bis 20,
f' = 0 bis 6,
g' = 0 bis 15,
h' = 8 bis 16,
x',y'= Zahlen, die durch die Wertigkeit und Häufigkeit der von Sauerstoff verschiede- nen Elemente in V bestimmt werden und
p,q = Zahlen, deren Verhältnis p/q 0,1 bis 10 beträgt,

enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1{}_a \cdot Y^2{}_b \cdot O_x \cdot$, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Besonders vorteilhafte erfindungsgemäße Multimetalloxidmassen V sind solche, in denen $Y^1$ nur Wismut ist.

Unter diesen werden wiederum jene bevorzugt, die der allgemeinen Formel VI,

$$[Bi_a \cdot Z^2{}_b \cdot O_x \cdot]_p \cdot [Z^2{}_{12} Z^3{}_c \cdot Z^4{}_d \cdot Fe_e \cdot Z^5{}_f \cdot Z^6{}_g \cdot Z^7{}_h \cdot O_y \cdot]_q \cdot \qquad (VI),$$

in der die Varianten folgende Bedeutung haben:

$Z^2$ = Molybdän oder Molybdän und Wolfram,
$Z^3$ = Nickel und/oder Kobalt,
$Z^4$ = Thallium, ein Alkalimetall und/oder ein Erdalkalimetall,
$Z^5$ = Phosphor, Arsen, Bor, Antimon, Zinn, Cer und/oder Blei,
$Z^6$ = Silicium, Aluminium, Titan und/oder Zirkonium,
$Z^7$ = Kupfer, Silber und/oder Gold,

a" = 0,1 bis 1,
b" = 0,2 bis 2,
c" = 3 bis 10,
d" = 0,02 bis 2,
e" = 0,01 bis 5, vorzugsweise 0,1 bis 3,
f" = 0 bis 5,
g" = 0 bis 10;
h" = 0 bis 1,
x",y"= Zahlen, die durch die Wertigkeit und Häufigkeit der von Sauerstoff verschiede- nen Element in VI bestimmt werden,
p",q"= Zahlen, deren Verhältnis p"/q" 0,1 bis 5, vorzugsweise 0,5 bis 2 beträgt,

entsprechen, wobei diejenigen Massen VI ganz besonders bevorzugt werden, in denen $Z^2{}_{b"}$ = (Wolfram)$_{b"}$ und $Z^2{}_{12}$ =

(Molybdän)$_{12}$ ist.

Ferner ist es von Vorteil, wenn wenigstens 25 mol-% (bevorzugt wenigstens 50 mol-% und besonders bevorzugt wenigstens 100 mol-%) des gesamten Anteils [Y$^1_a$·Y$^2_b$·O$_{x'}$]$_p$ ([Bi$_{a''}$Z$^2_{b''}$O$_{x''}$]$_{p''}$) der erfindungsgemäß geeigneten Multimetalloxidmassen V (Multimetalloxidmassen VI) in den erfindungsgemäß geeigneten Multimetalloxidmassen V (Multimetalloxidmassen VI) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung Y$^1_a$·Y$^2_b$·O$_{x'}$, [Bi$_{a''}$Z$^2_{b''}$O$_{x''}$] vorliegen, deren Größtdurchmesser im Bereich 1 nm bis 100 μm liegt.

Hinsichtlich der Formgebung gilt bezüglich Multimetalloxidmassen V-Katalysatoren das bei den Multimetalloxidmassen IV-Katalysatoren Gesagte.

Die Herstellung von Multimetalloxidmassen V-Aktivmassen ist z. B. in der EP-A 575 897 sowie in der DE-A 198 55 913 beschrieben.

Die vorstehend empfohlenen inerten Trägermaterialien kommen u.a. auch als inerte Materialien zur Verdünnung und/oder Abgrenzung der entsprechenden Katalysatorfestbetten, bzw. als deren schützende und/oder das Gasgemisch aufheizende Vorschüttung in Betracht.

Für die dritte Reaktionsstufe, die heterogen katalysierte Gasphasen-Partialoxidation von Acrolein zu Acrylsäure, kommen, wie bereits gesagt, prinzipiell alle Mo und V enthaltenden Multimetalloxidmassen als Aktivmassen für die benötigten Katalysatoren in Betracht, z. B. jene der DE-A 100 46 928.

Eine Vielzahl derselben, z. B. diejenigen der DE-A 198 15 281, lässt sich unter der allgemeinen Formel VII,

$$Mo_{12}V_aX^1_bX^2_cX^3_dX^4_eX^5_fX^6_gO_n \qquad (VII),$$

in der die Variablen folgende Bedeutung haben:

X$^1$ = W, Nb, Ta, Cr und/oder Ce,
X$^2$ = Cu, Ni, Co, Fe, Mn und/oder Z.n,
X$^3$ = Sb und/oder Bi,
X$^4$ = eines oder mehrere Alkalimetalle,
X$^5$ = eines oder mehrere Erdalkalimetalle,
X$^6$ = Si, Al, Ti und/oder Zr,

a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiede- nen Elemente in VII bestimmt wird,

subsumieren.

Erfindungsgemäß bevorzugte Ausführungsformen innerhalb der aktiven Multimetalloxide VII sind jene, die von den nachfolgenden Bedeutungen der Variablen der allgemeinen Formel VII erfasst werden:

X$^1$ = W, Nb, und/oder Cr,
X$^2$ = Cu, Ni, Co, und/oder Fe,
X$^3$ = Sb,
X$^4$ = Na und/oder K,

X$^5$ = Ca, Sr und/oder Ba,
X$^6$ = Si, Al, und/oder Ti,

a = 1,5 bis 5,
b = 0,5 bis 2,
c = 0,5 bis 3,
d = 0 bis 2,
e = 0 bis 0,2,
f = 0 bis 1 und

n = eine Zahl, die durch die Wertigkeit und Häufigkeit der von Sauerstoff verschiede- nen Elemente in VII bestimmt wird.

Erfindungsgemäß ganz besonders bevorzugte Multimetalloxide VII sind jedoch jene der allgemeinen Formel VIII,

$$Mo_{12}V_{a'}Y^1_{b'}Y^2_{c'}Y^5_f Y^6_{g'}O_{n'} \qquad (VIII),$$

mit

$Y^1$ = W und/oder Nb,
$Y^2$ = Cu und/oder Ni,
$Y^5$ = Ca und/oder Sr,
$Y^6$ = Si und/oder Al,

a' = 2 bis 4,
b' = 1 bis 1,5,
c' = 1 bis 3,
f = 0 bis 0,5
g' = 0 bis 8 und
n' = eine Zahl, die durch die Wertigkeit und Häufigkeit der von Sauerstoff verschiede- nen Elementen in VIII bestimmt wird.

Die erfindungsgemäß geeigneten Multimetalloxidaktivmassen (VII) sind in an sich bekannter, z. B. in der DE-A 43 35 973 oder in der EP-A 714 700 offenbarter, Weise erhältlich.

Prinzipiell können für den Schritt "Acrolein → Acrylsäure" (die dritte Reaktionsstufe) geeignete Multimetalloxidaktivmassen, insbesondere solche der allgemeinen Formel VII, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600˚C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemische aus Inertgas und reduzierenden Gasen wie $H_2$, $NH_3$, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen VII kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen VII kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150˚C erfolgt.

Die resultierenden Multimetalloxidmassen, insbesondere jene der allgemeinen Formel VII, können für die Acroleinoxidation sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm (z. B. 8,2 mm oder 5,1 mm) betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 2 909 671, der EP-A 293 859 oder aus der EP-A 714 700 bekannt ist.

Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 $\mu$m, bevorzugt im Bereich 50 bis 500 $\mu$m und besonders bevorzugt im Bereich 150 bis 250 $\mu$m liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z. B. Kugeln oder Hohlzylinder mit Splittauflage, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. D. h., geeignete Kugelgeometrien können Durchmesser von 8,2 mm oder von 5,1 mm aufweisen. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige für den Schritt "Acrolein → Acrylsäure" (die dritte Reaktionsstufe) zu verwendende Multimetalloxidaktivmassen sind ferner Massen der allgemeinen Formel IX,

$$[D]_p[E]_q \qquad (IX),$$

in der die Variablen folgende Bedeutung haben:

| | | |
|---|---|---|
| D | = | $Mo_{12}V_{a''}Z1_{b''}Z2_{c''}Z3_{d''}Z4_{e''}Z5_{f''}Z6_{g''}O_{x''}$, |
| E | = | $Z7_{12}Cu_{h''}H_{i''}O_{y''}$, |
| $Z^1$ | = | W, Nb, Ta, Cr und/oder Ce, |
| $Z^2$ | = | Cu, Ni, Co, Fe, Mn und/oder Zn, |
| $Z^3$ | = | Sb und/oder Bi, |
| $Z^4$ | = | Li, Na, K, Rb, Cs und/oder H |
| $Z^5$ | = | Mg, Ca, Sr und/oder Ba, |
| $Z^6$ | = | Si, Al, Ti und/oder Zr, |
| $Z^7$ | = | Mo, W, V, Nb und/oder Ta, vorzugsweise Mo und/oder W |

| | | |
|---|---|---|
| a" | = | 1 bis 8, |
| b" | = | 0,2 bis 5, |
| c" | = | 0 bis 23, |
| d" | = | 0 bis 50, |
| e" | = | 0 bis 2, |
| f" | = | 0 bis 5, |
| g" | = | 0 bis 50, |
| h" | = | 4 bis 30, |
| i" | = | 0 bis 20 und |
| x",y" | = | Zahlen, die durch die Wertigkeit und Häufigkeit der von Sauerstoff ver-schiedenen Element in IX bestimmt werden und |
| p,q | = | von Null verschiedene Zahlen, deren Verhältnis p/q 160:1 bis 1:1 beträgt, |

und die dadurch erhältlich sind, dass man eine Multimetalloxidmasse E

$$Z7_{12}Cu_{h''}H_{i''}O_{y''} \qquad (E),$$

in feinteiliger Form getrennt vorbildet (Ausgangsmasse 1) und anschließend die vorgebildete feste Ausgangsmasse 1 in eine wäßrige Lösung, eine wäßrige Suspension oder in ein feinteiliges Trockengemisch von Quellen der Elemente Mo, V, $Z^1$, $Z^2$, $Z^3$, $Z^4$, $Z^5$, $Z^6$, die die vorgenannten Elemente in der Stöchiometrie D

$$Mo_{12}V_{a''}Z^1_{b''}Z^2_{c''}Z^3_{d''}Z^4_{e''}Z^5_{f''}Z^6_{g''} \qquad (D),$$

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind die Multimetalloxidmassen IX, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen VI-Katalysatoren enthalten z. B. die EP-A 668 104, die DE-A 197 36 105, die DE-A 100 46 928, die DE-A 197 40 493 und die DE-A 195 28 646.

Hinsichtlich der Formgebung gilt bezüglich Multimetalloxidmassen IX-Katalysatoren das bei den Multimetalloxidmassen VII-Katalysatoren Gesagte.

Für den Schritt "Acrolein → Acrylsäure" in hervorragender Weise geeignete Multimetalloxidkatalysatoren sind auch jene der DE-A 198 15 281, insbesondere mit Multimetalloxidaktivmassen der allgemeinen Formel I dieser Schrift.

Vorteilhaft werden für den Schritt vom Propylen zum Acrolein Vollkatalysatorringe und für den Schritt vom Acrolein zur Acrylsäure Schalenkatalysatorringe verwendet.

Die Realisierung der zweiten Reaktionsstufe, die Partialoxidation von Propylen zu Acrolein, kann mit den beschriebenen Katalysatoren z. B. (ganz allgemein in Rohrbündelreaktoren durchgeführt werden; anwendungstechnisch zweckmäßig beläuft sich die im Rohrbündelbehälter untergebrachte Anzahl an Kontaktrohren auf wenigstens 5000, vorzugsweise auf wenigstens 10000, häufig 15000 bis 30000; eine Anzahl oberhalb von 40000 bildet eher die Ausnahme; innerhalb des Behälters sind die Kontaktrohre im Normalfall homogen verteilt angeordnet, wobei die Verteilung zweckmäßig so gewählt wird, dass der Abstand der zentrischen Innenachse von zueinander nächstliegenden Kontaktrohren (die sogenannte Kontaktrohrteilung) 35 bis 45 mm beträgt (vgl. z. B. EP-A 468 290); als Wärmeaustauschmittel eignen sich insbesondere fluide Temperiermedien; dies können Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle sein;) in einem Einzonen-Vielkontaktrohr-Festbettreaktor durchgeführt werden, wie ihn die DE-A 4 431 957 beschreibt. Dabei können Reaktionsgasgemisch 2 und Wärmeträger (Wärmeaustauschmittel) über den Reaktor betrachtet im Gleichstrom oder im Gegenstrom geführt werden.

Der Reaktionsdruck liegt üblicherweise im Bereich von 1 bis 3 bar und die Gesamtraumbelastung des Katalysatorfestbetts mit Reaktionsgas(ausgangs)gemisch 2 beträgt vorzugsweise 1500 bis 4000 bzw. 6000 Nl/l•h oder mehr. Die Propylenlast (die Propylenbelastung des Katalysatorfestbetts) beträgt typisch 90 bis 200 Nl/l•h oder bis 300 Nl/l·h oder mehr. Propylenlasten oberhalb von 135 Nl/l·h bzw. ≥ 140 Nl/l·h, oder ≥ 150 Nl/l·h, oder ≥ 160 Nl/l·h sind erfindungsgemäß besonders bevorzugt, da das erfindungsgemäße Reaktionsgasausgangsgemisch 2 sowie die erfindungsgemäße Verfahrensweise ein günstiges Heißpunktverhalten bedingt (alles Vorgenannte gilt auch unabhängig von der speziellen Wahl des Festbettreaktors).

Bevorzugt wird der Einzonen-Vielkontaktrohr-Festbettreaktor mit dem Beschickungsgasgemisch von oben angeströmt. Als Wärmeaustauschmittel wird zweckmäßig eine Salzschmelze, bevorzugt bestehend aus 60 Gew.-% Kaliumnitrat ($KNO_3$) und 40 Gew.-% Natriumnitrit ($NaNO_2$) oder aus 53 Gew.-% Kaliumnitrat ($KNO_3$) 40 Gew.-% Natriumnitrit ($NaNO_2$) und 7 Gew.-% Natriumnitrat ($NaNO_3$), eingesetzt.

Über den Reaktor betrachtet können, wie bereits gesagt, Salzschmelze und Reaktionsgasgemisch 2 sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt mit Katalysator zu beschicken (für die Anströmung von unten nach oben wird die Beschickungsabfolge in zweckmäßiger Weise umgekehrt):

- zunächst auf einer Länge von 40 bis 80 bzw. bis 60 % der Kontaktrohrlänge entweder nur Katalysator, oder ein Gemisch aus Katalysator und Inertmaterial, wobei letzteres, bezogen auf die Mischung, einen Gewichtsanteil von bis zu 20 Gew.-% ausmacht (Abschnitt C);

- daran anschließend auf einer Länge von 20 bis 50 bzw. bis 40 % der Gesamtrohrlänge entweder nur Katalysator, oder ein Gemisch aus Katalysator und Inertmaterial, wobei letzteres, bezogen auf die. Mischung, einen Gewichtsanteil von bis zu 40 Gew.-% ausmacht (Abschnitt B); und

- abschließend auf einer Länge von 10 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt.

Vorgenannte Beschickungsvariante ist insbesondere dann zweckmäßig, wenn als Katalysatoren solche gemäß Beispiel 1 der DE-A 100 46 957 oder gemäß Beispiel 3 der DE-A 100 46 957 und als Inertmaterial Ringe aus Steatit mit der Geometrie 7 mm x 7 mm x 4 mm (Außendurchmesser x Höhe x Innendurchmesser) verwendet werden. Hinsichtlich der Salzbadtemperatur gilt das in der DE-A 4 431 957 Gesagte.

Die Realisierung der zweiten Reaktionsstufe, die erfindungsgemäße Partialoxidation vom Propylen zum Acrolein (und gegebenenfalls Acrylsäure), kann mit den beschriebenen Katalysatoren aber auch z. B. in einem Zweizonen-Vielkontaktrohr-Festbettreaktor durchgeführt werden, wie es die DE-A 199 10 506 beschreibt. Erfindungsgemäß vorteilhaft erfolgt die erfindungsgemäße Partialoxidation von Propen zu Acrolein wie in der EP-A 1159244 und ganz besonders bevorzugt wie in der WO 04/085363 sowie in der WO 04/085362 beschrieben, jedoch unter Beachtung der erfindungsgemäßen Randbedingungen für $U^p$.

Die Schriften EP-A 1159244, WO 04/085363 und WO 04/085362 werden als integraler Bestandteil dieser Schrift betrachtet.

D. h., die zweite Reaktionsstufe, die Partialoxidation des Propylens zu Acrolein, lässt sich besonders vorteilhaft an einem Katalysatorfestbett mit erhöhter Propylenbelastung durchführen, das wenigstens zwei Temperaturzonen aufweist.

D.h., eine vorteilhafte Ausführungsform der zweiten Reaktionsstufe ist ein Verfahren, bei dem man das Reaktionsgasausgangsgemisch 2 mit der Maßgabe über (durch) ein Katalysatorfestbett 2, dessen Aktivmasse wenigstens ein die Elemente Mo, Fe und Bi enthaltendes Multimetalloxid ist, führt, dass der Propylenumsatz bei einmaligem Durchgang den erfindungsgemäß vorgegebenen Wert für $U^P$ nicht überschreitet (die Selektivität der Acroleinbildung sowie der Acrylsäurenebenproduktbildung wird dabei zusammengenommen regelmäßig ≥ 90 mol-% betragen) und das dadurch gekennzeichnet ist, dass

a) die Belastung des Katalysatorfestbetts 2 mit dem im Reaktionsgasausgangsgemisch 2 enthaltenen Propen ≥ 140 bzw ≥ 160 NI Propen/I Katalysatorfestbett 2 • h beträgt,

b) das Katalysatorfestbett 2 aus einem in zwei räumlich aufeinanderfolgenden Reaktionszonen A*, B* angeordneten Katalysatorfestbett 2 besteht, wobei die Temperatur der Reaktionszone A* 300 bis 390°C und die Temperatur der Reaktionszone B* 305 bis 420°C beträgt und gleichzeitig wenigstens 5°C oberhalb der Temperatur der Reaktionszone A* liegt,

c) das Reaktionsgasausgangsgemisch 2 die Reaktionszonen A*, B* in der zeitlichen Abfolge "erst A*", "dann B*" durchströmt und

d) sich die Reaktionszone A* bis zu einem Umsatz des Propens von 40 bis 80 % des in der zweiten Reaktionsstufe erfindungsgemäß angestrebten Wertes für $U^p$ erstreckt:

Im übrigen wird auf die EP-A 1159244 verwiesen.

D. h. aber auch, eine ganz besonders bevorzugte Ausführungsform der erfindungsgemäßen zweiten Reaktionsstufe ist ein Verfahren, bei dem man das Reaktionsgasausgangsgemisch 2 mit der Maßgabe über ein Katalysatorfestbett 2, dessen Aktivmasse wenigstens ein die Elemente Mo, Fe und Bi enthaltendes Multimetalloxid ist, führt, dass

- das Katalysatorfestbett 2 in zwei räumlich aufeinanderfolgenden Temperaturzonen A, B angeordnet ist,

- sowohl die Temperatur der Temperaturzone A als auch die Temperatur der Temperaturzone B eine Temperatur im Bereich von 290 bis 380°C ist,

- das Katalysatorfestbett 2 aus wenigstens zwei räumlich aufeinander folgenden Katalysatorfestbettzonen besteht, wobei die volumenspezifische Aktivität innerhalb einer Katalysatorfestbettzone im wesentlichen konstant ist und in Strömungsrichtung des Reaktionsgasgemisches 2 beim Übergang von einer Katalysatorfestbettzone in eine andere Katalysatorfestbettzone sprunghaft zunimmt,

- sich die Temperaturzone A bis zu einem Umsatz des Propylens von 40 bis 80 % des in der zweiten Reaktionsstufe erfindungsgemäß angestrebten Wertes für $U^P$ erstreckt,

- bei einmaligem Durchgang des Reaktionsgasausgangsgemisches 2 durch das gesamte Katalysatorfestbett der Propenumsatz den Wert $U^p$ hat und die Selektivität der Acroleinbildung, bezogen auf umgesetztes Propen, ≥ 90 mol.-% beträgt,

- die zeitliche Abfolge, in der das Reaktionsgasgemisch 2 die Temperaturzonen A, B durchströmt, der alphabetischen Abfolge der Temperaturzonen entspricht,

- die Belastung des Katalysatorfestbetts 2 mit dem im Reaktionsgasausgangsgemisch 2 enthaltenen Propylen $\geq 90$ NI Propylen/I Katalysatorfestbett 2 • h beträgt, und

- die Differenz $T^{maxA - maxB}$, gebildet aus der höchsten Temperatur $T^{maxA}$, die das Reaktionsgasgemisch 2 innerhalb der Temperaturzone A aufweist, und der höchsten Temperatur $T^{maxB}$, die das Reaktionsgasgemisch 2 innerhalb der Temperaturzone B aufweist, $\geq 0°C$ beträgt,
  und das zusätzlich dadurch gekennzeichnet ist, dass der Übergang von der Temperaturzone A in die Temperaturzone B im Katalysatorfestbett 2 nicht mit einem Übergang von einer Katalysatorfestbettzone in eine andere Katalysatorfestbettzone zusammenfällt.

Nähere Angaben zu dieser Verfahrensweise finden sich in der WO 04/085362, die integraler Bestandteil dieser Schrift ist, sowie im weiteren Verlauf dieser Schrift bei der Beschreibung der besonders bevorzugten simultanen Gestaltung der zweiten und dritten Reaktionsstufe des erfindungsgemäßen Verfahrens.

Die Durchführung der dritten Reaktionsstufe, nämlich die Partialoxidation vom Acrolein zur Acrylsäure, kann mit den beschriebenen Katalysatoren (ganz allgemein in Rohrbündelreaktoren durchgeführt werden; anwendungstechnisch zweckmäßig beläuft sich die im Rohrbündelreaktor untergebrachte Anzahl an Kontaktrohren auf wenigstens 5000, vorzugsweise auf wenigstens 10000, häufig 15000 bis 30000; eine Anzahl oberhalb von 40000 bildet eher die Ausnahme; innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z. B. EP-A 468 290); als Wärmetauschmittel eignen sich insbesondere fluide Temperiermedien; dies können Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedene Metalle sein; zweckmäßig werden sowohl die zweite als auch die dritte Reaktionsstufe in solchen Rohrbündelreaktoren betrieben) z. B. in einem Einzonen-Vielkontaktrohr-Festbettreaktor durchgeführt werden, wie es die DE-A 44 31 949 beschreibt. Dabei können Reaktiongsgasgemisch 3 und Wärmeträger über den Reaktor betrachtet im Gleichstrom geführt werden. Erfindungsgemäß wird das Produktgasgemisch 2 der vorausgehenden erfindungsgemäßen Propylenpartialoxidation zu Acrolein grundsätzlich als solches (gegebenenfalls nach erfolgter Zwischenkühlung (diese kann indirekt oder direkt durch z. B. Sekundärluftzugabe erfolgen) desselben), d.h., ohne Nebenkomponentenabtrennung, in die dritte Reaktionsstufe, d.h. in die Acroleinpartialoxidation geführt.

Der für den dritten Schritt, die Acroleinpartialoxidation, benötigte molekulare Sauerstoff kann (dabei) schon im Reaktionsgasausgangsgemisch 2 für die erfindungsgemäße Reaktionsstufe 2 (die Propylenpartialoxidation zum Acrolein) enthalten sein (dies ist erfindungsgemäß bevorzugt). Er kann aber auch teilweise oder vollständig dem Produktgasgemisch 2 der ersten Reaktionsstufe unmittelbar zugegeben werden (dies erfolgt vorzugsweise als (Sekundär)Luft, kann jedoch auch in Form von reinem Sauerstoff oder von Gemischen aus Inertgas oder Sauerstoff erfolgen).

Wie in der zweiten Reaktionsstufe liegt auch in der dritten Reaktionsstufe der Reaktionsdruck üblicherweise im Bereich von 1 bis 3 bar, und die Gesamtraumbelastung des Katalysatorfestbetts 3 mit Reaktionsgas(ausgangs) gemisch 3 liegt vorzugsweise bei 1500 bis 4000 bzw. 6000 NI/I·h oder mehr. Die Acroleinlast (die Acroleinbelastung des Katalysatorfestbetts 3) beträgt typisch 90 bis 190 NI/I·h, oder bis 290 NI/I·h oder mehr Acroleinlasten oberhalb von 135 NI/I·h, bzw. $\geq 140$ NI/I·h, oder $\geq 150$ NI/I·h, oder $\geq 160$ NI/I·h sind besonders bevorzugt, da das erfindungsgemäße Beisein von Propan im Reaktionsgasausgangsgemisch 3 ebenfalls ein günstiges Heißpunktverhalten bedingt.

Bevorzugt wird der Einzonen-Vielkontaktrohr-Festbettreaktor mit dem Beschickungsgasgemisch 3 ebenfalls von oben angeströmt. Als Wärmeaustauschmittel wird auch in der zweiten Stufe in zweckmäßiger Weise eine Salzschmelze, bevorzugt aus 60 Gew.-% Kaliumnitrat ($KNO_3$) und 40 Gew.-% Natriumnitrit ($NaNO_2$) oder aus 53 Gew.-% Kaliumnitrat ($KNO_3$) 40 Gew.-% Natriumnitrit ($NaNO_2$) und 7 Gew.-% Natriumnitrat ($Na-NO_3$) bestehend, eingesetzt. Über den Reaktor betrachtet können, wie bereits gesagt, Salzschmelze und Reaktionsgasgemisch 3 sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt zu beschicken:

- zunächst auf einer Länge von 50 bis 80 bzw. bis 70 % der Kontaktrohrlänge entweder nur Katalysator oder ein Gemisch aus Katalysator und Inertmaterial, wobei letzteres, bezogen auf die Mischung, einen Gewichtsanteil von bis zu 20 Gew.-% ausmacht (Abschnitt C);

- daran anschließend auf einer Länge von 20 bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 50 bzw. bis zu 40 Gew.-% ausmacht (Abschnitt B); und

- abschließend auf einer Länge von 5 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt. Wie ganz allgemein bei der heterogen katalysierten Gasphasenpartialoxidation von Acrolein zu Acrylsäure (insbesondere bei hohen Acroleinbelastungen des Katalysatorfestbetts 3 und hohen Wasserdampfgehalten des Beschickungsgasgemischs 3), kann der Abschnitt B auch aus zwei aufeinanderfolgenden Katalysatorverdünnungen bestehen (zum Zweck der Minimierung von Heißpunkttempatur und Heißpunkttemperatursensitivität). Von unten nach oben zunächst mit bis zu 20 Gew.-% Inertmaterial und daran anschließend mit > 20 Gew.-% bis 50 bzw. bis 40 Gew.-% Inertmaterial. Der Abschnitt C ist dann bevorzugt unverdünnt.

Für eine Anströmung der Kontaktrohre von unten nach oben, wird die Kontaktrohrbeschickung in zweckmäßigerweise umgekehrt.

Vorgenannte Beschickungsvariante ist insbesondere dann zweckmäßig, wenn als Katalysatoren solche gemäß Herstellungsbeispiel 5 der DE-A 100 46 928 oder solche gemäß DE-A 198 15 281 und als Inertmaterial Ringe aus Steatit mit der Geometrie 7 mm x 7 mm x 4 mm oder 7 mm x 7 mm x 3 mm (jeweils Außendurchmesser x Höhe x Innendurchmessser) verwendet werden. Hinsichtlich der Salzbadtemperatur gilt das in der DE-A 443 19 49 Gesagte. Sie wird in der Regel so gewählt, dass der erfindungsgemäß angestrebte Acroleinumsatz $U^A$ bei einfachem Durchgang erreicht wird.

Die Durchführung der Partialoxidation vom Acrolein zur Acrylsäure, kann mit den beschriebenen Katalysatoren aber auch z. B. in einem Zweizonen-Vielkontaktrohr-Festbettreaktor durchgeführt werden, wie es die DE-199 10 508 beschreibt. Für den Acroleinumsatz gilt das oben Genannte. Auch in diesem Fall wird man zur Erzeugung des Beschickungsgasgemisches (Reaktionsgasausgangsgemischs 3) unmittelbar das Produktgasgemisch 2 (gegebenenfalls nach erfolgter indirekter oder direkter (z. B. durch Zufuhr von Sekundärluft) Zwischenkühlung desselben) verwenden (wie es vorstehend bereits beschrieben wurde). Der für die Acroleinpartialoxidation benötigte Sauerstoff wird vorzugsweise als Luft (gegebenenfalls aber auch als reiner molekularer Sauerstoff oder als Gemisch aus molekularem Sauerstoff und einem Inertgas) zugesetzt und z. B. dem Produktgasgemisch 2 des ersten Schritts der zweistufigen Partialoxidation (Propylen → Acrolein) unmittelbar zugegeben. Er kann aber auch, wie bereits beschrieben, bereits im Reaktionsgasausgangsgemisch 2 für die zweite Reaktionsstufe enthalten sein, was erfindungsgemäß vorteilhaft ist.

Bei einer wie beschrieben zweistufigen Partialoxidation von Propylen zu Acrylsäure mit unmittelbarer Weiterverwendung des Produktgasgemisches des ersten Schritts der Partialoxidation zur Beschickung des zweiten Schritts der Partialoxidation, wird man in der Regel zwei Einzonen-Vielkontaktrohr-Festbettreaktoren (bei hoher Reaktandenbelastung des Katalysatorbetts ist, wie ganz allgemein gültig, eine Gleichstromfahrweise zwischen Reaktionsgas und Salzbad (Wärmeträger) über den Rohrbündelreaktor betrachtet bevorzugt) oder zwei Zweizonen-Vielkontaktrohr-Festbettreaktoren hintereinanderschalten. Eine gemischte Hintereinanderschaltung (Einzonen/Zweizonen oder umgekehrt) ist auch möglich. "Einzonen" in der Propen → Acrolein Stufe und "Zweizonen" in der Acrolein → Acrylsäure Stufe ist günstig.

Zwischen den Reaktoren kann sich ein Zwischenkühler befinden, der gegebenenfalls Inertschüttungen enthalten kann, die eine Filterfunktion ausüben können. Die Salzbadtemperatur von Vielkontaktrohrreaktoren für den ersten Schritt der zweistufigen Partialoxidation von Propylen zu Acrylsäure beträgt in der Regel 300 bis 400˚C. Die Salzbadtemperatur von Vielkontaktrohrreaktoren für den zweiten Schritt der Partialoxidation von Propylen zu Acrylsäure, die Partialoxidation von Acrolein zu Acrylsäure, beträgt meist 200 bis 350˚C. Außerdem werden die Wärmeaustauschmittel (bevorzugt Salzschmelzen) normalerweise in solchen Mengen durch die relevanten Vielkontaktrohr-Festbettreaktoren geführt, dass der Unterschied zwischen ihrer Eingangs- und ihrer Ausgangstemperatur in der Regel ≤ 5˚C beträgt.

Es sei auch nochmals erwähnt, dass ein Teil des Reaktionsgasausgangsgemischs 2 für den ersten Schritt ("Propylen → Acrolein") aus der Partialoxidation kommendes Oxidationskreisgas (Restgas) sein kann.

Hierbei handelt es sich, wie bereits gesagt, um ein molekularen Sauerstoff enthaltendes Gas, das nach der Zielproduktabtrennung (Acrolein- und/oder Acrylsäureabtrennung) vom Produktgasgemisch der Partialoxidation verbleibt und teilweise als inertes Verdünnungsgas in die Beschickung für den ersten und/oder gegebenenfalls zweiten Schritt der Partialoxidation von Propylen zu Acrolein und/oder Acrylsäure rückgeführt werden kann.

Erfindungsgemäß bevorzugt wird solches Propan, molekularen Sauerstoff und nicht umgesetztes Propylen enthaltendes Oxidationskreisgas jedoch ausschließlich in die als Propylenquelle fungierende erste Reaktionsstufe (z. B. die heterogen katalysierte Propandehydrierung) rückgeführt.

Insgesamt bildet ein Rohrbündelreaktor innerhalb dessen sich die Katalysatorbeschickung längs der einzelnen Kontaktrohre mit Beendigung des ersten Reaktionsschrittes entsprechend ändert (derartige zweistufige Propylenpartialoxidationen im sogenannten "Single-Reactor" lehren z. B. die EP-A 91 13 13, die EP-A 97 98 13, die EP-A 99 06 36 und die DE-A 28 30 765) die einfachste Realisierungsform der beiden Oxidationsstufen für die beiden Schritte der Partialoxidation von Propylen zu Acrylsäure. Gegebenenfalls wird dabei die Beschickung der Kontaktrohre mit Katalysator durch eine Inertschüttung unterbrochen.

Vorzugsweise werden die beiden Oxidationsstufen jedoch in Form zweier hintereinander geschalteter Rohrbündelsysteme realisiert. Diese können sich in einem Reaktor befinden, wobei der Übergang von einem Rohrbündel zum anderen Rohrbündel von einer nicht im Kontaktrohr untergebrachten (zweckmäßigerweise begehbaren) Schüttung aus Inertmaterial gebildet wird. Während die Kontaktrohre in der Regel von einem Wärmeträger umspült werden, erreicht dieser eine wie vorstehend beschrieben angebrachte Inertschüttung nicht. Mit Vorteil werden daher die beiden Kontaktrohrbündel in räumlich voneinander getrennten Reaktoren untergebracht. In der Regel befindet sich dabei zwischen den beiden Rohrbündelreaktoren ein Zwischenkühler, um eine gegebenenfalls erfolgende Acroleinnachverbrennung im Produktgasgemisch 2, das die zweite Reaktionsstufe verlässt, zu mindern.

Die Reaktionstemperatur in der zweiten Reaktionsstufe (Propylen → Acrolein) liegt in der Regel bei 300 bis 450˚C, bevorzugt bei 320 bis 390˚C. Die Reaktionstemperatur in der dritten Reaktionsstufe (Acrolein → Acrylsäure) liegt in der Regel bei 200 bis 370˚C, häufig bei 220 bis 330˚C. Der Reaktionsdruck beträgt in beiden Oxidationsstufen zweckmäßig 0,5 bis 5, vorteilhaft 1 bis 3 bar. Die Belastung (NI/I·h) der Oxidationskatalysatoren mit Reaktionsgas beträgt in beiden Reaktionsstufen häufig 1500 bis 2500 NI/I·h bzw. bis 4000 NI/I·h. Die Belastung mit Propylen kann dabei in der Reaktionsstufe 2 bei 100 bis 200 oder 300 und mehr NI/I·h liegen.

Prinzipiell können die beiden Oxidationsstufen beim erfindungsgemäßen Verfahren so gestaltet werden, wie es z. B. in der DE-A 198 37 517, der DE-A 199 10 506, der DE-A 199 10 508 sowie der DE-A 198 37 519 beschrieben ist. In beiden Reaktionsstufen wirkt sich dabei ein Überschuss an molekularem Sauerstoff relativ zur reaktionsstöchiometrisch erforderlichen Menge in der Regel vorteilhaft auf die Kinetik der jeweiligen Gasphasenpartialoxidation aus.

Natürlich kann bei einer Ausführungsform einer erfindungsgemäßen zweistufigen Partialoxidation von Propylen zu Acrylsäure in Gestalt zweier hintereinandergeschalteter Oxidationsstufen aus dem das die erste Oxidationsstufe verlassenden Produktgasgemisch in selbigem enthaltenes, in der ersten Oxidationsstufe als Nebenprodukt entstandenes, Kohlenoxid und Wasserdampf bei Bedarf vor der Weiterleitung in die zweite Oxidationsstufe teilweise oder vollständig abgetrennt werden. Vorzugsweise wird man erfindungsgemäß eine Verfahrensweise wählen, die solch einer Abtrennung nicht vorsieht.

Als Quellen für eine zwischen beiden Oxidationsstufen durchgeführte Sauerstoffzwischeneinspeisung kommen, wie bereits gesagt, neben Luft (bevorzugt) sowohl reiner molekularer Sauerstoff als auch mit Inertgas wie $CO_2$, CO, Edelgasen, $N_2$ und/oder gesättigten Kohlenwasserstoffen verdünnter molekularer Sauerstoff in Betracht. Aber auch Stickoxide können an dieser Stelle als Sauerstoffquelle hinzugefügt werden.

Durch Zudosieren von z. B. kalter Luft zu heißem Produktgasgemisch 2 kann im Rahmen des erfindungsgemäßen Verfahrens auch auf direktem Weg eine Abkühlung desselben bewirkt werden, bevor es als Bestandteil eines Reaktionsgasausgangsgemisch 3 weiterverwendet wird.

Erfindungsgemäß vorteilhaft erfolgt die Partialoxidation von Acrolein zu Acrylsäure wie in der EP-A 11 59 246, und ganz besonders bevorzugt wie in der WO 04/085365 sowie in der WO 04/085370 beschrieben. Erfindungsgemäß bevorzugt wird dabei als Acrolein enthaltendes Reaktionsgasausgangsgemisch 3 das Produktgasgemisch einer erfindungsgemäßen Partialoxidation von Propylen zu Acrolein verwendet, das gegebenenfalls mit soviel Sekundärluft ergänzt wurde, dass das Verhältnis von molekularem Sauerstoff zu Acrolein in dem resultierenden Reaktionsgasausgangsgemisch 3 in jedem Fall 0,5 bis 1,5 beträgt. Die Schriften EP-A 1159246, WO 04/08536 und WO 04/085370 werden als integraler Bestandteil dieser Schrift betrachtet.

D. h., die erfindungsgemäße Partialoxidation von Acrolein zu Acrylsäure lässt sich vorteilhaft an einem Katalysatorfestbett 3 mit erhöhter Acroleinbelastung durchführen, das wenigstens zwei Temperaturzonen aufweist.

D. h., eine vorteilhafte Ausführungsform der erfindungsgemäßen Partialoxidation des Acroleins zu Acrylsäure (der Reaktionsstufe 3) ist ein Verfahren, bei dem man das Reaktionsgasausgangsgemisch 3 mit der Maßgabe über (durch) ein Katalysatorfestbett 3, dessen Aktivmasse wenigstens ein die Elemente Mo und V enthaltendes Multimetalloxid ist, führt, dass der Acroleinumsatz bei einmaligem Durchgang den angestrebten Wert für $U^A$ erreicht (die damit einhergehende Selektivität der Acrylsäurebildung wird regelmäßig ≥ 90 mol-% betragen) und das dadurch gekennzeichnet ist, dass

a) die Belastung des Katalysatorfestbetts 3 mit dem im Reaktionsgasausgangsgemisch 3 enthaltenen Acrolein ≥ 130 bzw. ≥ 150 NI Acrolein/I Katalysatorfestbett 3 · h beträgt,
b) das Katalysatorfestbett 3 aus einem in zwei räumlich aufeinanderfolgenden Reaktionszonen C*, D* angeordneten Katalysatorfestbett 3 besteht, wobei die Temperatur der Reaktionszone C* 230 bis 270˚C und die Temperatur der Reaktionszone D* 250 bis 300˚C beträgt und gleichzeitig wenigstens 5˚C oberhalb der Temperatur der Reaktionszone C* liegt,
c) das Reaktionsgasausgangsgemisch 3 die Reaktionszonen C*, D* in der zeitlichen Abfolge "erst C*", "dann D*" durchströmt und
d) sich die Reaktionszone C* bis zu einem Umsatz des Acroleins von 55 bis 85 mol-% des in der dritten Reaktionsstufe erfindungsgemäß angestrebten Wertes $U^A$ erstreckt.

Im übrigen wird auf die EP-A 11 59 246 verwiesen.

D. h. aber auch, eine ganz besonders bevorzugte Ausführungsform der erfindungsgemäßen Partialoxidation des Acroleins zu Acrylsäure ist ein Verfahren, bei dem man das Reaktionsgasausgangsgemisch 3 mit der Maßgabe über ein Katalysatorfestbett 3, dessen Aktivmasse wenigstens ein die Elemente Mo und V enthaltendes Multimetalloxid ist, führt, dass

- das Katalysatorfestbett 3 in zwei räumlich aufeinanderfolgenden Temperaturzonen C, D angeordnet ist,

- sowohl die Temperatur der Temperaturzone C als auch die Temperatur der Temperaturzone D eine Temperatur im Bereich von 230 bis 320˚C ist,

- das Katalysatorfestbett 3 aus wenigstens zwei räumlich aufeinanderfolgenden Katalysatorfestbettzonen besteht, wobei die volumenspezifische Aktivität innerhalb einer Katalysatorfestbettzone im wesentlichen konstant ist und in Strömungsrichtung des Reaktionsgasgemisches 3 beim Übergang von einer Katalysatorfestbettzone in eine andere Katalysatorfestbettzone sprunghaft zunimmt,

- sich die Temperaturzone C bis zu einem Umsatz des Acroleins von 45 bis 85 % des in der dritten Reaktionsstufe erfindungsgemäß angestrebten Wertes für $U^A$ erstreckt,

- bei einmaligem Durchgang des Reaktionsgasausgangsgemisches 3 durch das gesamte Katalysatorfestbett 2 der Acroleinumsatz den Wert $U^A$ hat und die Selektivität der Acrylsäurebildung, bezogen auf umgesetztes Acrolein, $\geq$ 90 mol-% beträgt,

- die zeitliche Abfolge, in der das Reaktionsgasgemisch 3 die Temperaturzonen C, D durchströmt, der alphabetischen Abfolge der Temperaturzonen entspricht,

- die Belastung des Katalysatorfestbetts 3 mit dem im Reaktionsgasausgangsgemisch 3 enthaltenen Acrolein $\geq$ 70 Nl Acrolein/l Katalysatorfestbett 3 · h beträgt, und

- die Differenz $T^{maxC} - T^{maxD}$, gebildet aus der höchsten Temperatur $T^{maxC}$, die das Reaktionsgasgemisch 3 innerhalb der Temperaturzone C aufweist, und der höchsten Temperatur $T^{maxD}$, die das Reaktionsgasgemisch 3 innerhalb der Temperaturzone D aufweist, $\geq$ 0˚C beträgt,

und das zusätzlich dadurch gekennzeichnet ist, dass der Übergang von der Temperaturzone C in die Temperaturzone D in dem Katalysatorfestbett 3 nicht mit einem Übergang von einer Katalysatorfestbettzone in eine andere Katalysatorfestbettzone zusammenfällt.

Nähere Angaben zu dieser Verfahrensweise finden sich in der WO 04/085370, die integraler Bestandteil dieser Schrift ist, sowie im weiteren Verlauf dieser Schrift bei der Beschreibung der besonders bevorzugten zweistufigen Partialoxidation von Propylen zu Acrylsäure.

Eine solche besonders bevorzugte zweistufige Partialoxidation von Propylen zu Acrylsäure kann vorteilhaft wie in der EP-A 1159248 sowie in der WO 04/085367 beschrieben durchgeführt werden. Beide Schriften bilden einen integralen Bestandteil dieser Schrift).

D. h., man wird zunächst ein Reaktionsgasausgangsgemisch 2 in der zweiten Reaktionsstufe mit der Maßgabe über (durch) ein Katalysatorfestbett 2, dessen Aktivmasse wenigstens ein die Elemente Mo, Fe und Bi enthaltendes Multimetalloxid ist, führen, dass der Propylenumsatz bei einmaligem Durchgang den erfindungsgemäß angestrebten Wert für $U^P$ erreicht und die damit einhergehende Selektivität der Acroleinbildung sowie der Acrylsäurenebenprodukt-bildung zusammengenommen $\geq$ 90 mol-% betragen, die Temperatur des die zweite Reaktionsstufe verlassenden Produktgasgemischs 2 durch indirekte und/oder direkte Kühlung gegebenenfalls verringern und dem Produktgasgemisch 2 gegebenenfalls molekularen Sauerstoff und/oder Inertgas zugeben, und danach als Acrolein, molekularen Sauerstoff und wenigstens ein Inertgas enthaltendes Reaktionsgasausgangsgemisch 3, das den molekularen Sauerstoff und das Acrolein in einem molaren Verhältnis $O_2:C_3H_4O \geq 0{,}5$ enthält, in einer dritten Reaktionsstufe mit der Maßgabe über (durch) ein Katalysatorfestbett 3, dessen Aktivmasse wenigstens ein Molybdän und Vanadin enthaltendes Multimetalloxid ist, führen, dass der Acroleinumsatz bei einmaligem Durchgang den erfindungsgemäß angestrebten Wert für $U^A$ erreicht und die Selektivität der über beide Reaktionsstufen bilanzierten Acrylsäurebildung, bezogen auf umgesetztes Propylen, $\geq$ 80 mol-% beträgt und dabei weiterhin so verfahren, dass

a) die Belastung des Katalysatorfestbetts 2 mit dem im Reaktionsgasausgangsgemisch 2 enthaltenen Propylen $\geq$ 140 bzw. $\geq$ 160 Nl Propylen/l Katalysatorfestbett 2 · h beträgt,
b) das Katalysatorfestbett 2 aus einem in zwei räumlich aufeinanderfolgenden Reaktionszonen A*, B* angeordneten Katalysatorfestbett 2 besteht, wobei die Temperatur der Reaktionszone A* 300 bis 390˚C und die Temperatur der Reaktionszone B* 305 bis 420˚C beträgt und gleichzeitig wenigstens 5˚C oberhalb der Temperatur der Reaktionszone A* liegt,

c) das Reaktionsgasausgangsgemisch 2 die Reaktionszonen A*, B* in der zeitlichen Abfolge "erst A*", "dann B*" durchströmt,

d) die Reaktionszone A* sich bis zu einem Umsatz des Propens von 40 bis 80 % des in der ersten Reaktionsstufe erfindungsgemäß angestrebten Wertes für $U^P$ erstreckt,

e) die Belastung des Katalysatorfestbetts 3 mit dem im Reaktionsgasausgangsgemisch 3 enthaltenen Acrolein ≥ 120 bzw. ≥ 140 Nl Acrolein/l Katalysatorfestbett 3 · h beträgt,

f) das Katalysatorfestbett 3 aus einem in zwei räumlich aufeinanderfolgenden Reaktionszonen C*, D* angeordneten Katalysatorfestbett 3 besteht, wobei die Temperatur der Reaktionszone C* 230 bis 270˚C und die Temperatur der Reaktionszone D* 250 bis 300˚C beträgt und gleichzeitig wenigstens 10˚C oberhalb der Reaktionszone C* liegt,

g) das Reaktionsgasausgangsgemisch 3 die Rektionszonen C*, D* in der zeitlichen Abfolge "erst C*", "dann D*" durchströmt und

h) sich die Reaktionszone C* bis zu einem Umsatz des Acroleins von 55 bis 85 % des in der ersten Reaktionsstufe für $U^A$ erfindungsgemäß angestrebten Wertes erstreckt.

Im übrigen wird auf die EP-A 11 59 248 verwiesen.

Besonders bevorzugt wird man sie jedoch gemäß der WO 04/085369 durchführen, die integraler Bestandteil dieser Schrift ist.

D. h., man wird zunächst ein erfindungsgemäßes Reaktionsgasausgangsgemisch 2 in einer zweiten Reaktionsstufe mit der Maßgabe über ein Katalysatorfestbett 2, dessen Aktivmasse wenigstens ein die Elemente Mo, Fe und Bi enthaltendes Multimetalloxid ist, führen, dass

- das Katalysatorfestbett 2 in zwei räumlich aufeinanderfolgenden Temperaturzonen A, B angeordnet ist,
- sowohl die Temperatur der Temperaturzone A als auch die Temperatur der Temperaturzone B eine Temperatur im Bereich von 290 bis 380˚C ist,
- das Katalysatorfestbett 2 aus wenigstens zwei räumlich aufeinander folgenden Katalysatorfestbettzonen besteht, wobei die volumenspezifische Aktivität innerhalb einer Katalysatorfestbettzone im wesentlichen konstant ist und in Strömungsrichtung des Reaktionsgasgemisches 2 beim Übergang von einer Katalysatorfestbettzone in eine andere Katalysatorfestbettzone sprunghaft zunimmt,
- sich die Temperaturzone A bis zu einem Umsatz des Propens von 40 bis 80 mol-% des in der zweiten Reaktionsstufe erfindungsgemäß angestrebten Wertes für $U^p$ erstreckt,
- bei einmaligem Durchgang des Reaktionsgasausgangsgemisches 2 durch das gesamte Katalysatorfestbett 2 der Propenumsatz den erfindungsgemäß angestrebten Wert für $U^p$ hat und die Selektivität der Acroleinbildung sowie der Acrylsäurenebenproduktbildung zusammengenommen und bezogen auf umgesetztes Propen ≥ 90 mol-% beträgt,
- die zeitliche Abfolge, in der das Reaktionsgasgemisch 2 die Temperaturzonen A, B durchströmt, der, alphabetischen Abfolge der Temperaturzonen A, B entspricht,
- die Belastung des Katalysatorfestbetts 2 mit dem im Reaktionsgasausgangsgemisch 2 enthaltenen Propen ≥ 90 Nl Propen/l Katalysatorfestbett 2 · h beträgt, und
- die Differenz $T^{maxA}$ - $T^{maxB}$, gebildet aus der höchsten Temperatur $T^{maxA}$, die das Reaktionsgasgemisch 2 innerhalb der Temperaturzone A aufweist, und der höchsten Temperatur $7^{maxB}$, die das Reaktionsgasgemisch 2 innerhalb der Temperaturzone B aufweist, ≥ 0˚C beträgt,

danach die Temperatur des die zweite Reaktionsstufe verlassenden Produktgasgemisches 2 durch Kühlung gegebenenfalls verringern und dem Produktgasgemisch 2 gegebenenfalls molekularen Sauerstoff und/oder Inertgas, vorzugsweise gegebenenfalls Luft, zugeben, und es anschließend als Acrolein, molekularen Sauerstoff und wenigstens ein Inertgas enthaltendes Reaktionsgasausgangsgemisch 3, das den molekularen Sauerstoff und das Acrolein in einem molaren Verhältnis $O_2{:}C_3H_4O \geq 0{,}5$ enthält, in einer dritten Reaktionsstufe mit der Maßgabe über ein Katalysatorfestbett 3, dessen Aktivmasse wenigstens ein die Elemente Mo und V enthaltendes Multimetalloxid- ist, führen, dass

- das Katalysatorfestbett 3 in zwei räumlich aufeinanderfolgenden Temperaturzonen C, D angeordnet ist,
- sowohl die Temperatur der Temperaturzone C als auch die Temperatur der Temperaturzone D eine Temperatur im Bereich von 230 bis 320˚C ist,
- das Katalysatorfestbett 3 aus wenigstens zwei räumlich aufeinanderfolgenden Katalysatorfestbettzonen besteht, wobei die volumenspezifische Aktivität innerhalb einer Katalysatorfestbettzone im wesentlichen konstant ist und in Strömungsrichtung des Reaktionsgasgemisches 3 beim Übergang von einer Katalysatorfestbettzone in eine andere Katalysatorfestbettzone sprunghaft zunimmt,

- sich die Temperaturzone C bis zu einem Umsatz des Acroleins von 45 bis 85 % des in der dritten Reaktionsstufe angestrebten Wertes für $U^A$ erstreckt,
- bei einmaligem Durchgang des Reaktionsgasausgangsgemisches 3 durch das gesamte Katalysatorfestbetts 3 der Acroleinumsatz den erfindungsgemäßen angestrebten Wert $U^A$ hat und die Selektivität der Acrylsäurebildung, bezogen auf über beide Reaktionsstufen umgesetztes Propen, $\geq$ 80 mol-% beträgt,
- die zeitliche Abfolge, in der das Reaktionsgasgemisch 3 die Temperaturzonen C, D durchströmt, der alphabetischen Abfolge der Temperaturzonen C, D entspricht,
- die Belastung des Katalysatorfestbetts 3 mit dem im Reaktionsgasausgangsgemisch 3 enthaltenen Acrolein $\geq$ 70 NI Acrolein/I Katalysatorfestbett 3·h beträgt, und
- die Differenz $T^{maxC} - T^{maxD}$, gebildet aus der höchsten Temperatur $T^{maxC}$, die das Reaktionsgasgemisch 3 innerhalb der Temperaturzone C aufweist, und der höchsten Temperatur $T^{maxD}$, die das Reaktionsgasgemisch 3 innerhalb der Temperaturzone D aufweist, $\geq$ 0˚C beträgt,

mit der Maßgabe, dass das Verfahren zusätzlich dadurch gekennzeichnet ist, dass weder der Übergang von der Temperaturzone A in die Temperaturzone B im Katalysatorfestbett 2, noch der Übergang von der Temperaturzone C in die Temperaturzone D im Katalysatorfestbett 3 mit einem Übergang von einer Katalysatorfestbettzone in eine andere Katalysatorfestbettzone zusammenfällt.

Unter der Temperatur einer Temperaturzone wird dabei die Temperatur des in der Temperaturzone befindlichen Teils des Katalysatorfestbetts bei Ausübung des erfindungsgemäßen Verfahrens, jedoch in Abwesenheit einer chemischen Reaktion verstanden. Ist diese Temperatur innerhalb der Temperaturzone nicht konstant, so meint der Begriff Temperatur einer Temperaturzone hier den (Zahlen)mittelwert der Temperatur des Katalysatorfestbetts längs der Reaktionszone. Wesentlich ist dabei, dass die Temperierung der einzelnen Temperaturzonen im wesentlichen unabhängig voneinander erfolgt.

Da sowohl die heterogen katalysierte partielle Gasphasenoxidation von Propen zu Acrolein als auch die heterogen katalysierte partielle Gasphasenoxidation von Acrolein zu Acrylsäure eine ausgeprägt exotherme Reaktion ist, ist sowohl die Temperatur des Reaktionsgasgemisches 2 als auch die Temperatur des Reaktionsgasgemisches 3 beim reaktiven Durchgang durch das Katalysatorfestbett 2 bzw. Katalysatorfestbett 3 in der Regel von der Temperatur einer Temperaturzone verschieden. Sie liegt normalerweise oberhalb der Temperatur der Temperaturzone und durchläuft innerhalb einer Temperaturzone in der Regel ein Maximum (Heißpunktmaximum) oder fällt von einem Maximalwert ausgehend ab.

In der Regel wird beim erfindungsgemäßen Verfahren die Differenz $T^{maxA} - T^{maxB}$ nicht mehr als 80˚C betragen. Erfindungsgemäß bevorzugt beträgt $T^{maxA} - T^{maxB} \geq$ 3˚C und $\leq$ 70˚C. Ganz besonders bevorzugt beträgt $T^{maxA} - T^{maxB}$ beim erfindungsgemäßen Verfahren $\geq$ 20˚C und $\leq$ 60˚C.

Die erfindungsgemäß geforderten Differenzen $T^{maxA} - T^{maxB}$ stellen sich bei der Ausübung des erfindungsgemäßen Verfahren im Fall von eher niederen $\geq$ 90 NI/I· h und $\leq$ 160 NI/I · h).Propenbelastungen des Katalysatorfestbetts normalerweise dann ein, wenn einerseits sowohl die Temperatur der Reaktionszone A als auch die Temperatur der Reaktionszone B. im Bereich von 290 bis 380˚C liegt und andererseits die Differenz zwischen der Temperatur der Reaktionszone B ($T_B$) und der Temperatur der Reaktionszone A ($T_A$), d.h., $T_B - T_A$, $\leq$ 0˚C und $\geq$ -20˚C oder $\geq$-10˚C bzw. $\leq$ 0˚C und $\geq$ -5˚C, oder häufig $\leq$ 0˚C und $\geq$ -3˚C beträgt.

Bei Ausübung des erfindungsgemäßen Verfahrens unter (erfindungsgemäß bevorzugt) erhöhten Propenbelastungen ($\geq$ 160 NI/I · h und $\leq$ 300 NI/I · h, bzw. $\leq$ 600 NI/I · h) stellen sich die erfindungsgemäß geforderten Differenzen $T^{maxA} - T^{maxB}$ normalerweise dann ein, wenn einerseits sowohl die Temperatur der Reaktionszone A als auch die Temperatur der Reaktionszone B im Bereich von 290 bis 380˚C liegt und $T_B - T_A \geq$ 0˚C und $\leq$ 50˚C, oder $\geq$ 5˚C und $\geq$ 45˚C, oder $\geq$ 10˚C und $\leq$ 40˚C, oder $\geq$ 15˚C und $\leq$ 30˚C oder $\leq$ 35˚C (z.B. 20˚C oder 25˚C) beträgt.

Die vorgenannte Aussage betreffend die Temperaturdifferenzen $T_B - T_A$ gilt regelmäßig auch dann, wenn die Temperatur der Reaktionszone A im bevorzugten Bereich von 305 bis 365˚C bzw. im besonders bevorzugten Bereich von 310 bis 340˚C liegt.

Die Propenbelastung des Katalysatorfestbetts kann somit beim beschriebenen Verfahren z.B. $\geq$ 90 NI/I · h und $\leq$ 300 NI/I · h, oder $\geq$ 110 NI/I · h und $\leq$ 280 NI/I · h oder $\geq$ 130 NI/I · h und $\leq$ 260 NI/I · h, oder $\geq$ 150 NI/I · h und $\leq$ 240 NI/I · h, oder $\geq$ 170 NI/I · h und $\leq$ 220 NI/I · h, oder $\geq$ 190 NI/I · h und $\leq$ 200 NI/I · h betragen.

Erfindungsgemäß bevorzugt erstreckt sich die Temperaturzone A bis zu einem Umsatz des Propens von 50 bis 70 % bzw. 60 bis 70 % des in der zweiten Reaktionsstufe für $U^P$ erfindungsgemäß angestrebten Wertes.

In der Regel wird beim erfindungsgemäßen Verfahren die Differenz $T^{maxC} - T^{maxD}$ nicht mehr als 75˚C betragen. Erfindungsgemäß bevorzugt beträgt $T^{maxC} - T^{maxD} \geq$ 3˚C und $\leq$ 60˚C. Ganz besonders bevorzugt beträgt $T^{maxC} - T^{maxD}$ beim erfindungsgemäßen Verfahren $\geq$ 5˚C und $\leq$ 40˚C.

Die erfindungsgemäß geforderten Differenzen $T^{maxC} - T^{maxD}$ stellen sich bei der Ausübung des erfindungsgemäßen Verfahren im Fall von eher niederen ($\geq$ 70 NI/I · h und $\leq$ 150 NI/I · h) Acroleinbelastungen des Katalysatorbetts 3 normalerweise dann ein, wenn einerseits sowohl die Temperatur der Reaktionszone C als auch die Temperatur der Reaktionszone D im Bereich von 230 bis 320˚C liegt und andererseits die Differenz zwischen der Temperatur der Reaktionszone D ($T_D$) und der Temperatur der Reaktionszone C ($T_C$), d.h., $T_D - T_C$, $\leq 0$˚C und $\geq -20$˚C oder $\geq -10$˚C bzw. $\leq 0$˚C und $\geq -5$˚C, oder häufig $\leq 0$˚C und $\geq -3$˚C beträgt.

Bei Ausübung des erfindungsgemäßen Verfahrens unter erhöhten Propenbelastungen und damit auch erhöhten Acroleinbelastungen ($\geq 150$ Nl/l · h und $\leq 300$ Nl/l · h, bzw. $\leq 600$ Nl/l · h) stellen sich die erfindungsgemäß geforderten Differenzen $T^{maxC} - T^{maxD}$ normalerweise dann ein, wenn einerseits sowohl die Temperatur der Reaktionszone C als auch die Temperatur der Reaktionszone D im Bereich von 230 bis 320˚C liegt und $T_D - T_C \geq 0$˚C und $\leq 40$˚C, oder $\geq 5$˚C und $\leq 35$˚C, bzw. 30˚C, oder $\geq 10$˚C und $\leq 25$˚C. bzw. $\leq 20$˚C, oder $\leq 15$˚C beträgt.

Die vorgenannte Aussage betreffend die Temperaturdifferenzen $T_D - T_C$ gilt regelmäßig auch dann, wenn die Temperatur der Reaktionszone C im bevorzugten Bereich von 250 bis 300˚C bzw. im besonders bevorzugten Bereich von 260 bis 280˚C liegt.

Die Acroleinbelastung des Katalysatorfestbetts 3 kann somit beim erfindungsgemäßen Verfahren z. B. $\geq 70$ Nl/l · h bzw. $\geq 90$ Nl/l · h und $\leq 300$ Nl/l · h, oder $\geq 110$ Nl/l · h und $\leq 280$ Nl/l · h oder $\geq 130$ Nl/l · h und $\leq 260$ Nl/l · h, oder $\geq 150$ Nl/l · h und $\leq 240$ Nl/l · h, oder $\geq 170$ Nl/l · h und $\leq 220$ Nl/l · h, oder $\geq 190$ Nl/l · h und $\leq 200$ Nl/l · h betragen.

Erfindungsgemäß bevorzugt erstreckt sich die Temperaturzone C bis zu einem Umsatz des Acroleins von 50 bis 85 % bzw. 60 bis 85 % des in der dritten Reaktionsstufe für $U^A$ angestrebten Wertes.

Der Arbeitsdruck kann dabei in beiden Reaktionsstufen sowohl unterhalb von Normaldruck (z. B. bis zu 0,5 bar) als auch oberhalb von Normaldruck liegen. Typischerweise wird der Arbeitsdruck in den beiden Reaktionsstufen bei Werten von 1 bis 5 bar, häufig 1 bis 3 bar liegen.

Normalerweise wird der Reaktionsdruck in keiner der beiden Reaktionsstufen 100 bar überschreiten.

Die Selektivität der Wertproduktbildung in der vorgeschriebenen zweiten Reaktionsstufe (Summe aus Acroleinbildung und Acrylsäurenebenproduktbildung) wird dabei bei in an sich bekannter Weise geeigneter Wahl (siehe in dieser Schrift empfohlene Katalysatoren) des Katalysatorfestbetts 2 erfindungsgemäß regelmäßig $\geq 92$ mol-%, oder $\geq$ 94 mol-%, häufig $\geq 95$ mol-%, oder $\geq 96$ mol-% bzw. $\geq 97$ mol-% betragen.

In der Regel wird beim vorstehend beschriebenen Verfahren die Acroleinbelastung des Katalysatorfestbetts 3 ferner wenigstens etwa 10 Nl/l · h, häufig etwa wenigstens 20 bzw. 25, oder wenigstens 30 bzw. 40, oder wenigstens 50 Nl/l · h unterhalb der Propenbelastung des Katalysatorfestbetts 2 liegen. Dies ist primär darauf zurückzuführen, dass in der zweiten Reaktionsstufe der Umsatz des Propens erfindungsgemäß limitiert gefahren wird.

Bei in an sich bekannter Weise geeigneter Wahl der Katalysatorfestbetten 2 und 3 (siehe die in dieser Schrift gegebenen Katalysatorempfehlungen) wird die bei der vorstehend beschriebenen Verfahrensweise über beide Reaktionsstufen bilanzierte Selektivität der Acrylsäurebildung, bezogen auf umgesetztes Propen erfindungsgemäß regelmäßig, bei Werten $\geq 83$ mol-%, häufig bei $\geq 85$ mol-%, oder $\geq 88$ mol-%, oft bei $\geq 90$ mol-%, oder $\geq 93$ mol-% liegen.

Für die beschriebene Verfahrensweise wesentlich ist, dass das Katalysatorfestbett 2 aus wenigstens zwei räumlich aufeinander folgenden Katalysatorfestbettzonen besteht, wobei die volumenspezifische Aktivität innerhalb einer Katalysatorfestbettzone im wesentlichen konstant ist und in Strömungsrichtung des Reaktionsgasgemisches 2 beim Übergang von einer Katalysatorfestbettzone in eine andere Katalysatorfestbettzone sprunghaft zunimmt.

Die votumenspezifische (d. h., die auf die Einheit der jeweiligen Schüttungsvolumens normierte) Aktivität einer Katalysatorfestbettzone kann nun in über die Katalysatorfestbettzone im wesentlichen konstanter Weise dadurch eingestellt werden, dass man von einer Grundmenge einheitlich hergestellter Katalysatorformkörper ausgeht (ihre Schüttung entspricht der maximal erzielbaren volumenspezifischen Aktivität) und diese in der jeweiligen Katalysatorfestbettzone mit sich bezüglich der heterogen katalysierten partiellen Gasphasenoxidation im wesentlichen inert verhaltenden Formkörpern (Verdünnungsformkörper) homogen verdünnt. Je höher der Anteil der Verdünnungsformkörper gewählt wird, desto geringer ist die in einem bestimmten Volumen der Schüttung enthaltene Aktivmasse bzw. Katalysatoraktivität. Als Materialien für solche inerten Verdünnungsformkörper kommen prinzipiell alle diejenigen in Betracht, die sich auch als Trägermaterial für erfindungsgemäß geeignete Schalenkatalysatoren eigenen.

Als solche Materialien kommen z. B. poröse oder unporöse Aluminiumoxide, Siliciumdioxid, Thoriumdioxid, Zirkondioxid, Siliziumcarbid, Silikate wie Magnesium -oder Aluminiumsilikat oder der bereits erwähnte Steatit (z. B. Steatit C-220 der Fa. Ceram Tec) in Betracht.

Die Geometrie solcher inerter Verdünnungsformkörper kann im Prinzip beliebig sein. D. h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

Erfindungsgemäß günstig ist es, wenn sich die chemische Zusammensetzung der verwendeten Aktivmasse über das gesamte Katalysatorfestbett 2 nicht verändert. D. h., die für einen einzelnen Katalysatorformkörper verwendete Aktivmasse kann zwar ein Gemisch aus verschiedenen, die Elemente Mo, Fe und Bi enthaltenden, Multimetalloxiden sein, für alle Katalysatorformkörper des Katalysatorfestbetts 2 ist dann jedoch das gleiche Gemisch zu verwenden.

Eine in Strömungsrichtung des Reaktionsgasgemisches 2 über das Katalysatorfestbett 2 zonenweise zunehmende volumenspezifische Aktivität lässt sich für das beschriebene Verfahren somit in einfacher Weise z. B. dadurch einstellen, dass man die Schüttung in einer ersten Katalysatorfestbettzone mit einem hohen Anteil an inerten Verdünnungsverformkörpern bezogen auf eine Sorte von Katalysatorformkörpern beginnt, und dann diesen Anteil an Verdünnungsformkörpern in Strömungsrichtung zonenweise verringert.

Eine zonenweise Zunahme der volumenspezifischen Aktivität ist aber auch z. B. dadurch möglich, dass man bei gleich bleibender Geometrie und Aktivmassenart eines Schalenkatalysatorformkörpers zonenweise die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse zonenweise den Anteil an Katalysatorformkörpern mit höherem Aktivmassenanteil steigert. Alternativ kann man auch die Aktivmassen selbst verdünnen, in dem man bei der Aktivmassenherstellung z. B. in das zu calcinierende Trockengemisch aus Ausgangsverbindungen inerte verdünnend wirkende Materialien wie hochgebranntes Silicimdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich z. B. auch dadurch erzielen, dass man bei Mischungen aus Vollkatalysatoren und aus Schalenkatalysatoren (bei identischer Aktivmasse) in entsprechender Weise das Mischungsverhältnis verändert. Des weiteren lässt sich eine Variation der volumenspezifischen Aktivität durch den Einsatz von Katalysatorgeometrien mit unterschiedlicher Schüttdichte erzielen (z.B. bei Vollkatalysatoren mit identischer Aktivmassenzusammensetzung der verschiedenen Geometrien). Selbstredend lassen sich die beschriebenen Varianten auch kombiniert anwenden.

Natürlich können für das Katalysatorfestbett 2 aber auch Mischungen aus Katalysatoren mit chemisch unterschiedlicher Aktivmassenzusammensetzung und als Folge dieser verschiedenen Zusammensetzung unterschiedlicher Aktivität verwendet werden. Diese Mischungen können wiederum zonenweise in ihrer Zusammensetzung variiert und/oder mit unterschiedlichen Mengen inerter Verdünnungsformkörper verdünnt werden.

Vorab und/oder im Anschluss an das Katalysatorfestbett 2 können sich ausschließlich aus Inertmaterial (z. B. nur Verdünnungsformkörpern) bestehende Schüttungen befinden (sie werden in dieser Schrift begrifflich nicht dem Katalysatorfestbett 2 zugerechnet, da sie keine Formkörper enthalten, die Multimetalloxidaktivmasse aufweisen). Dabei können die für die Inertschüttung verwendeten Verdünnungsformkörper die gleiche Geometrie wie die im Katalysatorfestbett 2 verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z. B. kugelförmig anstelle ringförmig).

Häufig weisen die für solche Inertschüttungen verwendeten Formkörper die ringförmige. Geometrie 7 mm x 7 mm x 4 mm (Außendurchmesser x Länge x Innendurchmesser) oder die kugelförmige Geometrie mit dem Durchmesser d = 4 - 5 mm auf. Die Temperaturzonen A und B können sich beim erfindungsgemäßen Verfahren auch auf die 1-nertschüttungen erstrecken. Erfindungsgemäß vorteilhaft erfassen sowohl die Temperaturzone A als auch die Temperaturzone B jeweils nicht mehr als drei Katalysatorfestbettzonen (erfindungsgemäß zwingend wird wenigstens eine Katalysatorfestbettzone von beiden Temperaturzonen erfasst).

Erfindungsgemäß besonders vorteilhaft umfasst das gesamte Katalysatorfestbett 2 nicht mehr als fünf, zweckmäßig nicht mehr als vier bzw. drei Katalysatorfestbettzonen.

Beim Übergang von einer Katalysatorfestbettzone in eine andere Katalysatorfestbettzone (in Strömungsrichtung des Reaktionsgasgemisches 2) des Katalysatorfestbetts 2 sollte (bei einheitlicher Aktivmasse über das gesamte Katalysatorfestbett 2) die volumenspezifische Aktivmasse (d. h., das Gewicht der im Einheitsschüttungsvolumen enthaltenen Multimetalloxidaktivmasse) erfindungsgemäß zweckmäßig um wenigstens 5 Gew.-%, bevorzugt um wenigstens 10 Gew.-% zunehmen (dies gilt insbesondere auch bei einheitlichen Katalysatorformkörpern über das gesamte Katalysatorfestbett 2). In der Regel wird diese Zunahme beim erfindungsgemäßen Verfahren nicht mehr als 50 Gew.-%, meist nicht mehr als 40 Gew.-% betragen. Ferner sollte bei einheitlicher Aktivmasse über das gesamte Katalysatorfestbett 2 der Unterschied in der volumenspezifischen Aktivmasse derjenigen Katalysatorfestbettzone mit der geringsten volumenspezifischen Aktivität und derjenigen Katalysatorfestbettzone mit der höchsten volumenspezifischen Aktivität erfindungsgemäß nicht mehr als 50 Gew.-%, vorzugsweise nicht mehr als 40 Gew.-% und in der Regel nicht mehr als 30 Gew.-% betragen.

Häufig wird beim erfindungsgemäßen Verfahren das Katalysatorfestbett 2 aus nur zwei Katalysatorfestbettzonen bestehen.

Erfindungsgemäß bevorzugt ist die in Strömungsrichtung des Reaktionsgasgemisches 2 letzte Katalysatorfestbettzone des Katalysatorfestbetts 2 unverdünnt. D. h., sie besteht vorzugsweise ausschließlich aus Katalysatorformkörpern. Im Bedarfsfall kann sie auch aus einer Schüttung aus Katalysatorformkörpern bestehen, deren volumenspezifische Aktivität z. B. durch Verdünnung mit Inertmaterial abgesenkt, z. B. um 10 %, ist.

Besteht des Katalysatorfestbett 2 nur aus zwei Katalysatorfestbettzonen, ist es erfindungsgemäß in der Regel vorteilhaft (wie ganz allgemein beim erfindungsgemäßen Verfahren), wenn die Katalysatorfestbettzone mit der höchsten volumenspezifischen Aktivität nicht bis in die Temperaturzone A hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt). D. h., in günstiger Weise wird die Katalysatorfestbettzone mit der geringeren volumenspezifischen Aktivität in die Temperaturzone B hineinragen und die Katalysatorfestbettzone mit der höheren volumenspezifischen Aktivität in der Temperaturzone B beginnen und enden. (d. h., hinter dem Übergang von der Temperaturzone A in die Temperaturzone B ihren Anfang haben.

Besteht das Katalysatorfestbett 2 nur aus drei Katalysatorfestbettzonen, ist es erfindungsgemäß in der Regel gleichfalls vorteilhaft, wenn die Katalysatorfestbettzone mit der höheren volumenspezifischen Aktivität nicht bis in die Temperaturzone A hineinragt sondern in der Temperaturzone B beginnt und endet, d. h., hinter dem Übergang von der Temperaturzone A in die Temperaturzone B ihren Anfang hat (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt). D. h., normalerweise wird in diesem Fall die Katalysatorfestbettzone mit der zweithöchsten volumenspezifischen Aktivität sowohl in die Temperaturzone A als auch in die Temperaturzone B hineinragen.

Besteht das Katalysatorfestbett 2 aus vier Katalysatorfestbettzonen, ist es erfindungsgemäß in der Regel vorteilhaft, wenn die Katalysatorfestbettzone mit der dritthöchsten volumenspezifischen Aktivität sowohl in die Temperaturzone A als auch in die Temperaturzone B hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch 2 strömt).

Im Fall einer Gleichstromführung von Reaktionsgasgemisch 2 und Wärmeträgern in den Temperaturzonen A und B kann es vorteilhaft sein, wenn beim erfindungsgemäßen Verfahren innerhalb des Katalysatorfestbetts 2 die Katalysatorfestbettzone mit der höchstens volumenspezifischen Aktivität in die Temperaturzone A hineinragt.

Generell lässt sich die volumenspezifische Aktivität zwischen zwei Katalysatorfestbettzonen eines Katalysatorfestbetts 2 experimentell in einfacher Weise so differenzieren, dass unter identischen Randbedingungen (vorzugsweise den Bedingungen des ins Auge gefassten Verfahrens) über Katalysatorfestbetten derselben Länge, aber jeweils der Zusammensetzung der jeweiligen Katalysatorfestbettzone entsprechend, das gleiche Propen enthaltende Reaktionsgasgemisch geführt wird. Die höhere umgesetzte Menge an Propen weist die höhere volumenspezifische Aktivtät aus.

Beträgt die Gesamtlänge des Katalysatorfestbetts 2 $L^1$, ist es erfindungsgemäß vorteilhaft, wenn sich im Bereich

$$X^1 \pm L^1 \cdot \frac{4}{100} \quad \text{bzw. im Bereich} \quad X^1 \pm L^1 \cdot \frac{3}{100} \quad \text{bzw. im Bereich} \quad X^1 \pm L^1 \cdot \frac{2}{100}$$

kein Übergang von einer Katalysatorfestbettzone in eine andere Katalysatorfestbettzone befindet, wobei X der Ort (die Stelle) innerhalb des Katalysatorfestbetts 2 ist, an dem der Übergang von der Temperaturzone A in die Temperaturzone B erfolgt.

Bevorzugt ist beim vorstehend beschrieben Verfahren das Katalysatorfestbett in Strömungsrichtung des Reaktionsgasgemisches 2 wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d. h. z. B. auf einer Länge von 0,70 bis 1,50 m, bevorzugt 0,90 bis 1,20 m), jeweils der Gesamtlänge des Katalysatorfestbetts 2, ein homogenes Gemisch aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diese erste Zone des Katalysatorfestbetts befindet sich dann vorteilhaft bis zum Ende der Länge des Katalysatorfestbetts (d. h., z. B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige (unverdünnte) Schüttung derselben Katalysatorformkörper, die auch in der ersten Zone verwendet worden sind. Das Vorgenannte trifft insbesondere dann zu, wenn im Katalysatorfestbett als Katalysatorformkörper Vollkatalysatorringe oder Schalenkatalysatorringe (insbesondere jene, die in dieser Schrift als bevorzugt genannt werden) eingesetzt werden. Mit Vorteil weisen im Rahmen der vorgenannten Strukturierung sowohl die Katalysatorformkörper als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

Das Vorgenannte trifft auch dann zu, wenn anstelle inerter Verdünnungsformkörper Schalenkatalysatorformkörper verwendet werden, deren Aktivmassenanteil um 2 bis 15 Gew.-% Punkte tiefer liegt, als der Aktivmassenanteil der am Ende des Katalysatorfestbetts 2 gegebenenfalls verwendeten Schalenkatalysatorformkörper.

Eine reine Inertmaterialschüttung, deren Länge, bezogen auf die Länge des Katalysatorfestbetts 2, zweckmäßig 5 bis 20 % beträgt, leitet in Strömungsrichtung des Reaktionsgasgemisches 2 in der Regel das Katalysatorefestbett 2 ein. Sie wird normalerweise als Aufheizzone für das Reaktionsgasgemisch 2 genutzt. Anstelle der Inertmaterialschüttung kann als Aufheizzone aber auch mit Inertmaterial verdünnte Katalysatorschüttung verwendet werden.

Erfindungsgemäß vorteilhaft erstreckt sich nun bei den vorgenannten Katalysatorfestbetten 2 die Katalysatorfestbettzone mit der geringeren volumenspezifischen Aktivität noch auf 5 bis 20 %, häufig auf 5 bis 15 % ihrer Länge in die Temperaturzone B.

Zweckmäßig erstreckt sich die Temperaturzone A auch auf eine für das Katalysatorfestbett 2 gegebenenfalls angewandte Vorschüttung aus Inertmaterial.

Für die Vorteilhaftigkeit der beschriebenen Verfahrensweise wesentlich ist ferner, dass das Katalysatorfestbett 3 aus wenigstens zwei räumlich aufeinander folgenden Katalysatorfestbettzonen besteht, wobei die volumenspezifische, Aktivität innerhalb einer Katalysatorfestbettzone im wesentlichen konstant ist und in Strömungsrichtung des Reaktionsgasgemisches 3 beim Übergang von einer Katalysatorfestbettzone in eine andere Katalysatorfestbettzone sprunghaft zunimmt.

Die volumenspezifische (d. h., die auf die Einheit des jeweiligen Schüttungsvolumens normierte) Aktivität einer Katalysatorfestbettzone kann nun in über die Katalysatorfestbettzone im wesentlichen konstanter Weise dadurch eingestellt werden, dass man von einer Grundmenge einheitlich hergestellter Katalysatorformkörper ausgeht (ihre Schüttung entspricht der maximal erzielbaren volumenspezifischen Aktivität) und diese in der jeweiligen Katalysatorfestbettzone mit sich bezüglich der heterogen katalysierten partiellen Gasphasenoxidation im wesentlichen inert verhaltenden Formkörpern (Verdünnungsformkörper) homogen verdünnt. Je höher der Anteil der Verdünnungsformkörper gewählt wird, desto geringer ist die in einem bestimmten Volumen der Schüttung enthaltene Aktivmasse bzw. Katalysatoraktivität. Als Materialien für solche inerten Verdünnungsformkörper kommen prinzipiell alle diejenigen in Betracht, die sich auch als Trägermaterial für erfindungsgemäß geeignete Schalenkatalysatoren eignen.

Als solche Materialien kommen z. B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z. B. Steatit C-220 der Fa. Cerm Tec) in Betracht.

Die Geometrie solcher inerter Verdünnungsformkörper kann im Prinzip beliebig sein. D. h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

Erfindungsgemäß günstig ist es, wenn sich die chemische Zusammensetzung der verwendeten Aktivmasse über das gesamte Katalysatorfestbett 3 nicht verändert. D.h., die für einen einzelnen Katalysatorformkörper verwendete Aktivmasse kann zwar ein Gemisch aus verschiedenen, die Elemente Mo und V enthaltenden, Multimetalloxiden sein, für alle Katalysatorformkörper des Katalysatorfestbetts 3 ist dann jedoch das gleiche Gemisch zu verwenden.

Eine in Strömungsrichtung des Reaktionsgasgemisches 3 über das Katalysatorfestbett 3 zonenweise zunehmende volumenspezifische Aktivität lässt sich für das beschriebene Verfahren somit in einfacher Weise z. B. dadurch einstellen, dass man die Schüttung in einer ersten Katalysatorfestbettzone mit einem hohen Anteil an inerten Verdünnungsformkörpern bezogen auf eine Sorte von Katalysatorformkörpern beginnt, und dann diesen Anteil an Verdünnungsformkörpern in Strömungsrichtung zonenweise verringert.

Eine zonenweise Zunahme der volumenspezifischen Aktivität ist aber auch z. B. dadurch möglich, dass man bei gleich bleibender Geometrie und Aktivmassenart eines Schalenkatalysatorformkörpers zonenweise die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse zonenweise den Anteil an Katalysatorformkörpern mit höherem Aktivmassengewichtsanteil steigert. Alternativ kann man auch die Aktivmassen selbst verdünnen, in dem man bei der Aktivmassenherstellung z. B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich z. B. auch dadurch erzielen, dass man bei Mischungen aus Vollkatalysatoren und aus Schalenkatalysatoren (bei identischer Aktivmasse) in entsprechender Weise das Mischungsverhältnis verändert. Des weiteren lässt sich eine Variation der volumenspezifischen Aktivität durch den Einsatz von Katalysatorgeometrien mit unterschiedlicher Schüttdichte erzielen (z. B. bei Vollkatalysatoren mit identischer Aktivmassenzusammensetzung der verschiedenen Geometrien). Selbstredend lassen sich die beschriebenen Varianten auch kombiniert anwenden.

Natürlich können für das Katalysatorfestbett 3 aber auch Mischungen aus Katalysatoren mit chemisch unterschiedlicher Aktivzusammensetzung und als Folge dieser verschiedenen Zusammensetzung unterschiedlicher Aktivität verwendet werden. Diese Mischungen können wiederum zonenweise in ihrer Zusammensetzung variiert und/oder mit unterschiedlichen Mengen inerter Verdünnungsformkörper verdünnt werden.

Vorab und/oder im Anschluss an das Katalysatorfestbett 3 können sich ausschließlich aus Inertmaterial (z. B. nur Verdünnungsformkörpern) bestehende Schüttungen befinden (sie werden in dieser. Schrift begrifflich nicht dem Katalysatorfestbett 3 zugerechnet, da sie keine Formkörper enthalten, die Multimetalloxidaktivmasse aufweisen). Dabei können die für die Inertschüttung verwendeten Verdünnungsformkörper die gleich Geometrie wie die in der Festbettkatalysatorschüttung verwendeten Verdünnungsformkörper aufweisen. Die Geometrie der für die Inertschüttung verwendeten Verdünnungsformkörper kann aber auch von der vorgenannten Geometrie der Katalysatorformkörper verschieden sein (z. B. kugelförmige anstatt ringförmig).

Häufig weisen die für solche Inertschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4 - 5 mm auf. Die Temperaturzone C und D können sich beim erfindungsgemäßen Verfahren auch auf die Inertschüttungen erstrecken. Erfindungsgemäß vorteilhaft erfassen sowohl die Temperaturzone C als auch die Temperaturzone D jeweils nicht mehr als drei Katalysatorfestbettzonen (erfindungsgemäß zwingend wird wenigstens eine Katalysatorfestbettzone von beiden Temperaturzonen erfasst).

Erfindungsgemäß besonders vorteilhaft umfasst das gesamte Katalysatorfestbett 3 nicht mehr als fünf, zweckmäßig nicht mehr als vier bzw. drei Katalysatorfestbettzonen.

Beim Übergang von einer Katalysatorfestbettzone in eine andere Katalysatorfestbettzone (in Strömungsrichtung des Reaktionsgasgemisches 3) sollte (bei einheitlicher Aktivmasse über das gesamte Katalysatorfestbett 3) die volumenspezifische Aktivmasse (d. h., das Gewicht der im Einheitsschüttungsvolumen enthaltenen Multimetalloxidaktivmasse) erfindungsgemäß zweckmäßig um wenigstens 5 Gew.-%, bevorzugt um wenigstens 10 Gew.-% zunehmen (dies gilt insbesondere auch bei einheitlichen Katalysatorformkörpern über das gesamte Katalysatorfestbett 3). In der Regel wird diese Zunahme beim erfindungsgemäßen Verfahren nicht mehr als 50 Gew.-%, meist nicht mehr als 40 Gew.-% betragen. Ferner sollte bei einheitlicher Aktivmasse über das gesamte Katalysatorfestbett 3 der Unterschied in der volumenspezifischen Aktivmasse derjenigen Katalysatorfestbettzone mit der geringsten volumenspezifischen Aktivität und derjenigen Katalysatorfestbettzone mit der höchsten volumenspezifischen Aktivität erfindungsgemäß nicht mehr als 50 Gew.-%, vorzugsweise nicht mehr als 40 Gew.-% und besonders bevorzugt nicht mehr als 30 Gew.-% betragen.

Häufig wird beim erfindungsgemäßen Verfahren das Katalysatorfestbett 3 aus nur zwei Katalysatorfestbettzonen bestehen.

Erfindungsgemäß bevorzugt ist die in Strömungsrichtung des Reaktionsgasgemisches 3 letzte Katalysatorfestbettzone des Katalysatorfestbetts 3 unverdünnt. D. h., sie besteht vorzugsweise ausschließlich aus Katalysatorformkörpern. Im Bedarfsfall kann sie auch aus einer Schüttung aus Katalysatorformkörpern bestehen, deren volumenspezifische Aktivität z. B. durch Verdünnung mit Inertmaterial abgesenkt, z. B. um 10 %, ist. Besteht das Katalysatorfestbett 3 nur aus zwei Katalysatorfestbettzonen, ist es erfindungsgemäß in der Regel vorteilhaft (wie ganz allgemein beim erfindungsgemäßen Verfahren), wenn die Katalysatorfestbettzone mit der höchsten volumenspezifischen Aktivität bis in die Temperaturzone C hineinragt (insbesondere dann, wenn in der Temperaturzone C und in der Temperaturzone D die Temperierung mittels eines fließenden Wärmeträgers erfolgt, der jeweils im Gegenstrom zum Reaktionsgasgemisch 3 strömt).

Besteht das Katalysatorfestbett 3 nur aus drei Katalysatorfestbettzonen, ist es erfindungsgemäß in der Regel gleichfalls vorteilhaft, wenn die Katalysatorfestbettzone mit der höchsten volumenspezifischen Aktivität bis in die Temperaturzone C hineinragt (insbesondere dann, wenn in der Temperaturzone C und in der Temperaturzone D die Temperierung mittels eines fließenden Wärmeträgers erfolgt, der jeweils im Gegenstrom zum Reaktionsgasgemisch 3 strömt).

Besteht das Katalysatorfestbett 3 aus vier Katalysatorfestbettzonen, ist es erfindungsgemäß in der Regel vorteilhaft, wenn die Katalysatorfestbettzone mit der zweithöchsten volumenspezifischen Aktivität sowohl in die Temperaturzone C als auch in die Temperaturzone D hineinragt (insbesondere dann, wenn in der Temperaturzone C und in der Temperaturzone D die Temperierung mittels eines fließenden Wärmeträgers erfolgt, der jeweils im Gegenstrom zum Reaktionsgasgemisch 3 strömt.

Im Fall einer Gleichstromführung von Reaktionsgasgemisch 3 und Wärmeträgern in den Temperaturzonen C und D kann es erfindungsgemäß vorteilhaft sein, wenn innerhalb des Katalysatorfestbetts 3 die Katalysatorfestbettzone mit der höchsten volumenspezifischen Aktivität nicht in die Temperaturzone C hineinragt, sondern erst hinter dem Übergang von der Temperaturzone C in die Temperaturzone D ihren Anfang hat.

Die volumenspezifische Aktivität zwischen zwei Katalysatorfestbettzonen innerhalb des Katalysatorfestbett 3 lässt sich experimentell in einfacher Weise so differenzieren, dass unter identischen Randbedingungen (vorzugsweise den Bedingungen des ins Auge gefassten Verfahrens) über Katalysatorfestbetten der selben Länge, aber jeweils der Zusammensetzung der jeweiligen Katalysatorfestbettzone entsprechend, das gleiche Acrolein enthaltende Reaktionsgasausgangsgemisch geführt wird. Die höhere umgesetzte Menge an Acrolein weist die höhere volumenspezifische Aktivität aus.

Beträgt die Gesamtlänge des Katalysatorfestbett 3 $L^2$, ist es erfindungsgemäß vorteilhaft, wenn sich im Bereich

$$X^2 \pm L^2 \, \frac{4}{100} \quad \text{bzw. im Bereich} \quad X^2 \pm L^2 \, \frac{3}{100} \quad \text{bzw. im Bereich 100 100} \quad X^2 \pm L^2 \, \frac{2}{100}$$

kein Übergang von einer Katalysatofestbettzone in eine andere Katalysatorfestbettzone befindet, wobei X der Ort innerhalb des Katalysatorfestbetts 3 ist, an dem der Übergang von der Temperaturzone C in die Temperaturzone D erfolgt.

Bevorzugt ist beim vorstehend beschriebenen Verfahren das Katalysatorfestbett 3 in Strömungsrichtung des Reaktionsgasgemisches 3 wie folgt strukturiert.

Zunächst auf einer Länge von 10 bis .60 %, bevorzugt 10 bis 50 %, besonders bevorzugt 20 bis 40 % und ganz besonders bevorzugt 25 bis 35 % (d. h., z. B. auf einer Länge von 0,70 bis 1,50 m, bevorzugt 0,90 bis 1,20 m), jeweils der Gesamtlänge das Katalysatorfestbetts 3, ein homogenes Gemisch oder zwei (mit abnehmender Verdünnung) aufeinander folgende homogene Gemische aus Katalysatorformkörpern und Verdünnungsformkörpern (wobei beide vorzugsweise im wesentlichen die gleich Geometrie aufweisen), wobei der Anteil der Verdünnungsformkörper so bemessen ist, dass die volumenspezifische Aktivmasse, bezogen auf eine nur aus den Katalysatorformkörpern bestehende Schüttung, um 10 bis 50 Gew.-%, vorzugsweise 20 bis 45 Gew.-% und besonders bevorzugt 25 bis 35 Gew.-% abgesenkt ist. Im Anschluss an diese erste bzw. an diese beiden ersten Zonen befindet sich dann erfindungsgemäß vorteilhaft bis zum Ende der Länge des Katalysatorfestbetts 3 (d. h., z. B. auf einer Länge von 2,00 bis 3,00 m, bevorzugt 2,50 bis 3,00 m) entweder eine nur in geringerem Umfang (als in der ersten bzw. in den ersten beiden Zonen) verdünnte Schüttung der Katalysatorformkörper, oder, ganz besonders bevorzugt, eine alleinige Schüttung derselben Katalysatorformkörper, die auch in den ersten Zonen verwendet worden sind.

Das Vorgenannte trifft insbesondere dann zu, wenn im Katalysatorfestbett 3 als Katalysatorformkörper Schalenkatalysatorringe oder Schalenkatalysatorkugeln eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 7 mm x 3 mm x 4 mm (Außendurchmesser x Länge x Innendurchmesser) auf.

Das oben Genannte trifft auch dann zu, wenn anstelle inerter Verdünnungsformkörper Schalenkatalysatorformkörper verwendet werden, deren Aktivmassenanteil um 2 bis 15 Gew.-% Punkte tiefer liegt, als der Aktivmassenanteil der Schalenkatalysatorformkörper am Ende des Katalysatorfestbetts 3.

Eine reine Inertmaterialschüttung, deren Länge, bezogen auf die Länge des Katalysatorfestbetts 3, zweckmäßig 5 bis 20.% beträgt, leitet in Strömungsrichtung des Reaktionsgasgemisches 3 in der Regel das Katalysatorfestbett 3 ein. Sie dient normalerweise dem Zweck der Temperierung des Reaktionsgasgemisches 3. Anstelle der Inertmaterialschüttung kann als Aufheizzone aber auch eine mit Inertmaterial verdünnte Katalysatorschüttung verwendet werden.

Erfindungsgemäß vorteilhaft erstreckt sich nun bei den vorgenannten Katalysatorfestbetten 3 die Temperaturzone C (die sich erfindungsgemäß vorteilhaft auch auf die Vorschüttung aus Inertmaterial erstreckt) noch auf 5 bis 20 %, häufig auf 5 bis 15 % der Länge der in Strömungsrichtung des Reaktionsgasgemisches 3 letzten (volumenspezifisch aktivsten) Katalysatorfestbettzone der Katalysatorfestbetts 3.

In anwendungstechnisch zweckmäßiger Weise erfolgt die Durchführung der zweiten Reaktionsstufe des vorstehend beschriebenen Verfahrens in einem Zweizonenrohrbündelreaktor, wie er z. B. in den DE-A's 199 10 508, 199 48 523, 199 10 506 und 199 48 241 beschrieben ist. Eine bevorzugte Variante eines erfindungsgemäß einsetzbaren Zweizonenrohrbündelreaktors offenbart die DE-C 28 30 765. Aber auch die in der DE-C 25 13 405, der US-A 3,147,084, der DE-A 22 01 528, der EP-A 38 32 24 und der DE-A 29 03 218 offenbarten Zweizonenrohrbündelreaktoren sind für eine Durchführung der zweiten Reaktionsstufe des vorstehend beschriebenen Verfahrens geeignet.

D. h., in einfachster Weise befindet sich das zu verwendende Katalysatorfestbett 2 (eventuell mit vor- und/oder nachangeordneten Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Temperaturzone. D. h., in einfachster Weise umströmt z. B. ein Salzbad A denjenigen Abschnitt der Rohre (die Temperaturzone A), in welchem sich die oxidative Umsetzung des Propens (beim einfachen Durchgang) bis zum Erreichen eines Umsatzes im Bereich von 40 bis 80 % des in der zweiten Reaktionsstufe erfindungsgemäß angestrebten Wertes $U^P$ vollzieht und ein Salzbad B umströmt den Abschnitt der Rohre (die Reaktionszone B), in welchem sich die oxidative Anschlussumsetzung des Propens (beim einfachen Durchgang) bis zum Erreichen des erfindungsgemäß angestrebten Wertes für $U^P$ vollzieht (bei Bedarf können sich an die erfindungsgemäß anzuwendenden Temperaturzonen A, B weitere Reaktionszonen anschließen, die auf individuellen Temperaturen gehalten werden).

Anwendungstechnisch zweckmäßig umfasst die beschriebene zweite Reaktionsstufe keine weiteren Temperaturzonen. D. h., das Salzbad B umströmt zweckmäßig den Abschnitt der Rohre, in welchem sich die oxidative Anschlussumsetzung des Propens (beim einfachen Durchgang) bis zum erfindungsgemäß angestrebten $U^P$-Wert vollzieht.

üblicherweise liegt der Beginn der Temperaturzone B hinter dem Heißpunktmaximum der Temperaturzone A.

Die beiden Salzbäder A, B können erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden. Selbstverständlich kann erfindungsgemäß auch in der Temperaturzone A eine Gleichströmung und in der Temperaturzone B eine Gegenströmung (oder umgekehrt) angewandt werden.

Selbstverständlich kann man in allen vorgenannten Fallkonstellationen innerhalb der jeweiligen Temperaturzonen der, relativ zu den Reaktionsrohren, erfolgenden Parallelströmung der Salzschmelze noch eine Querströmung überlagern, so dass die einzelne Reaktionszone einem wie in der EP-A 700 714 oder in der EP-A 700 893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Zweckmäßigerweise wird beim beschriebenen Verfahren das Reaktionsgasausgangsgemisch 2 dem Katalysatorfestbett 2 auf die Reaktionstemperatur vorerwärmt zugeführt.

Üblicherweise sind in den Zweizonenrohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 2 bis 4 m, bevorzugt 2,5 bis 3,5 m. In jeder Temperaturzone belegt die Festbettkatalysatorschüttung 1 wenigstens 60 % bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilüng) 35 bis 45 mm beträgt (vgl. z. B. EP-B 468 290).

Als Wärmeaustauschmittel eignen sich auch für die Zweizonenfahrweise insbesondere fluide Temperiermedien. Besonders günstig ist die Verwendung von Schmelzen von Salzen wie Kaliumnitrat, Kaliumnitrit, Natriumnitrit und/ oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreakloren die Fließgeschwindigkeit innerhalb der beiden erforderlichen Wärmeaustauschmittelkreisläufen so gewählt, dass die Temperatur des Wärmeaustauschmittels von der Eintrittsstelle in die Temperaturzone bis zur Austrittstelle aus der Temperaturzone (bedingt durch die Exothermie der Reaktion) um 0 bis 15˚C ansteigt. D. h., das vorgenannte ΔT kann erfindungsgemäß 1 bis 10˚C, oder 2 bis 8˚C oder 3 bis 6˚C betragen.

Die Eintrittstemperatur des Wärmeaustauschmittels in die Temperaturzone A liegt erfindungsgemäß normalerweise im Bereich von 290 bis 380˚C, bevorzugt im Bereich von 305 bis 365˚C und besonders bevorzugt im Bereich von 310 bis 340˚C bzw. bei 330˚C. Bei Propenbelastungen des Katalysatorfestbetts 2 von ≥ 90 Nl/l·h und ≤ 160 Nl/l·h wird die Eintrittstemperatur des Wärmeaustauschmittels in die Temperaturzone B erfindungsgemäß ebenfalls im Bereich von 290 bis 380˚C, gleichzeitig aber normalerweise erfindungsgemäß zweckmäßig ≥ 0˚C bis ≤ 20˚C, oder ≤ 10˚C, bzw. ≥ 0˚C und ≤ 5˚C, oder häufig ≥ 0˚C und ≤ 3˚C unterhalb der Eintrittstemperatur des in die Temperaturzone A eintretenden Wärmeaustauschmittels liegen. Bei Propenbelastungen des Katalysatorfestbetts 2 von ≥ 160 Nl/l·h und (in der Regel) ≤ 300 Nl/l·h (bzw. 600 Nl/l·h) wird die Eintrittstemperatur des Wärmeaustauschmittels in die Temperaturzone B erfindungsgemäß ebenfalls im Bereich von 290 bis 380˚C, aber normalerweise erfindungsgemäß zweckmäßig ≥ 0˚C bis ≤ 50˚C, oder ≥ 5˚C und ≤ 45˚C, oder ≥ 10˚C und ≤ 40˚C, oder ≥ 15˚C und ≤ 30˚C oder ≤ 35˚C (z. B. 20˚C oder 25˚C) oberhalb der Eintrittstemperatur des in die Temperaturzone A eintretenden Wärmeaustauschmittels liegen.

Es sei an dieser Stelle auch noch einmal darauf hingewiesen, dass für eine Durchführung der Reaktionsstufe 2 des erfindungsgemäßen Verfahrens insbesondere auch der in der DE-AS 22 01 528 beschriebene Zweizonenrohrbündelreaktortyp verwendet werden kann, der die Möglichkeit beinhaltet, vom heißeren Wärmeaustauschmittel der Temperaturzone B eine Teilmenge an die Temperaturzone A abzuführen, um gegebenenfalls ein Anwärmen eines kalten Reaktionsgasausgangsgemisches 2 oder eines kalten Kreisgases zu bewirken. Ferner kann die Rohrbündelcharakteristik innerhalb einer individuellen Temperaturzone wie in der EP-A 382 098 beschrieben gestaltet werden.

Im Übrigen hat es sich als zweckmäßig erwiesen, das die zweite Reaktionsstufe verlassende Produktgasgemisch 2 vor dem Eintritt in die dritte Reaktionsstufe auf direkte und/oder indirekte Weise abzukühlen, um so eine Nachvollverbrennung von Teilen des in der zweiten Reaktionsstufe gebildeten Acroleins zu unterdrücken. üblicherweise wird dazu zwischen die beiden Reaktionsstufen ein Nachkühler geschaltet. Dies kann im einfachsten Fall ein indirekter Rohrbündelwärmeüberträger sein. Das Produktgasgemisch 2 wird dabei in der Regel durch die Rohre geführt und um die Rohre wird ein Wärmetauschermedium geführt, dessen Art der für die Rohrbündelreaktoren empfohlenen Wärmetauschermedien entsprechen kann. Mit Vorteil ist das Rohrinnere mit inerten Füllkörpern (z. B. Spiralen aus Edelstahl, Ringe aus Steatit, Kugeln aus Steatit etc.) gefüllt. Selbige verbessern den Wärmeaustausch und fangen gegebenenfalls aus dem Katalysatorfestbett 2 der zweiten Reaktionsstufe sublimierendes Molybdäntrioxid vor einem Eintritt desselben in die dritten Reaktionsstufe ab. Es ist von Vorteil, wenn der Nachkühler aus mit Zinksilicatfarbe beschichtetem rostfreiem Stahl gefertigt ist.

Die in der zweiten Reaktionsstufe bei einfachem Durchgang resultierende Selektivität der Acroleinbildung sowie der Acrylsäurenebenproduktbildung wird erfindungsgemäß zusammen regelmäßig ≥ 92 mol-% oder ≥ 94 mol-%, häufig ≥ 95 mol-% oder ≥ 96 mol-% bzw. ≥ 97 mol-% betragen.

Anwendungstechnisch zweckmäßig wird das Produktgasgemisch 2 der zweiten Reaktionsstufe im bereits erwähnten Nachkühler auf eine Temperatur von 210 bis 290˚C, häufig 230 bis 280˚C oder 250 bis 270˚C abgekühlt. Dabei kann die Abkühlung des Produktgasgemisches 2 der zweiten Reaktionsstufe durchaus auf Temperaturen erfolgen, die unterhalb der Temperatur der dritten Reaktionsstufe liegen. Die beschriebene Nachkühlung ist jedoch keineswegs zwingend und kann insbesondere dann in aller Regel entfallen, wenn der Weg des Produktgasgemisches 2 von der zweiten Reaktionsstufe in die dritten Reaktionsstufe kurz gehalten wird. Vorteilhaft wird das beschriebene zweistufige Partialoxidationsverfahren ferner so verwirklicht, dass man den Sauerstoffbedarf in der dritten Reaktionsstufe nicht bereits durch einen entsprechend hohen Sauerstoffgehalt des Reaktionsgasausgangsgemisches 2 deckt, sondern den benötigten Sauerstoff im Bereich zwischen zweiter und dritter Reaktionsstufe zugibt ("Sekundärsauerstoffzusatz"). Dies kann vor, während, nach und/oder zur Nachkühlung erfolgen. Als Quelle für den in der dritten Reaktionsstufe erforderlichen molekularen Sauerstoff kommen sowohl reiner Sauerstoff als auch Gemische aus Sauerstoff und Inertgas, z. B. Luft (ist erfindungsgemäß bevorzugt) oder an molekularem Stickstoff entreicherte Luft (z. B. $\geq$ 90 Vol-% $O_2$, $\leq$ 10 Vol-% $N_2$) in Betracht. Die Zugabe der Sauerstoffquelle erfolgt regelmäßig in auf den Reaktionsdruck komprimierter Form. Selbstredend kann beim erfindungsgemäßen Verfahren der Sauerstoffbedarf in der dritten Reaktionsstufe bereits durch einen entsprechend hohen Sauerstoffbedarf in der zweiten Reaktionsstufe gedeckt werden. Natürlich kann bei Bedarf als Sekundärgas auch ein inertes Verdünnungsgas zugesetzt werden.

Wie die Durchführung der zweiten Reaktionsstufe erfolgt auch die Durchführung der dritten Reaktionsstufe des erfindungsgemäßen Verfahrens in anwendungstechnisch zweckmäßiger Weise in einem Zweizonenrohrbündelreaktor, wie er für die zweite Reaktionsstufe bereits beschrieben wurde. Die Ausführungen hinsichtlich des Zweizonenrohrbündelreaktors für die zweite Reaktionsstufe gelten deshalb auch für den Zweizonenrohrbündelreaktor für die dritte Reaktionsstufe.

D. h., in einfacher Weise befindet sich das erfindungsgemäß zu verwendende Katalysatorfestbett 3. (gegebenenfalls einschließlich der Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Temperaturzone.

D. h. in einfacher Weise umströmt z. B. ein Salzbad C diejenigen Abschnitte der Rohre (die Temperaturzone C), in welchem sich die oxidative Umsetzung des Acroleins (beim einfachen Durchgang) bis zum Erreichen eines Umsatzwertes im Bereich von 45 bis 85 % (bevorzugt 50 bis 85 %, besonders bevorzugt 60 bis 85 % des in der dritten Reaktionsstufe für $U^A$ erfindungsgemäß angestrebten Wertes) vollzieht und ein Salzbad D umströmt den Abschnitt der Rohre (die Temperaturzone D), in welchem sich die oxidative Anschlussumsetzung des Acroleins (beim einfachen Durchgang) bis zum Erreichen des erfindungsgemäßen Wertes für $U^A$ vollzieht (bei Bedarf können sich an die erfindungsgemäß anzuwendenden Temperaturzonen C, D weitere Temperaturzonen anschließen, die auf individuellen Temperaturen gehalten werden).

Anwendungstechnisch zweckmäßig umfasst die Reaktionsstufe 3 des erfindungsgemäßen Verfahrens keine weiteren Temperaturzonen. D. h., das Salzbad D umströmt zweckmäßig den Abschnitt der Rohre, in welchem sich die oxidative Anschlussumsetzung des Acroleins (beim einfachen Durchgang) bis zum erfindungsgemäß angestrebten Wert für $U^A$ vollzieht.

Üblicherweise liegt der Beginn der Temperaturzone D hinter dem Heißpunktmaximum der Temperaturzone C

Die beiden Salzbäder C, D können erfindungsgemäß relativ zur Strömungsrichtung des durch die Reaktionsrohre strömenden Reaktiongasgemisches 3 im Gleichstrom oder im Gegenstrom durch den die Reaktionsrohre umgebenden Raum geführt werden. Selbstverständlich kann erfindungsgemäß auch in der Temperaturzone C eine Gleichströmung und in der Temperaturzone D eine Gegenströmung (oder umgekehrt) angewandt werden.

Selbstredend kann man in allen vorgenannten Fallkonstellationen innerhalb der jeweiligen Temperaturzone der, relativ zu den Reaktionsrohren, erfolgenden Parallelströmung der Salzschmelze noch eine Querströmung überlagern, so dass die einzelne Reaktionszone einem wie in der EP-A 700 714 oder in der EP-A 700 893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Üblicherweise sind in den vorgenannten Zweizonen-Rohrbündelreaktoren für die dritte Reaktionsstufe die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 3 bis 4, bevorzugt 3,5 m. In jeder Temperaturzone belegt das Katalysatorfestbett 3 wenigstens 60 %, bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468 290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren der dritten Reaktionsstufe die Fließgeschwindigkeit innerhalb der beiden erforderlichen Wärmeaustauschmittelkreisläufe so gewählt, dass die Temperatur des Wärmeaustauschmittels von der Eintrittstelle in die Temperaturzone bis zur Austrittsstelle aus der Temperaturzone um 0 bis 15˚C ansteigt. D. h., das vorgenannte ΔT kann erfindungsgemäß 1 bis 10˚C, oder 2 bis 8˚C oder 3 bis 6˚C betragen.

Die Eintrittstemperatur des Wärmeaustauschmittels in die Temperaturzone C liegt erfindungsgemäß normalerweise im Bereich von 230 bis 320˚C, bevorzugt im Bereich von 250 bis 300˚C und besonders bevorzugt im Bereich von 260 bis 280˚C. Bei Acroleinbelastungen des Katalysatorfestbett 3 von ≥ 70 Nl/l·h und ≤ 150 Nl/l·h wird die Eintrittstemperatur des Wärmeaustauschmittels in die Temperaturzone D erfindungsgemäß ebenfalls im Bereich von 230 bis 320˚C, gleichzeitig aber normalerweise erfindungsgemäß zweckmäßig ≥ 0˚C bis ≤ 20˚C oder ≤ 10˚C, bzw ≥ 0˚C und ≤ 5˚C, oder häufig ≥ 0˚C und ≥ 3˚C unterhalb der Eintrittstemperatur des in die Temperaturzone C eintretenden Wärmeaustauschmittels liegen. Bei Acroleinbelastung des Katalysatorfestbetts 3 von ≥ 150 Nl/l·h und (in der Regel) ≤ 300 Nl/l·h (bzw. 600 Nl/l·h) wird die Eintrittstemperatur des Wärmeaustauschmittels in die Temperaturzone D erfindungsgemäß ebenfalls im Bereich von 230 bis 320˚C, gleichzeitig aber normalerweise erfindungsgemäß zweckmäßig ≥ 0˚C bis ≤ 4.0˚C, oder ≥ 5˚C und ≤ 35˚C, bzw. 30˚C, oder ≥ 10˚C und ≤ 25˚C, bzw. 20˚C, oder 15˚C oberhalb der Eintrittstemperatur des in die Temperaturzone C eintretenden Wärmeaustauschmittels liegen.

Es sei an dieser Stelle auch noch einmal darauf hingewiesen, dass für eine Durchführung der dritten Reaktionsstufe des beschriebenen Verfahrens insbesondere auch der in der DE-AS 22 01 528 beschriebene Zweizonenrohrbündelreaktortyp verwendet werden kann, der die Möglichkeit beinhaltet, vom heißeren Wärmeaustauschmittel der Temperaturzone D eine Teilmenge an die Temperaturzone C abzuführen, um gegebenenfalls ein Anwärmen eines zu kalten Reaktionsgasausgangsgemisches 3 zu bewirken. Ferner kann die Rohrbündelcharakteristik, innerhalb einer individuellen Reaktionszone wie in der EP-A 382 098 beschrieben gestaltet werden.

Selbstredend können zur Durchführung des beschriebenen Verfahrens zwei Zweizonenrohrbündelreaktoren zu einem Vierzonenrohrbündelreaktor verschmolzen werden, wie es in der WO 01/36364 beschrieben ist. Normalerweise befindet sich in diesen Fällen zwischen dem Katalysatorfestbett 2 und dem Katalysatorfestbett 3 eine Inertschüttung. Allerdings kann auf eine solche Zwischeninertschüttung auch verzichtet werden. Die Länge der Reaktionsrohre entspricht im Verschmelzungsfall vielfach der Summe der Längen der nicht verschmolzenen Rohrbündelreaktoren.

An dieser Stelle sei noch festgehalten, dass als Aktivmassen sowohl für das Katalysatorfestbett 2 als auch für das Katalysatorfestbett 3 auch die Multimetalloxidmassen der DE-A 102 61 186 günstig sind.

Günstige Ausgestaltungen eines Zweizonenrohrbündelreaktors für die zweite Reaktionsstufe können wie folgt beschaffen sein (die konstruktive Detailgestaltung kann wie in den Gebrauchsmusteranmeldungen 202 19 277.6, 2002 19 278.4 und 202 19 279.2 bzw. in den PCT-Anmeldungen PCT/EP02/14187, PCT/EP02/14188 oder PCT/EP02/14189 erfolgen):

Kontaktrohre:

| | |
|---|---|
| Material der Kontaktrohre: | ferritischer Stahl; |
| Abmessungen der Kontaktrohre: | z. B. 3500 mm Länge; |
| | z. B. 30 mm Außendurchmesser; |
| | z. B. 2 mm Wandstärke; |

Anzahl der Kontaktrohre im Rohrbündel: z. B. 30000, oder 28000, oder 32000, oder 34000; zusätzlich bis zu 10 Thermorohre (wie in EP-A 873 783 und EP-A 12 70 065 beschrieben), die wie die Kontaktrohre beschickt sind (schneckenartig von ganz außen nach innen drehend) z. B. der selben Länge und Wandstärke, aber mit einem Außendurchmesser von z. B. 33,4 mm und einer zentrierten Thermohülse von z. B. 10 mm Außendurchmesser und z. B. 1 mm Wandstärke;

Reaktor (Material wie die Kontaktrohre):

Zylinderförmiger Behälter eines Innendurchmessers von 6000 - 8000 mm;

Reaktorhauben plattiert mit Edelstahl des Typs 1,4541; Plattierungsdicke: einige mm;

ringförmig angeordnetes Rohrbündel, z. B. mit einem freien zentralen Raum;

Durchmesser des zentralen freien Raumes: z. B. 1000 - 2500 mm (z. B. 1200 mm, oder 1400 mm, oder 1600 mm, oder 1800 mm, oder 2000 mm, oder 2200 mm, oder 2400 mm);
normalerweise homogene Kontaktrohrverteilung im Rohrbündel (6 äquidistante Nachbarrohre pro Kontaktrohr), Anordnung in gleichseitigem Dreieck, Kontaktrohrteilung (Abstand der zentrischen Innenachsen von zueinander nächstliegenden Kontaktrohren): 35 - 45 mm, z. B. 36 mm, oder 38 mm, oder 40 mm, oder 42 mm, oder 44 mm;
die Kontaktrohre sind mit ihren Enden in Kontaktrohrböden (oberer Boden und unterer Boden z. B. mit einer Dicke von 100 - 200 mm) abdichtend befestigt und münden am oberen Ende in eine mit dem Behälter verbundene Haube, die einen Zulass für das Reaktionsgasausgangsgemisch 2 aufweist; ein z. B. auf der halben Kontaktrohrlänge befindliches Trennblech einer Dicke von 20 - 100 mm, teilt den Reaktorraum symmetrisch in zwei Temperaturzonen A (obere Zone) und B (untere Zone); jede Temperaturzone wird durch eine Umlenkscheibe in 2 äquidistante Längsabschnitte geteilt; die Umlenkscheibe weist bevorzugt Ringgeometrie auf; die Kontaktrohre sind mit Vorteil am Trennblech abdichtend befestigt; an den Umlenkscheiben sind sie nicht abdichtend befestigt, so dass die Querströmungsgeschwindigkeit der Salzschmelze innerhalb einer Zone möglichst konstant ist;

jede Zone wird durch eine eigene Salzpumpe mit Salzschmelze als Wärmeträger versorgt; die Zufuhr der Salzschmelze ist z. B. unterhalb der Umlenkscheibe und die Entnahme ist z. B. oberhalb der Umlenkscheibe;

aus beiden Salzschmelzekreisen wird z. B. ein Teilstrom entnommen und z. B. in einem gemeinsamen oder zwei getrennten indirekten Wärmetauschern abgekühlt (Dampferzeugung);

im ersten Fall wird der abgekühlte Salzschmelzestrom aufgeteilt, mit dem jeweiligen Reststrom vereinigt und durch die jeweilige Pumpe in den entsprechenden Ringkanal, der die Salzschmelze über den Behälterumfang verteilt, in den Reaktor gedrückt;

durch im Reaktormantel befindliche Fenster gelangt die Salzschmelze zum Rohrbündel; die Einströmung erfolgt z. B in radialer Richtung zum Rohrbündel;

die Salzschmelze fließt in jeder Zone der Vorgabe des Umlenkblechs folgend z. B. in der Abfolge

- von außen nach innen,
- von innen nach außen,

um die Kontaktrohre;

durch um den Behälterumfang angebrachte Fenster sammelt sich die Salzschmelze an jedem Zonenende in einem um den Reaktormantel angebrachten Ringkanal, um einschließlich Teilstromkühlung im Kreis gepumpt zu werden; über jede Temperaturzone wird die Salzschmelze von unten nach oben geführt;

Das Reaktionsgasgemisch verlässt den Reaktor der zweiten Stufe mit einer Temperatur wenige Grad höher als die Salzbadeintrittstemperatur des Reaktors. Das Reaktionsgasgemisch wird für die Weiterverarbeitung zweckmäßigerweise in einem separaten Nachkühler, der dem Reaktor der 2. Stufe nachgeschaltet ist, auf 220°C bis 280°C, bevorzugt 240°C bis 260°C abgekühlt.

Der Nachkühler ist in der Regel unterhalb des unteren Rohrbodens angeflanscht und besteht normalerweise aus Rohren mit ferritischem Stahl. In die Rohre des Nachkühlers sind mit Vorteil innen Edelstahl-Blechspiralen, die teil- oder vollgewendelt sein können, zur Verbesserung des Wärmeübergangs eingeführt.

Salzschmelze:

Als Salzschmelze kann ein Gemisch aus 53 Gew.-% Kaliumnitrat, 40 Gew.-% Natriumnitrit und 7 Gew.-% Natriumnitrat verwendet werden; beide Reaktionszonen und der Nachkühler wenden mit Vorteil eine Salzschmelze derselben Zusammensetzung an;

die in den Reaktionszonen umgepumpte Salzmenge kann je Zone ca. 10000 $m^3$/h betragen.

Stromführung:

das Reaktionsgasausgangsgemisch 2 strömt zweckmäßig von oben nach unten durch den Zweitstufenreaktor, während die unterschiedlich temperierten Salzschmelzen der einzelnen Zonen zweckmäßig von unten nach oben gefördert werden;

Kontaktrohr- und Thermorohrbeschickung (von oben nach unten) z. B.:

Abschnitt 1: 50 cm Länge
Steatitringe der Geometrie 7 mm x 7 mm x 4 mm (Außendurchmesser x Länge x Innendurchmesser) als Vorschüttung.

Abschnitt 2: 140 cm Länge
Katalysatorbeschickung mit einem homogenen Gemisch aus 20 Gew.-% an Steatitringen der Geometrie 5 mm x 3 mm x 2 mm (Außendurchmes- ser x Länge x Innendurchmesser) und 80 Gew.-% Vollkatalysator aus Abschnitt 3 (alternativ kann auch ein homogenes Gemisch aus nur 70 Gew.-% Vollkatalysator aus Abschnitt 3 und 30 Gew.-% der vorge- nannten Steatitringe verwendet werden).

Abschnitt 3: 160 cm Länge
Katalysatorbeschickung mit ringförmigem (5 mm x 3 mm x 2 mm = Au- ßendurchmesser x Länge x Innendurchmesser) Vollkatalysator gemäß Beispiel 1 der DE-A 10046957 (Stöchiometrie: $[Bi_2W_2O_9 \times 2\ WO_3]_{0,5}\ [Mo_{12}Co_{5,5}Fe_{2,94}Si_{1,59}\ K_{0,08}O_x]_1$).

Günstige Ausgestaltungen eines Zweizonenrohrbündelreaktors für die dritte Reaktionsstufe können wie folgt beschaffen sein:

Alles wie beim Zweizonenrohrbündelreaktor für die zweite Reaktionsstufe. Die Dicke des oberen und unteren Kontaktrohrbodens beträgt jedoch häufig 100 - 200 mm, z. B. 110 mm, oder 130 mm, oder 150 mm, oder 170 mm, oder 190 mm.

Der Nachkühler entfällt; statt dessen münden die Kontaktrohre mit ihren unteren Öffnungen in eine am unteren Ende mit dem Behälter verbundene Haube mit Auslaß für das Produktgasgemisch; die obere Temperaturzone ist die Zone C und die untere Temperaturzone ist die Temperaturzone D. Zwischen Auslaß "Nachkühler" und Einlass "Reaktor für die dritte Reaktionsstufe" besteht zweckmäßig eine Zufuhrmöglichkeit für komprimierte Luft.

Die Kontaktrohr- und Thermorohrbeschickung (von oben nach unten) kann z. B. wie folgt sein:

Abschnitt 1: 20 cm Länge
Steatitringe der Geometrie 7 mm x 7 mm x 4 mm (Außendurchmesser x Länge x Innendurchmesser) als Vorschüttung.

Abschnitt 2: 90 cm Länge
Katalysatorbeschickung mit einem homogenen Gemisch aus 20 Gew.-% an Steatitringen der Geometrie 7 mm x 3 mm x 4 mm (Außendurchmes- ser x Länge x Innendurchmesser) und 80 Gew.-% Schalenkatalysator aus Abschnitt 4 (alternativ kann auch ein homogenes Gemisch aus nur 70 Gew.% Schalenkatalysator aus Abschnitt 4 und 30 Gew.-% der vor- genannten Steatitringe verwendet werden).

Abschnitt 3: 50 cm Länge
Katalysatorbeschickung mit einem homogenen Gemisch aus 15 Gew.-% an Steatitringen der Geometrie 7 mm x 3 mm x 4 mm (Außendurchmes- ser x Länge x Innendurchmesser) und 85 Gew.-% Schalenkatalysator aus Abschnitt 4 (alternativ kann auch ein homogenes Gemisch aus nur 80 Gew.-% Schalenkatalysator aus Abschnitt 4 und 20 Gew.-% der vor- genannten Steatitringe verwendet werden).

Abschnitt 4: 190 cm Länge
Katalysatorbeschickung mit ringförmigem (7 mm x 3 mm x 4 mm = Au- ßendurchmesser x Länge x Innendurchmesser) Schalenkatalysator ge- mäß Herstellungsbeispiel 5 der DE-A 100 46 928 (Stöchiometrie: $MO_{12}V_3W_{1,2}CU_{2,4}O_x$).

Die Drittstufen-Kontaktrohr- und Thermorohrbeschickung kann (von oben nach unten) auch so aussehen:

Abschnitt 1: 20 cm Länge
Steatitringe der Geometrie 7 mm x 7 mm x 4 mm (Außendurchmesser x Länge x Innendurchmesser) als Vorschüttung.

Abschnitt 2: 140 cm Länge
Katalysatorbeschickung mit einem homogenen Gemisch aus 20 Gew.-% an Steatitringen der Geometrie 7 mm x 3 mm x 4 mm (Außendurchmes- ser x Länge x Innendurchmesser) und 80 Gew.-% Schalenkatalysator aus Abschnitt 3 (alternativ kann auch ein homogenes Gemisch aus nur 75 Gew.-% Schalenkatalysator aus Abschnitt 3 und 25 Gew.-% der vor- genannten Steatitringe verwendet werden).

Abschnitt 3: 190 cm Länge
Katalysatorbeschickung mit ringförmigem (7 mm x 3 mm x 4 mm = Au- ßendurchmesser x Länge x Innendurchmesser) Schalenkatalysator ge- mäß Herstellungsbeispiel 5 der DE-A 100 46 928 (Stöchiometrie: $MO_{12}V_3W_{1,2}Cu_{2,4}O_x$).

In den genannten Zweitstufenbeschickungen kann der Vollkatalysator aus Beispiel 1 der DE-A 100 46 957 auch ersetzt werden durch:

a) einen Katalysator gemäß Beispiel 1c aus der EP-A 15 565 oder einen gemäß diesem Beispiel herzustellenden Katalysator, der jedoch die Aktivmasse $Mo_{12}Ni_{6,5}Zn_2Fe_2Bi_1P_{0,0065}K_{0,06}O_x \cdot 10\ SiO_2$ aufweist;

b) Beispiel Nr. 3 aus der DE-A 198 55 913 als Hohlzylindervollkatalysator der Geometrie 5mm x 3mm x 2mm bzw.

5mm x 2mm x 2mm;

c) Multimetalloxid II - Vollkatalysator gemäß Beispie 1 der DE-A 197 46 210;

d) einen der Schalenkatalysatoren 1, 2 und 3 aus der DE-A 100 63 162, jedoch bei gleicher Schalendicke auf Trägerringe der Geometrie 5 mm x 3 mm x 1,5 mm bzw. 7 mm x 3 mm x 1,5 mm aufgebracht;

e) die Katalysatoren, insbesondere die Ausführungsbeispiele, der DE-A 10344149 und der DE-A 10353954.

In allen vorgenannten Drittstufenbeschickungen kann der Schalenkatalysator gemäß Herstellungsbeispiel 5 der DE-A 100 46 928 ersetzt werden durch:

a) Schalenkatalysator S1 oder S7 aus der DE-A 4442346 mit einem Aktivmassenanteil von 27 Gew.-% und einer Schalendicke von 230 $\mu$m;

b) einem Schalenkatalysator gemäß den Beispielen 1 bis 5 aus der DE-A 198 15 281, jedoch auf Trägerringe der Geometrie 7 mm x 3 mm x 4 mm mit einem Aktivmassenanteil von 20 Gew.-% aufgebracht;

c) Schalenkatalysator mit zweiphasiger Aktivmasse der Stöchiometrie $(Mo_{10,4}V_3W_{1,2}O_x)$ $(CuMo_{0,5}W_{0,5}O_4)_{1,6}$, hergestellt gemäß DE-A 197 36 105 und mit einem Aktivmassenanteil von 20 Gew.-% auf den vorgenannten 7 mm x 3 mm x 4 mm Träger aufgebracht.

Im Übrigen werden das Katalysatorfestbett 2 und das Katalysatorfestbett 3 erfindungsgemäß zweckmäßig so gewählt (z. B. durch Verdünnung mit z. B. Inertmaterial), dass der Temperaturunterschied zwischen dem Heißpunktmaximum des Reaktionsgasgemisches in den einzelnen Reaktionszonen und der jeweiligen Temperatur der Reaktionszone-in der Regel 80˚C nicht überschreitet. Meist beträgt dieser Temperaturunterschied $\leq$ 70˚C, häufig liegt er bei 20 bis 70˚C, vorzugsweise ist dieser Temperaturunterschied gering. Außerdem werden die Katalysatorfestbetten aus Sicherheitsgründen in dem Fachmann an sich bekannter Weise so gewählt (z. B. durch Verdünnung mit z. B. Inertmaterial), dass die "peak-to-salt-temperature sensitivity" gemäß Definition in der EP-A 1106598 $\leq$ 9˚C, oder $\leq$ 7˚C, oder $\leq$ 5˚C, oder $\leq$ 3˚C beträgt.

Nachkühler und Reaktor für die dritte Stufe sind durch ein Verbindungsrohr verbunden, dessen Länge weniger als 25 m beträgt.

In der vorstehenden Reaktoranordnung können in der dritten Reaktionsstufe die ringförmigen Verdünnungsformkörper und die ringförmigen Katalysatorformkörper auch durch kugelförmige Verdünnungsformkörper und kugelförmige Katalysatorformkörper (jeweils mit Radius 2 bis 5 mm und mit einem Aktivmassenanteil von 10 bis 30 Gew.-%, häufig 10 bis 20 Gew.-%) ersetzt werden.

Das das erfindungsgemäße Verfahren 3 nach der dritten Reaktionsstufe verlassende Produktgasgemisch 3 ist in der Regel im wesentlichen zusammengesetzt aus dem Zielprodukt Acrylsäure, nicht umgesetztem molekularem Sauerstoff (mit Blick auf die Lebensdauer der verwendeten Katalysatoren ist es günstig, wenn der Sauerstoffgehalt im Produktgasgemisch 3 noch wenigstens 1,5 bis 4 Vol.-% beträgt, Propan, nicht umgesetztem Propylen, molekularem Stickstoff, als Nebenprodukt entstandenem und/oder als Verdünnungsgas mitverwendetem Wasserdampf, als Nebenprodukt und/oder als Verdünnungsgas mitverwendeten Kohlenoxiden, sowie geringen Mengen sonstiger niederer Aldehyde, niederer Alkancarbonsäuren (z. B. Essigsäure, Ameisensäure und Propionsäure) sowie Maleinsäureanhydrid, Benzaldehyd, aromatische Carbonsäuren und aromatische Carbonsäureanhydride (z.B. Phthalsäureanhydrid und Benzoesäure), gegebenenfalls weiteren Kohlenwasserstoffen, wie z. B. C4-Kohlenwasserstoffe (z. B. Buten-1 und eventuell sonstige Butene), und anderen inerten Verdünnungsgasen.

Das Zielprodukt kann aus dem Produktgasgemisch 3 in an sich bekannter Weise in einer Trennzone abgetrennt werden (z. B. durch partielle oder vollständige sowie gegebenenfalls fraktionierende Kondensation der Acrylsäure oder durch Absorption von Acrylsäure in Wasser oder in einem hochsiedenden hydrophoben organischen Lösungsmittel sowie anschließende Aufarbeitung der Kondensate und/oder Absorbate; erfindungsgemäß bevorzugt wird das Produktgasgemisch 3 fraktionierend kondensiert werden; vgl. z. B. EP-A 13 88 533, EP-A 13 88 532, DE-A 102 35 847, EP-A 79 28 67, WO 98/01415, EP-A 10 15 411, EP-A 10 15 410, WO 99/50219, WO 00/53560, WO 02/09839, DE-A 102 35 847, WO 03/041833, DE-A 102 23 058, DE-A 102 43 625, DE-A 103 36 386, EP-A 85 41 29, US-A 4,317,926, DE-A 198 37 520, DE-A 196 06 877, DE-A 190 50 1325, DE-A 102 47 240, DE-A 197 40 253, EP-A 69 57 36, EP-A 98 22 87, EP-A 10 41 062, EP-A 11 71 46, DE-A 43 08 087, DE-A 43 35 172, DE-A 44 36 243, DE-A 19 924 532, DE-A 103 32 758 sowie DE-A 19 924 533). Eine Acrylsäureabtrennung kann auch wie in der EP-A 98 22 87, der EP-A 98 22 89, der DE-A 103 36 386, der DE-A 101 15 277, der DE-A 196 06 877, der DE-A 197 40 252, der DE-A 196 27 847, der EP-A 92 04 08, der. EP-A 10 68 174, der EP-A 10 66 239, der EP-A 10 66 240, der WO 00/53560, der WO 00/53561, der DE-A 100.53 086 und der EP-A 98 22 88 vorgenommen werden. Vorzugsweise wird wie in Fig. 7 der WO/0196271 bzw. wie in der DE-A 10 2004 032 129 und deren äquivalenten Schutzrechten beschrieben abgetrennt. Günstige Abtrennweisen sind auch die in den Schriften WO 2004/063138, WO 2004/035514, DE-A 102 43 625 und DE-A 102 35 847 beschriebenen Verfahren. Die Weiterverarbeitung einer dabei gewonnenen rohen Acrylsäure kann z. B. wie in den Schriften WO 01/77056, WO 03/041832, WO 02/055469, WO 03/078378 und WO 03/041833 beschrieben erfolgen.

Gemeinsames Merkmal der vorgenannten Trennverfahren ist (wie eingangs bereits erwähnt), dass am Kopf der jeweiligen trennwirksame Einbauten enthaltenden Trennkolonne, in deren unteren Teil das Produktgemisch 3, normalerweise nach vorheriger direkter und/oder indirekter Kühlung desselben, zugeführt wird, normalerweise ein Restgasstrom verbleibt, der im wesentlichen diejenigen Bestandteile des Produktgasgemischs 3 enthält, deren Siedepunkt bei Normaldruck (1 bar) ≤ -30˚C beträgt (d. h., die schwer kondensierbaren oder auch leicht flüchtigen Bestandteile).

Im unteren Teil der Trennkolonne fallen normalerweise die schwerer flüchtigen Bestandteile des Produktgasgemischs 3, einschließlich des Zielprodukts Acrylsäure, in kondensierter Phase an.

Die Restgasbestandteile sind in erster Linie Propan, in der Partialoxidation nicht umgesetztes Propylen, molekularer Sauerstoff sowie häufig die in der Partialoxidation sonstigen mitverwendeten inerten Verdünnungsgase, wie z. B. Stickstoff und Kohlendioxid. Wasserdampf kann je nach angewandtem Trennverfahren im Restgas nur noch in Spuren oder in Mengen von bis zu 20 Vol.-% oder mehr enthalten sein.

Das in diesem (Haupt)Restgas enthaltene Propan und Propylen wird, wie bereits beschrieben, erfindungsgemäß rückgeführt.

Bei der Aufarbeitung der kondensierten Phase (zum Zweck der Abtrennung des Zielproduktes) können weitere restliche Gase anfallen, da erfindungsgemäß vorteilhaft versucht werden wird, die insgesamt im Produktgasgemisch 3 bzw. 2 enthaltene Menge an nicht umgesetztem Propan und Propylen in die erste Reaktionsstufe (z. B. in die heterogen katalysierte Propandehydrierung und/oder Propanoxidehydrierung) zurückzuführen. Diese enthalten in der Regel zwar noch Propan sowie Propylen, häufig jedoch keinen molekularen Sauerstoff mehr. Üblicherweise werden sie mit dem Hauptrestgas zu einem Gesamtrestgas vereint in die erste Reaktionsstufe (z. B. in die heterogen katalysierte Propandehydrierung und/oder Propanoxidehydrierung) rückgeführt. Es ist aber auch eine separate Rückführung dieser Restgase, z.B. auch in die zweite und/oder dritte Reaktionsstufe, möglich.

Durch die vorzugsweise vollständige Rückführung des verbliebenen Propan und Propylen kann so im kontinuierlichen Betrieb eine kontinuierliche Umsetzung von Propan zu Acrylsäure erfolgen.

Erfindungsgemäß wesentlich ist dabei, dass durch die beschriebene Rückführung in die erste Reaktionsstufe in letzterer trotz beschränktem Propylenumsatz in der Reaktionsstufe 2 über das Gesamtverfahren eine Überführung von Propan zu Propylen mit nahezu hundertprozentiger Selektivität erreichbar ist.

Die Vorteilhaftigkeit einer solchen Verfahrensweise ist dabei sowohl bei niederen (≤ 30 mol-%) als auch bei hohen (≥ 30 mol-%) Dehydrierumsätzen (bezogen auf einmaligen Durchgang von Frischpropan durch die Dehydrierung) gegeben. Generell ist es bei einer solchen Rückführung von Oxidationskreisgas günstig, wenn der Wasserstoffgehalt im Reaktionsgasausgangsgemisch 1 in einem wenigstens stöchiometrischen Verhältnis (bezüglich einer Sauerstoffverbrennung zu Wasser) zur über Oxidationskreisgas ins Reaktionsgasausgangsgemisch 1 zurückgeführten Sauerstoffmenge steht.

Die erfindungsgemäße Kreisgasfahrweise ist in entsprechender Weise anwendbar, wenn die Partialoxidation eine partielle Ammoxidation von Propen zu Acrylnitril ist. Es ist auch dann entsprechend anwendbar, wenn in der Dehydrierung das Propan durch iso-Butan ersetzt wird und das dabei resultierende iso-Buten in entsprechender Weise in einer Partialoxidation zu Methacrolein und/oder Methacrylsäure partialoxidiert wird.

Ein Vorteil der erfindungsgemäßen Verfahrensweise besteht grundsätzlich darin, dass an allen Stellen dieser Schrift, einschließlich des nachfolgenden Ausführungsbeispiels, dort wo mit Inertmaterial verdünnte Katalysatorbeschickungen beschrieben und/oder gefordert werden, die entsprechenden Katalysatoren bei gleicher Bettlänge auch unverdünnt eingesetzt (verwendet) werden können.

An dieser Stelle sei auch nochmals festgehalten, dass die Abtrennung von Acrylsäure aus einem erfindungsgemäß erhaltenen Produktgasgemisch 3 (insbesondere auch aus dem Produktgasgemisch 3 des Ausführungsbeispiels dieser Schrift) bevorzugt so erfolgt, dass man das zuvor gegebenenfalls durch direkte und/oder indirekte Kühlung abgekühlte Produktgasgemisch 3 in einer trennwirksame Einbauten enthaltenden Kolonne unter Seitenabzug einer rohen Acrylsäure in sich selbst aufsteigend fraktionierend kondensiert und/oder mittels Wasser und/oder wässriger Lösung absorbiert, wie es die WO 2004/035514 und die DE-A 10243625 beispielhaft beschreiben. Die entnommene rohe Acrylsäure wird nachfolgend bevorzugt einer Suspensionskristallisation unterworfen und das dabei gebildete Acrylsäuresuspensionskristallisat bevorzugt mittels einer Waschkolonne von verbliebener Mutterlauge abgetrennt. Mit Vorteil wird dabei als Waschflüssigkeit die Schmelze von in der Waschkolonne vorab abgetrennten Acrylsäurekristallen verwendet. Ferner ist die Waschkolonne bevorzugt eine solche mit erzwungenem Transport des Kristallbetts. Besonders bevorzugt handelt es sich um eine hydraulische oder um eine mechanische Waschkolonne. Im einzelnen kann der Beschreibung der WO 01/77056, der WO 03/041832 sowie der WO 03/041833 gefolgt werden. D.h., vorzugsweise wird verbliebene Mutterlauge in die fraktionierende Kondensation rückgeführt (vgl. auch EP-A 1015410). Der Nebenkomponentenauslaß ist normalerweise unterhalb des Seitenabzugs der rohen Acrylsäure als purge-Strom. Unter Anwendung von lediglich einer Kristallisationsstufe ist so Acrylsäure mit einer Reinheit ≥ 99,8 Gew.-% erhältlich, die sich in hervorragender Weise zur Herstellung von Superabsorbern auf der Basis von Poly-Na-Acrylat eignet.

Beispiel (alle Drücke sind wie stets in dieser Schrift Absolutdrücke, soweit nichts anderes explizit erwähnt wird)

A) Gestaltung der Reaktionsstufe 1 (der Dehydrierstufe)

Die Dehydrierstufe bestand aus drei hintereinander geschalteten identischen Rohrreaktoren, die in identischer Weise mit Dehydrierkatalysator beschickt waren.

Der einzelne Rohrreaktor war ein Stahlrohr (Edelstahl der DIN Werkstoffnummer 1.4841) der Länge 1300 mm, der Wanddicke 3,6 mm und des Innendurchmessers 53,1 mm. Die Rohrreaktoren wurden vom Reaktionsgasgemisch 1 jeweils von oben nach unten durchströmt.

Am unteren Ende jedes Rohrreaktors befand sich ein Tragerost aus demselben Edelstahl. Auf dem Tragerost befand sich von unten nach oben die folgende Beschickung:

175 mm    Schüttlänge aus Steatitkugeln (Durchmesser 4-5 mm) des Steatits C-220 der Fa. CeramTec;

21 mm    Schüttlänge aus Steatitkugeln (Durchmesser 1,5-2,5 mm) des Steatits C- 220 der Fa. CeramTec;

210 mm    Schüttlänge Dehydrierkatalysator (Pt/Sn-Legierung, die mit den Elementen Cs, K und La in oxidischer Form promoviert war und die auf die äußere und innere Oberfläche von $ZrO_2 \cdot SiO_2$ Mischoxidträgerstränglingen (mittlere Länge (gaußverteilt im Bereich von 3 mm bis 12 mm mit Maximum bei ca. 6 mm): 6 mm, Durchmesser: 2 mm) in der Elementstöchiometrie (Massen- verhältnisse einschließlich Träger) $Pt_{0,3}Sn_{0,6}La_{3,0}Cs_{0,5}K_{0,2}$ $(ZrO_2)_{88,3}(SiO_2)_{7,1}$ aufgebracht war (Katalysatorvorläuferherstellung und Aktivierung zum akti- ven Katalysator wie in Beispiel 4 der DE-A 10 219 879).

21 mm    Schüttlänge Steatitkugeln (Durchmesser 1,5-2,5 mm) des Steatits C-220 der Fa. CeramTec; und

abschließend auf der Restlänge des Rohrreaktors wieder eine Schüttung aus Steatitkugeln (Durchmesser 4-5 mm) des Steatits C-220 der Fa. CeramTec.

Jeder der Rohrreaktoren war außen im Sinne einer Vorheizstrecke auf den ersten 500 mm Rohrlänge von oben nach unten (zum Tragerost hin) in zwei, eine Gleichverteilung der zugeführten Wärmemenge gewährleistenden, Halbschalen aus Kupfer (Schalendicke = 200 mm) eingelegt, die mittels einer sie im vollen Umfang umgebenden Heizmanschette (Fa. Horst, DE-Heidelberg, 500 mm Länge, 100 mm Innendurchmesser) elektrisch beheizt wurden.

Von unten nach oben war jeder der Rohrreaktoren im Sinne einer adiabaten Strecke auf einer Länge von 600 mm in jeweils zwei Paare von thermisch isolierend wirkenden Halbschalen (Dicke einer Halbschale = 25 mm) aus MPS-Super G der Fa. Microtherm in DE, die um 90˚ gegeneinander versetzt übereinander angebracht waren, eingebracht. Die isolierend wirkenden Halbschalen waren ihrerseits von einer zylindrischen Einhüllenden aus Edelstahl (Außendurchmesser = 173 mm, Innendurchmesser = 167 mm) umgeben, an die zum Zweck der Begleitheizung eine Heizmanschette (Länge = 675 mm, Innendurchmesser = 173 mm) der Fa. Horst, DE-Heidelberg, angelegt war. Auf diese Weise konnte auf der adiabaten Strecke der Wärmefluss aus der Umgebung in das Reaktionsrohr hinein und aus dem Reaktionsrohr heraus in die Umgebung minimiert werden.

In jedes Reaktionsrohr war mittig zusätzlich eine 1370 mm lange Thermohülse eingeführt (Außendurchmesser: 6mm, Innendurchmesser: 4mm ), in die ein Mehrfach-Thermoelement (vom unteren Reaktorende nach oben alle 4 cm insgesamt 10 Messstellen, Dicke 3,2mm) eingeführt war.

Vor jeden einzelnen Rohrreaktor war ein mit Steatitringen ( aus Steatit C-220 der Fa. CeramTec und von der Geometrie 7 mm x 3 mm x 3 mm = Außendurchmesser x Innendurchmesser x Höhe) gefülltes Stahlrohr der Länge 1300 mm als Erhitzer geschaltet. In ihm wurde das Reaktionsgasgemisch 1 jeweils auf die Eintrittstemperatur des nachfolgenden Rohreaktors vorgeheizt und gleichzeitig ideal durchmischt. Zu diesem Zweck wurden die Erhitzerrohre (Edelstahl der DIN Werkstoffnummer 1.4841, Wanddicke 3,6 mm, Innendurchmessers 53,1 mm) auf einer rohrmittigen Länge von 1200 mm mittels um sie angelegter Heizmanschetten der Fa. Horst, DE-Heidelberg elektrisch beheizt. Die Verbindung zwischen Erhitzer und Rohrreaktoren wurde durch mit üblichen Wärmedämmmaterialien thermisch isolierten Edelstahlrohren (Edelstahl der DIN Werkstoffnummer 1.4841, Außendurchmesser 21.3 mm, Innendurchmesser 16.1 mm, Länge ca. 700 mm) bewerkstelligt.

Vor dem Eintritt des Reaktionsgasgemischs 1 in den jeweiligen Erhitzer war ein Zufuhrhahn angebracht, über den dem Reaktionsgasgemisch 1 jeweils Druckluft zugeführt werden konnte. Beschrieben wird nachfolgend der stationäre Betrieb.

Dem ersten Dehydrierreaktor wurde ein Reaktionsgasausgangsgemisch 1 aus 300 g/h Roh-Propan (erster Propan enthaltender Zufuhrstrom), 375 g/h Wasser und 3768 g/h Gesamt-C3-Kreisgas (zweiter (nicht umgesetztes) Propan (und nicht umgesetztes Propylen) enthaltender Zufuhrstrom) mit einer Temperatur von 400°C und einem Druck von 2,6 bar absolut zugeführt (im großtechnischen Betrieb würde der Eingangsdruck zweckmäßig ca. 0,5 bar höher gewählt, um dem erhöhten Druckverlust (bedingt durch höhere Strömungsgeschwindigkeiten) in der Reaktionsstufe 1 Rechnung zu tragen)

Das Roh-Propan enthielt:

|  | Vol.-% |
|---|---|
| Methan | 0 |
| Ethan | 0,156 |
| Ethen | 0 |
| Propan | 96,18 |
| Propen (Propylen) | 0,002 |
| $H_2$ | 0 |
| $O_2$ | 0 |
| $N_2$ | 1,70 |
| CO | 0 |
| $CO_2$ | 0 |
| iso-Butan | 1,245 |
| n-Butan | 0,711 |
| trans-Buten | 0,0005 |
| iso-Buten | 0 |
| cis-Buten | 0,0015 |
| 1-Buten | 0,0048 |
| Butadien | 0 |

Das Gesamt-$C_3$-Kreisgas enthielt:

|  | Vol.-% |
|---|---|
| Methan | 0,009 |
| Ethan | 0,88 |
| Ethen | 0,038 |
| Propan | 29,56 |
| Propen | 0,122 |
| $H_2$ | 0,050 |
| $O_2$ | 3,35 |
| $N_2$ | 64,05 |
| CO | 0,538 |
| $CO_2$ | 1,85 |
| iso-Butan | 0,234 |
| n-Butan | 0,098 |
| trans-Buten | 0,00005 |
| iso-Buten | 0,00051 |

(fortgesetzt)

|  | Vol.-% |
| --- | --- |
| cis-Buten | 0,00144 |
| 1-Buten | 0,00048 |
| Butadien | 0,0087 |

Die Bestimmung der Zusammensetzung des Roh-Propans und aller anderen Gaszusammensetzungen erfolgte gaschromatographisch [HP 6890 mit Chem-Station, Detektoren: FID, WLD, Trennsäulen: $Al_2O_3$/KCL (Chrompack), Carboxen 1010 (Supelco)]. Bei Wasserdampf enthaltenden Gasgemischen wurde dieses vorab der gaschromatographischen Analyse durch Abkühlen und gegebenenfalls Entspannen in einem Wasserabscheider auskondensiert. Das nicht kondensierte verbleibende Gas wurde analysiert und auf dieses trocken gerechnete Gas (d.h., die im eigentlich zu analysierenden Gasgemisch enthaltene Wasserdampfmenge blieb unberücksichtigt) beziehen sich alle Angaben.

Das Reaktionsgasausgangsgemisch 1 wurde in einem Verdampfer erzeugt, der dem ersten Erhitzer vorgeschaltet war. Der Verdampfer selbst war ebenfalls wie ein Erhitzer ausgeführt. Ihm wurden 300 g/h gasförmiges Roh-Propan (65˚C, 5 bar), 3768 g/h Gesamt-$C_3$-Kreisgas (50˚C, 2,8 bar) und 375 g/h Wasser (20˚C, 3 bar) zugeführt. Die Beheizung des Verdampfers war auf eine Gasgemischaustrittstemperatur von 200˚C geregelt. Der Verdampfer war mit dem ersten Erhitzer in entsprechender Weise verbunden wie die Reaktoren an die Erhitzer angebunden waren.

Die Beheizung des ersten Erhitzers war so geregelt, dass das vom Verdampfer in den ersten Erhitzer geleitete Gasgemisch den ersten Erhitzer mit einer Temperatur von 400˚C verließ (die dazu erforderliche Wandtemperatur betrug ca. 440˚C). Dann wurde das Reaktionsgasausgangsgemisch 1 in den ersten Rohrreaktor geführt und in der Vorheizstrecke desselben auf eine Reaktionszoneneintrittstemperatur von 460˚C weiter erhitzt.

Die Temperatur des Reaktionsgasgemischs 1 durchlief beim Durchgang durch den ersten Rohrreaktor eine Maximum (Heißpunkttemperatur genannt) von 549,1˚C (die hier angegebenen quantitativen Angaben beziehen sich auf den Betriebszustand nach 200 Betriebsstunden; im weiteren Verlauf des Betriebs wurden die verschiedenen Temperaturen so nachgezogen dass der auf Einmaldurchgang bezogene Umsatz und die Raum-Zeit-Ausbeute im wesentlichen konstant blieben; in entsprechender Weise wurde auch bereits in den ersten 200 Betriebsstunden verfahren), das im Verlauf des kontinuierlichen Betriebs der Versuchsanlage infolge allmählicher Katalysatordeaktivierung in Strömungsrichtung wanderte (die Wanderungsgeschwindigkeit betrug ca. 0,03 mm/h).

Das den ersten Dehydrierreaktor verlassende Reaktionsgasgemisch 1 wies folgende Gehalte auf:

|  | Vol.-% |
| --- | --- |
| Methan | 0,045 |
| Ethan | 0,109 |
| Ethen | 0,042 |
| Propan | 30,3 |
| Propen | 2,88 |
| $H_2$ | 5,00 |
| $O_2$ | 0 |
| $N_2$ | 59,13 |
| CO | 0,06 |
| $CO_2$ | 3,24 |
| iso-Butan | 0,257 |
| n-Butan | 0,116 |
| trans-Buten | 0,05 |
| iso-Buten | 0,001 |
| cis-Buten | 0,004 |
| 1-Buten | 0,001 |

(fortgesetzt)

|  | Vol.-% |
|---|---|
| Butadien | 0,003 |

Seine Temperatur betrug 509˚C und sein Druck lag bei ca. 2,56 bar.

Vor dem Eintritt in den nachfolgenden Erhitzer wurden dem Reaktionsgasgemisch 1 80 Nl/h Druckluft zudosiert (23˚C, 4,2 bar).

Dann wurde das Reaktionsgasgemisch 1 mittels der elektrischen Beheizungsmöglichkeiten des Erhitzers (Wandtemperatur ca. 540˚C) und der Vorheizstrecke des nachfolgenden (zweiten) Reaktionsrohres (Wandtemperatur ca. 560˚C) auf 465˚C (Eintritt 2. Reaktionszone) erwärmt. Der Druck des Reaktionsgasgemischs 1 an dieser Stelle lag bei 2,56 bar.

Beim Durchgang durch den zweiten Rohrreaktor durchlief die Temperatur des Reaktionsgasgemischs 1 ein Maximum von ca. 560˚C, das im Verlauf des kontinuierlichen Betriebs der Versuchsanlage infolge allmählicher Katalysatordeaktivierung in Strömungsrichtung wanderte (die Wanderungsgeschwindigkeit betrug ca. 0,1mm/h). Das den zweiten Dehydrierreaktor verlassende Reaktionsgasgemisch 1 wies folgende Gehalte auf:

|  | Vol % |
|---|---|
| Methan | 0,078 |
| Ethan | 0,144 |
| Ethen | 0,063 |
| Propan | 26,6 |
| Propen | 4,94 |
| $H_2$ | 6,43 |
| $O_2$ | 0 |
| $N_2$ | 58,58 |
| CO | 0,384 |
| $CO_2$ | 3,58 |
| iso-Butan | 0,22 |
| n-Butan | 0,094 |
| trans-Buten | 0,063 |
| iso-Buten | 0,001 |
| cis-Buten | 0,01 |
| 1-Buten | 0 |
| Butadien | 0,004 |

Seine Temperatur betrug ca. 493˚C und sein Druck lag bei ca. 2,52 bar.

Vor dem Eintritt in den nachfolgenden Erhitzer wurden dem Reaktionsgasgemisch 1 98 Nl/h Druckluft zudosiert (23˚C, 4,2 bar).

Dann wurde das Reaktionsgasgemisch 1 mittels der elektrischen Beheizungsmöglichkeiten des Erhitzers (Wandtemperatur ca. 540˚C) und der Vorheizstrecke des nachfolgenden (dritten) Reaktionsrohres (Wandtemperatur ca. 540˚C) auf 521˚C (Eintritt 3. Reaktionszone) erwärmt. Der Druck des Reaktionsgasgemischs 1 an dieser Stelle lag bei 2,52 bar.

Beim Durchgang durch den dritten Rohrreaktor durchlief die Temperatur des Reaktionsgasgemischs 1 ein Maximum von 570˚C, das im Verlauf des kontinuierlichen Betriebs der Versuchsanlage infolge allmählicher Katalysatordeaktivierung in Strömungsrichtung wanderte (die Wanderungsgeschwindigkeit betrug ca. 0,1 mm/h). Das den dritten Dehydrierreaktor verlassende Reaktionsgasgemisch 1 wies folgende Gehalte auf:

|  | Vol % |
|---|---|
| Methan | 0,1046 |
| Ethan | 0,144 |
| Ethen | 0,0743 |
| Propan | 25,22 |
| Propen | 5,51 |
| $H_2$ | 4,69 |
| $O_2$ | 0 |
| $N_2$ | 60,10 |
| CO | 0,237 |
| $CO_2$ | 3,54 |
| iso-butan | 0,201 |
| n-Butan | 0,085 |
| trans-Buten | 0,070 |
| iso-Buten | 0,0013 |
| cis-Buten | 0,011 |
| 1-Buten | 0,0004 |
| Butadien | 0,0056 |

Seine Temperatur betrug 480,4˚C und sein Druck lag bei 2,48bar.

Damit ergab sich über die Dehydrierstufe (Reaktionsstufe 1) ein auf Einmaldurchgang des Reaktionsausgangsgasgemischs 1 bezogener Gesamtdehydrierumsatz des Propans von 19,91 mol-%.

Bei fortgeschrittener Deaktivierung der Dehydrierkatalysatorschüttungen wurde das Verfahren unterbrochen und diese wie in der DE-A 10028582 beschrieben regeneriert. Dies war im wesentlichen immer dann der Fall, wenn die Heißpunkttemperatur in allen drei Rohrreaktoren ca. 580˚C betrug.

In überraschender Weise schritt die Deaktivierung der Dehydrierkatalysatorschüttungen bei erhöhtem Arbeitsdruck langsamer voran.

B) Gestaltung des Nebenkomponentenauslass

Durch Direktkühlung mit versprühtem gekühltem Wasser (T = 20˚C) wurde das den dritten Dehydrierreaktor verlassende Produktgasgemisch 1 in einem Direktkühler (Quench) im Gleichstrom auf 40˚C abgekühlt (das dabei gasförmig verbleibende Gemisch wurde in der Gegenrichtung zur Produktgaseinströmrichtung aus dem Wasserquench herausgeführt). Etwa 75 Gew.-% des im Produktgasgemisch 1 enthaltenen Wasserdampf (Wasserdampf wurde dem Reaktionsgasausgangsgemisch 1 zugesetzt und bildete sich in der Dehydrierstufe durch Verbrennung von Wasserstoff sowie möglicherweise Kohlenwasserstoff mit Luftsauerstoff; die Verbrennungswärme hielt die Reaktionstemperatur im Reaktionsgasgemisch 1 in der Reaktionsstufe 1 weitgehend aufrecht) kondensierte bei der Direktkühlung aus. Das sich dabei bildende Kondensat wurde mittels Standregelung aus dem Wasserquench heraus- und seiner Entsorgung zugeführt. Im übrigen wurde das zur Direktkühlung eingesetzte Wasser im Kreis geführt (gepumpt), durch indirekten Wärmeaustausch rückgekühlt und zum Zweck der Direktkühlung wieder versprüht.

Anstelle der beschriebenen Direktkühlung mit Wasser, kann das Produktgasgemisch 1 aus der Dehydrierstufe auch zunächst dadurch abgekühlt werden, dass man mit dem Produktgasgemisch 1 in einem indirekten Wärmeaustauscher (z.B. in einem Rohrbündelwärmeaustauscher im Gleichstrom oder im Gegenstrom) das der Dehydrierstufe zuzuführende Reaktionsgasausgangsgemisch 1 aufwärmt (z.B. auf eine Temperatur im Bereich von 350 bis 450˚C). Das Produktgasgemisch 1 der Dehydrierstufe kühlt dabei in entsprechendem Maß von der hohen Austrittstemperatur der Dehydrierstufe (z.B. von 450 bis 550˚C) auf ca. 200 bis 300˚C ab.

Eine weitere Abkühlung des Produktgasgemisches 1 der Dehydrierstufe kann dadurch erfolgen, dass es in einem indirekten Wärmeaustauscher dazu verwendet wird, das Reaktionsgasausgangsgemisch 2 für die nachfolgend noch zu beschreibende Partialoxidation des in der Dehydrierstufe erzeugten Propens aufzuwärmen, und/oder dass es dazu verwendet wird, die im weiteren noch zu beschreibende Absorberabgaskühlung beim, vorzugsweise zweistufigen, Expandieren desselben mittels Expansionsturbinen auszugleichen, oder durch Vorabanwärmung zu kompensieren.

Danach befindet sich das Produktgasgemisch 1 bei einer Temperatur von etwa 180˚C. Anschließend kann mittels Luft- und/oder Oberflächenwasserkühler auf eine Temperatur im Bereich von 30˚C bis 60˚C abgekühlt werden.

In die Kühler integrierte oder diesen nachgeschaltete Tropfenabscheider sammeln das beim Abkühlen auskondensierte Wasser und führen es standgeregelt der Entsorgung zu.

Das so abgekühlte und von Wasserdampf entlastete, bei einem Druck von etwa 2 bar befindliche Produktgasgemisch 1 der Dehydrierstufe wurde nachfolgend auf einen Druck von 10 bis 13 bar verdichtet.

In anwendungstechnisch zweckmäßiger Weise wurde die Verdichtung zweistufig durchgeführt, um zu hohe Verdichtungstemperaturen zu vermeiden (diesem Zweck diente auch schon die vorab durchgeführte Abkühlung; die Wasserdampfabscheidung entlastet die aufzuwendende Verdichterleistung zusätzlich). In der ersten Stufe wurde auf einen Druck von 4 bis 4,5 bar verdichtet. Die Austrittstemperatur des Gasgemisches betrug beim Verlassen des Verdichters etwa 115˚C.

In einem nach geschalteten indirekten Wärmeaustauscher (Luftkühler oder Oberflächenwasserkühler) wurde das Gasgemisch wieder auf 40 bis 60˚C abgekühlt, wobei weitere Wasserdampfkondensation erfolgte. Tropfenabscheider sammelten das Kondensat und schleusten es standgeregelt aus.

In der zweiten Verdichterstufe wurde ausgehend von einem Druck von etwa 4 bar auf einen Enddruck von 10 bar verdichtet (hier kann gegebenenfalls auch auf einen Druck von bis zu 13 bar und mehr verdichtet werden). Die Austrittstemperatur beim Verlassen des Verdichters betrug etwa 126˚C.

In zwei weiteren nachgeschalteten indirekten Wärmeaustauschern (zunächst ein Luftkühler (ist normalerweise ein Rohrbündelwärmetauscher; das zu kühlende Gas durchströmt zweckmäßig das Rohrinnere) und dann ein Oberflächenwasserkühler) wurde das verdichtete Gasgemisch zunächst auf 40 bis 60˚C und dann auf 30˚C abgekühlt. Dabei auskondensierendes Wasser wurde wieder mittels Tropfenabscheidern abgeschieden und herausgeführt. Beim Verlassen des zweiten Verdichters enthält das Gasgemisch nur noch ca. 0,2 Gew.-% Wasser. Der geringe Wassergehalt verringert den Wasseranfall und vermeidet so durch eine Zweiphasigkeit der Flüssigkeit bedingte Betriebsprobleme in der nachfolgenden Absorption und der hohe Druck mindert die zum Zweck der Absorption benötigte Menge an Absorptionsmittel.

Während großtechnisch Turboverdichter (nicht ölgeschmierte, trocken laufende, berührungsfreie Verdichter) zum Zweck der Verdichtung eingesetzt werden (z.B. vom Typ 12 MH 4B, der Fa. Mannesmann DEMAG, DE), wurden hier Membran-Verdichter MV 3459 II der Fa. Sera verwendet. Prinzipiell können die Verdichter sowohl über Elektromotoren als auch über Dampf- oder Gasturbinen angetrieben werden. Häufig ist der Antrieb über Dampfturbinen der wirtschaftlichste. Das wie beschrieben verdichtete und abgekühlte Gasgemisch wurde direkt oberhalb des Sumpfs einer Absorptionskolonne zugeführt (ca. 4650 g/h). Es wies nachfolgende Gehalte auf:

|  | Vol.-% |
|---|---|
| Stickstoff | 61,22 |
| Sauerstoff | 0,25 |
| Propan | 24,23 |
| Propen | 5,07 |
| Methan | 0,02 |
| Ethan | 0,11 |
| Ethen | 0,03 |
| n-Butan | 0,06 |
| iso-Butan | 0,09 |
| n-Butene | 0,04 |
| iso-Buten | 0,10 |
| 1,3-Butadien | 0,00 |
| Wasserstoff | 5,57 |
| kohlenmonoxid | 0,05 |

(fortgesetzt)

|  | Vol.-% |
|---|---|
| Kohlendioxid | 3,15 |

Die Absorptionskolonne bestand aus Edelstahl 1.4571. Der Kolonneninnendurchmesser betrug 80 mm, die Wanddicke 4 mm und die Kolonnenlänge 1,70 m.

Etwa 70 % des Volumens der Absorptionskolonne waren mit Packungselementen der Fa. Montz (Montz BSH-750, spezifische Oberfläche 750 $m^2/m^3$) als trennwirksame Einbauten gefüllt. Die Packungselemente folgten unmittelbar aufeinander und begannen auf der Höhe von 1/5 der Kolonnenlänge von unten. Die Absorptinskolonne war von außen weder gekühlt noch beheizt. Am Kolonnenkopf wurde mit einer Aufgabetemperatur von 30˚C technisches Tetradecan der Fa. Haltermann, DE, vorn Typ PKWF 4/7 af als Absorptionsmittel aufgegeben (gaschromatographische Analyse mittels FiD-Detektion ergab zu Beginn (frisch) nachfolgende GC-Flächen-% Zusammensetzung:

n-Tridecan 7,6 %,
n-Tetradecan 47,3 %,
n-Pentadecan 7,0 %,
n-Hexadecan 3,2 %,
n-Heptadecan 14,1 % und
Restsumme 20,7 %;

diese Zusammensetzung änderte sich im Verlauf (nach ca. 3000 $h^{-1}$) des kontinuierlichen Verfahrensbetriebs auf folgende Werte:

n-Tridecan 2,6 %,
n-Tetradecan 39,5 %,
n-Pentadecan 9,4 %,
n-Hexadecan 4,8 %,
n-Heptadecan 23,4 und
Restsumme 20,3 %).

Die Berieselungsdichte lag bei 15 $m^3$ Absorptionsmittel pro $m^2$ freie Querschnittsfläche und Stunden (= 28 kg/h Tetradecan).

Der aus der Absorptionskolonne als Nebenkomponentenauslaß zur Verbrennung geführte Abgasstrom enthielt noch 950 Vol.-ppm Propan und 250 Vol.-ppm Propen. Großtechnisch wird dieser Abgasstrom über einen Expander (z.B. eine Expansionsturbine) in die Verbrennung geführt, um den Großteil der in der zweistufigen Verdichtung aufgewendeten Verdichtungsleistung rückzugewinnen und in die zweistufige Verdichtung rückzuführen. In zweckmäßiger Weise wird die Expansion auch zweistufig durchgeführt, um unerwünschte Kondensation zu vermeiden. Die bei der Entspannung gewonnene mechanische Energie kann sowohl direkt als Mit- oder Hauptantrieb für einen der Verdichter und/oder zur Erzeugung von Strom genutzt werden.

Bevor das entspannte Absorberabgas zur Verbrennung geführt wird, kann es großtechnisch zweckmäßig sein, den darin enthaltenen Wasserstoff abzutrennen. Dies kann z.B. dadurch erfolgen, dass man das Abgas über eine, in der Regel zu einem Rohr gestaltete, Membran leitet, die lediglich für den molekularen Wasserstoff durchlässig ist. Der so abgetrennte molekulare Wasserstoff kann z.B. in die heterogen katalysierte Dehydrierung rückgeführt oder einer sonstigen Verwertung (z.B. in Brennstoffzellen) zugeführt werden.

Prinzipiell kann die Wasserstoffabtrennung auch durch partielle Kondensation, Adsorption (Druckwechseladsorption) und/oder Rektifikation (vorzugsweise unter Druck) vorgenommen werden. Großtechnisch ist es zu dem in der Regel zweckmäßig, das Absorberabgas durch das im weiteren noch zu beschreibende Sauerwasser zu führen, um dieses einzuengen.

Das Absorbat enthielt folgende Gehalte (Gew.-% bezogen auf das Gewicht des Absorbats):

|  | Gew.-% |
|---|---|
| Stickstoff | 0,147 |
| Propan | 4,58 |
| Propen | 0,915 |

(fortgesetzt)

|  | Gew.-% |
|---|---|
| Ethan | 0,07 |
| n-Butan | 0,015 |
| iso-Butan | 0,023 |
| n-Butene | 0,009 |
| iso-Buten | 0,024 |
| Kohlendioxid | 0,086 |
| Ethen | 0,000 |
| Tetradekan | ca. Rest bis zu 100 Gew.-% |

Bevor das Absorbat zum Kopf der nachfolgenden Desorptionskolonne geführt wurde, wurde es in einem indirekten Wärmeaustauscher auf 60˚C erwärmt.

Anschließend wurde das Absorbat ( dies kann z.B. in einer inversen Pumpe oder mittels eines Ventils ausgeführt werden) auf einen Druck von 2,7 bar entspannt (die im Fall der inversen Pumpe dabei frei gesetzte mechanische Energie wird zweckmäßig zur Rückverdichtung von in der Desorptionskolonne befreitem Absorptionsmittel mitverwendet) und das dabei erzeugte zweiphasige Gemisch am Kopf in die Desorptionskolonne geführt.

In die Desorptionskolonne (Innendurchmesser = 80 mm, Länge = 1,70 m, Wanddicke = 4 mm) wurde von unten im Gegenstrom zum vom Desorptionskolonnenkopf ablaufenden Absorbat mit einem Druck von 3 bar Luft geführt (1269 Nl/h). Die am Kopf aufgegebene Absorbatmenge betrug 33,7 1/h. Das unten aus der Desorptionskolonne herausgeführte, im wesentlichen von desorbierten Komponenten befreite, Absorptionsmittel wurde durch indirekten Wärmeaustausch gekühlt, auf den in der Absorptionskolonne geforderten Druck verdichtet, durch nochmaligen indirekten Wärmeaustausch (großtechnisch zweckmäßig mit Oberflächenwasser) auf 16˚C gekühlt und dann zum Kopf der Absorptionskolonne rückgeführt. Als trennwirksame Einbauten enthielt die Desorptionskolonne strukturierte Blechpackungen der Fa. Montz (Montz BSH-750, spezifische Oberfläche 750 $m^2/m^3$). Um Absorptionsmittel zurückzuhalten wurde der aus der Desorptionskolonne (gegebenenfalls über einen mechanischen Tropfenabscheider) geführte Gasstrom mit Wasser gewaschen. D.h., er wurde durch ein Packungselement der Fa. Montz (Montz BSH-750, spezifische Oberfläche 750 $m^2/m^3$) geführt, auf das Wasser (70 1/h) mit einer Temperatur von 18 ˚C im Gegenstrom aufgegeben wurde. Unterhalb der Packung war ein Fangboden (Kaminboden) angebracht, von dem die wässrige Phase herausgeführt werden konnte. In einem Phasenscheider wurde in eine wässrige Phase und in eine organische Phase getrennt. Die sehr geringe Menge organische Phase wurde mit dem zum Kopf der Absorptionskolonne rückgeführten Absorptionsmitteltrom vereinigt. Die wässrige Phase wurde rückgekühlt und mit Frischwasser ergänzt (um Verdampfungsverluste auszugleichen) wieder auf das Packungselement aufgegeben. Das Waschen erfolgte der Desorptionskolonne aufgesetzt.

Aus dem Waschabschnitt wurde der gewaschene Gasstrom über mechanische Tropfenabscheider (abgeschiedene Flüssigphase wird in die Wäsche rückgeführt) mit den nachfolgenden Gehalten herausgeführt (wird in der Reaktionsstufe 1 der Dehydrierumsatz höher gewählt, kann der nachfolgende Propengehalt auch bei 8 bis 12 Vol.-% liegen; beispielsweise kann der gewaschene Gasstrom auch 15 Vol.-% Propan, 10 Vol.-% Propen und 14,5 Vol.-% $O_2$ aufweisen):

|  | Vol.-% |
|---|---|
| Stickstoff | 46,69 |
| Sauerstoff | 11,84 |
| Propan | 32,53 |
| Propen | 6,81 |
| Ethan | 0,07 |
| n-Butan | 0,08 |
| iso-Butan | 0,12 |
| n-Butene | 0,05 |

(fortgesetzt)

|  | Vol.-% |
|---|---|
| iso-Buten | 0,13 |
| Wasserstoff | 0,07 |
| Kohlenmonoxid | 0,00 |
| Kohlendioxid | 0,61 |
| Wasser | 1,00 |
| Ethen | 0,00 |

Die Temperatur des Gasgemischs wurde durch indirekten Wärmeaustausch auf 250˚C erhöht und das Gasgemisch mit der vorgenannten Zusammensetzung in einer Menge von 2128 Nl/l•h und einem Eingangsdruck von 2,1 bar als neues Reaktionsgasausgangsgemisch 2 in die nachfolgende Partialoxidationsvorrichtung geführt.

C) Gestaltung der zweiten und der dritten Reaktionsstufe (die Partialoxidation)

1. Erster Festbettreaktor für den Schritt der Partialoxidation des Propens (Propylens) zu Acrolein

Verwendetes Wärmeaustauschmittel: Salzschmelze, bestehend aus
53 Gew.-% Kaliumnitrat,
40 Gew.-% Nariumnitrit und
7 Gew.-% Natriumnitrat.

Abmessung des Kontaktrohres: 4200 mm Gesamtlänge,
26 mm Innendurchmesser,
30 mm Außendurchmesser,
2 mm Wandstärke.

Reaktor: Bestand aus einem doppelwandigen Zylinder aus Edelstahl (zylindrisches Führungsrohr, umgeben von einem zylindrischen Außenbehälter). Die Wanddicken betrugen überall 2 bis 5 mm. Der Innendurchmesser des äußeren Zylinders betrug 168 mm. Der Innen- durchmesser des Führungsrohres betrug ca. 60 mm.
Oben und unten war der doppelwandige Zylinder durch einen Deckel be- ziehungsweise Boden abgeschlossen. Das Kontaktrohr war im zylindrischen Behälter durch das zylindrische Füh- rungsrohr geführt so untergebracht, dass es am oberen bzw unteren Ende desselben (abgedichtet) durch den Deckel bzw. Boden jeweils um 250 mm herausgeführt war.
Das Wärmeaustauschmittel war im zylindrischen Behälter eingeschlossen. Um über die gesamte im zylindrischen Behälter befindliche Kontaktrohrlän- ge (3700 mm) möglichst gleichmäßige thermische Randbedingungen an der Außenwand des Kontaktrohres zu gewährleisten, wurde das Wärme- austauschmittel durch Einperlen von Stickstoff im zylindrischen Behälter umgewälzt.
Mittels des aufsteigenden Stickstoffs wurde das Wärmeaustauschmittel im zylindrischen Führungsrohr von unten nach oben befördert, um dann im Zwischenraum zwischen zylindrischem Führungsrohr und zylindrischem Außenbehälter wieder nach unten zu strömen (eine Umwälzung gleicher Güte kann auch durch Umpumpen (z.B. Propellerpumpen) erreicht wer- den). Durch eine auf den Außenmantel aufgebrachte elektrische Heizung könnte die Temperatur des Wärmeaustauschmittels auf das gewünschte Niveau geregelt werden. Im übrigen bestand Luftkühlung.

| Reaktorbeschickung: | Über den Reaktor betrachtet wurden Salzschmelze und Reaktionsgasgemisch 2 im Gegenstrom geführt. Das Re- aktionsgasgemisch 2 trat oben in den Reaktor ein. Es wurde jeweils mit einer Temperatur von 250˚C ins Reakti- onsrohr geführt. |

Die Salzschmelze trat unten mit einer Temperatur $T^{ein}$ = 335˚C in das zylindrische Führungsrohr ein und oben mit einer Temperatur $T^{aus}$ aus dem zylindrischen Führungs- rohr aus. Der Unterschied zwischen $T^{ein}$ und $T^{aus}$ betrug etwa 2˚C. $T^{mittel}$ = ($T^{ein}$ + $T^{aus}$)/2.

| Kontaktrohrbeschickung: (von oben nach unten) | Abschnitt A: 50 cm Länge |

Vorschüttung aus Steatitringen (Steatit C 220 der Fa. Ce- ram.Tec) der Geometrie 7 mm x 7 mm x 4 mm (Außendurchmesser x Länge x Innendurchmesser).

Abschnitt B: 100 cm Länge

Kontaktrohrbeschickung mit einem homogenen Gemisch aus 20 Gew.-% (alternativ 30 Gew.-%) an Steatitringen (Steatit C 220 der Fa. CeramTec) der Geometrie 5 mm x 3 mm x 2 mm (Außendurch- messer x Länge x Innen- durchmesssser) und 80 Gew.-% (alternativ 70 Gew.-%) Vollkatalysator aus Abschnitt C.

Abschnitt C: 170 cm Länge

Katalysatorbeschickung mit ringförmigem (5 mm x 3 mm x 2 mm = Außendurchmesser x Länge x Innendurchmesser) Vollkatalysator gemäß Beispiel 1 der DE-A 10046957.

Abschnitt D: 50 cm Länge

Nachschüttung aus Steatitringen (Steatit C 220 der Fa. CeramTec) der Geometrie 7 mm x 7 mm x 4 mm (Außendurchmesser x Länge x Innendurchmesser).

2. Zwischenkühlung und Sauerstoffzwischeneinspeisung

Das den ersten Festbettreaktor verlassende Produktgasgemisch 2 wurde zum Zweck der Zwischenkühlung (indirekt mittels Luft) durch ein Verbindungsrohr (Länge = 400 mm, Innendurchmesser = 26 mm, Wanddicke = 2 mm, Material = Edelstahl) geführt, das, auf einer Länge von 200 mm zentriert untergebracht, mit einer Inertschüttung aus Steatitkugeln (Steatit der fa. CeramTec) des Durchmessers 5 bis 6 mm beschickt und unmittelbar an das Kontraktrohr des ersten Festbettreaktors angeflanscht war.

Das Gasgemisch trat mit einer Temperatur von mehr als 310˚C in das Verbindungsrohr ein und verließ es mit einer Temperatur von etwa 140˚C. Anschließend wurden dem Gasgemisch als Sauerstoffquelle 269 Nl/h an komprimierter Luft zugemischt.

Das dabei resultierende, an einem statischen Mischer vermischte, Beschickungsgasgemisch wurde mit einer Temperatur von 220˚C dem Festbettreaktor für den Schritt der Partialoxidation des Acroleins zu Acrylsäure zugeführt.

3. Zweiter Festbettreaktor für den Schritt der Partialoxidation des Acroleins zu Acrylsäure

Es wurde ein Festbettreaktor verwendet, der mit jenem für den ersten Schritt baugleich war. Salzschmelze und Reaktionsgasgemisch wurden über den Reaktor betrachtet im Gleichstrom geführt. Die Salzschmelze trat unten ein, das Reaktionsgasgemisch ebenfalls.

Die Kontaktrohrbeschickung (von unten nach oben) war:

Abschnitt A: 20 cm Länge

Vorschüttung aus Steatitringen (Steatit C 220 der Fa. CeramTec) der Geometrie 7 mm x 7 mm x 4 mm (Außendurchmesser x Länge x Innendurchmesser).

Abschnitt B: 100 cm Länge

Katalysatorbeschickung mit einem homogenen Gemisch aus 20 Gew.-% (alternativ 30 Gew.-%) an Steatitringen (Steatit C 220 der Fa. CeramTec) der Geometrie 7 mm x 3 mm x 4 mm

(Außendurchmesser x Länge x Innendurchmesser) und 80 Gew.-% (alternativ 70 Gew.-%) Schalenkatalysator aus Abschnitt C.

Abschnitt C: 200 cm Länge

Katalysatorbeschickung mit ringförmigem

(7 mm x 3 mm x 4 mm = Außerdurchmesser x Länge x Innendurchmesser) Schalenkatalysator gemäß Herstellungsbeispiel 5 der DE-A 10046928 (an dieser Stelle können auch dazu analoge und in entsprechender Weise hergestellte Schalenkatalysatoren eingesetzt werden, deren Aktivmasse jedoch eine Stöchiometrie von $Mo_{12}V_{2,8}W_{1,2}Cu_{2,4}O_x$ oder von $MO_{12}V_{3,5}W_{1,3}Cu_{2,4}O_x$ aufweist).

Abschnitt D: 50 cm Länge

Nachschüttung aus Steatit-Ringen (Steatit C 220 der Fa. CeramTec) der Geometrie 7 mm x 7 mm x 4 mm (Außendurchmesser x Länge x Innendurchmesser).

Der zweite Reaktor wurde mit ca. 3607 g/h an Beschickungsgasgemisch (Reaktionsgasausgangsgemisch 3) belastet. $T^{mittel}$ ist wie für den Reaktor der zweiten Reaktionsstufe definiert und betrug 284˚C.

Der Propenumsatz in der ersten Reaktionsstufe betrug 98,0 mol-% und der Acroleinumsatz in zweiten Reaktionsstufe betrug ca. 99,8 mol-%.

Die Gehalte des den zweiten Festbettreaktor mit einer Temperatur von 281˚C und einem Druck von 1,8 bar verlassenden Produktgasgemisches 3 waren:

|  | Vol.-% |
|---|---|
| Stickstoff | 51,54 |
| Sauerstoff | 2,3 |
| Propan | 29,20 |
| Propen | 0,110 |
| Methan | 0 |
| Ethan | 0,077 |
| n-Butan | 0,101 |
| iso-Butan | 0,236 |
| n-Butene | 0 |
| iso-Buten | 0,001 |
| 1,3-Butadien | 0,009 |
| Wasserstoff | 0,05 |
| Kohlenmonoxid | 0,596 |
| Kohlendioxid | 1,72 |
| Wasser | 8,21 |
| Acrolein | 0,009 |
| Acrylsäure | 5,28 |
| Essigsäure | 0,240 |
| Propionsäure | 0,002 |
| Ameisensäure | 0,019 |
| Formaldehyd | 0,198 |
| Benzaldehyd | 0,005 |
| Maleinsäureanhydrid | 0,047 |
| Methacrolein | 0,020 |
| Methacrylsäure | 0,011 |
| Ethen | 0,032 |

Die in den beiden Reaktionsstufen 2. 3 verwendeten Katalysatoren können auch durch die in den Beispielen der DE-A 10351269 verwendeten Katalysatoren ersetzt werden. Der Katalysator der zweiten Reaktionsstufe kann auch durch die Katalysatoren der Beispiele und Vergleichsbeispiele der DE-A 10344149 ersetzt werden. Der Katalysator der dritten Reaktionsstufe kann auch durch die Katalysatoren der Beispiele der DE-A 10360057 und DE-A 10360058 ersetzt werden. Ferner kann die Partialoxidation als Hochlastverfahren durchgeführt werden, wie es in der DE-A 10313210, der DE-A 10313213, der DE-A 10313212, der DE-A 10313211, der DE-A 10313208, der DE-A 10313209 und in der DE-A 10313214 und dem in diesen Schriften gewürdigten Stand der Technik beschrieben ist.

D) Gestaltung der Trennzone A (Abtrennung des Zielproduktes Acrylsäure aus dem Produktgasgemisch 3 der Partialoxidation)

In einem Direktkühler wurde der heiße Produktgasgemischstrom (das Produktgasgemisch 3) im Gleichstrom (nach Vereinigung mit dem weiter unten beschriebenen beladenen Stripgasstrom) durch Eindüsen einer Quenchflüssigkeit (enthaltend 57,4 Gew.-% Diphenylether, 20,7 Gew.-% Diphenyl, 20 Gew.-% o-Dimethylphthalat und als Restmenge bis zu 100 Gew.-% Phenothiazin (bis zu 1 Gew.-%) sowie Hochsieder (z.B. Acrylsäureoligomere)) der Temperatur 140 bis 150°C, die auch als Absorptionsmittel verwendet wurde, auf ca. 180°C abgekühlt. Im unteren Teil des Direktkühlers wurde die nicht verdampfte Quenchflüssigkeit gesammelt, entnommen und über einen Wärmeaustauscher, in welchem sie wieder auf Vorlauftemperatur gebracht wurde, in den oberen Teil des Quenchgefäßes rückgesprüht. Der Produktgasgemischstrom 3 (vereint mit dem beladenen Stripgas) und die zu Strahlen verdüste Quenchflüssigkeit wurden dem Direktkühler parallel zugeführt. Der abgekühlte Produktgasgemischstrom 3 strömte dann in die nachfolgende Absorptionskolonne. Im Quenchumlauf reicherten sich hochsiedende Nebenprodukte an, die ausgeschleust werden mussten. Aus dem Quenchumlauf wurde deshalb einmal je Tag diskontinuierlich eine Teilmenge von 1 kg Quenchflüssigkeit in einen Behälter abgelassen, der mit Hilfe eines Rotationsverdampfers (8 mbar, 180°C) diskontinuierlich aufgearbeitet wurde. Das dabei gewonnene gereinigte Absorptionsmittelkondensat wurde dem weiter unten beschriebenen Pufferbehälter für den Sumpfablauf aus der Restgas-Edelstahl-Druck-Waschkolonne zugeführt und dabei einmal je Woche die beim Aufarbeiten im Rotationsverdampfer erlittenen Verluste an Absorptionsmittel von rund 2,9 g/h durch Zusatz von frischem Gemisch bestehend aus Diphenylether (21,2 Gew-%), Diphenyl (58,8 Gew.-%) und o-Dimethylphthalat (20 Gew.-%) zum Kondensat ersetzt.

Das abgekühlte Produktgasgemisch 3 wurde oberhalb des Sumpfes und unterhalb der ersten Packung in, die Absorptionskolonne geführt. Die Absorptionskolonne (ohne Sumpf) war 5420 mm lang, hatte einen Innendurchmesser von 50 mm und enthielt auf insgesamt 3,3 m Länge Kuehni (CH) Rhombopak 9M Packungen.

Die Absorptionskolonne war aus Glas gefertigt und wies aus Gründen der Temperierung einen segmentierten Glasdoppelmantel auf. Unmittelbar oberhalb der zweiten Packung, von unten betrachtet, war ein Druckentkopplungsventil installiert (eine einstellbare Drossel), mit dessen Hilfe ein Druckverlust über die Absorptionskolonne (wie er großtechnisch auftreten würde) von 100 bis 300 mbar simuliert werden konnte. Die Absorptionskolonne wurde mit einem Kopfdruck von 1,30 bar und einem Sumpfdruck von 1,40 bar betrieben. Die Sumpftemperatur betrug ca. 113°C. Der Glasdoppelmantel war in fünf aufeinanderfolgende getrennt betriebene Segmente unterteilt, über die der Absorptionskolonne das nachfolgende Temperaturprofil aufgeprägt wurde. Dieser Segmentierung folgte auch die innere Struktur der Absorptionskolonne.

Die Segmente wiesen von unten nach oben die folgenden Temperaturen auf (ihre Thermostatisierung erfolgte jeweils mittels umgepumptem Wasser, bzw. im 1.Segment mit Wärmeträgeröl der entsprechenden Temperatur) und waren wie folgt gestaltet:

1. Segment: 1020 mm Länge (nach oben gerichtet an der Zufuhrstelle des Produktgasgemischs 3 beginnend), 113°C, 0,4 m Rhombopak zwischen der unmittelbar oberhalb des Sumpfes der Absorptionskolonne befindlichen Zufuhrstelle des Produktgasgemischs 3 und der Zufuhrstelle von aus dem Kolonnensumpf kontinuierlich entnommener und in die Kolonne rezierkulierter ( mit ca. 113 °C, in einer Menge von ca. 200 l/h) Sumpfflüssigkeit und 0,6 m Rhombopak oberhalb dieser Aufgabestelle. Der Abstand zwischen beiden Rhombopaks betrug ca. 2 cm.

2. Segment: 1250 mm Länge, 75°C, am unteren Ende befand sich das Druckentkopplungsventil und oberhalb des Druckentkopplungsventils bis zur Zufuhrstelle des Leichtsiederkondensatstroms aus der weiter unten beschriebenen Reindestillation befanden sich 0,5 m Rhombopak.

3. Segment: 950 mm Länge, 60°C, 0,6 m Rhombopak, die unmittelbar oberhalb der Leichtsiederkondensataufgabestelle des 2. Segments beginnen.

4. Segment: 950 mm Länge, 50-55°C, 0,6 m Rhombopak, die unmittelbar unterhalb der Aufgabestelle des im folgenden beschriebenen Absorptionsmittelhauptstroms enden.

**EP 1 765 759 B1**

5. Segment: 1250 mm Länge, 20˚C, 0,6m Rhombopak zwischen dem unmittelbar oberhalb der Absorptionsmittelaufgabe im 4. Segment für den Ablauf des Sauerwassers (im folgenden beschrieben) angebrachten Fangbodens und der Aufgabestelle von rezierkuliertem Sauerwasser am Kopf der Absorptionskolonne.

Vom vorgenannten im 5.Segment befindlichen Fangboden wurde kontinuierlich wasserreiches Kondensat (Sauerwasser) abgezogen (ca. 70,2 l/h). Es wurde über einen Wärmetauscher indirekt , auf 20˚C abgekühlt und überwiegend oberhalb der obersten Rhombopak-Packung wieder in die Absorptionskolonne rückgeführt (70 Liter/h). Der nicht rückgeführte Anteil des entnommenen Sauerwassers (das Reaktionswasser) wurde standgeregelt ausgeschleust und einer Sauerwasserextraktionsstufe zugeführt. In dieser wurde das ausgeschleuste Sauerwasser in einem auf Umgebungstemperatur gehaltenen Glasrührgefäß (1 Liter Volumen, 80 mm Durchmesser) bei Umgebungsdruck mit dem kleineren Teilstrom des ( weiter unten beschriebenen) gering beladenen (mit Acrylsäure und Nebenkomponenten) Absorptionsmittels vereinigt und gemischt. Die resultierende Mischung wurde in einen ebenfalls auf Umgebungstemperatur gehaltenen gläsernen Phasenscheidebehälter (7,5 Liter Volumen, 150 mm Durchmesser) durch freien Überlauf kontinuierlich abgelassen. Dort trennte sich das übergelaufene flüssige Gemisch in zwei flüssige Phasen, wobei kurzkettige saure Nebenkomponenten (z.B. Essigsäure) bevorzugt in die spezifisch leichtere wässrige Phase und Acrylsäure bevorzugt in die spezifisch schwerere organische Phase übergehen. Die organische Phase wird mittels einer zwangsfördernden Membrandosierpumpe mit dem grösseren Teilstrom des gering beladenen Absorptionsmittels vereinigt und dieser Gesamtstrom (ca. 3,0 l/h, 40˚C) wie bereits beschrieben direkt unterhalb des Sauerwasserfangbodens als Absorptionsmittelhauptstrom der Absorptionskolonne zugeführt.

Das die Absorptionskolonne am Kopf verlassende (Haupt)Restgas (T = 20 ˚C, P = 1,30 bar) wies folgende Gehalte auf:

|  | Vol.-% |
|---|---|
| Stickstoff | 59,9 |
| Sauerstoff | 2,67 |
| Propan | 33,93 |
| Propen | 0,128 |
| Methan | 0 |
| Ethan | 0,09 |
| n-Butan | 0,118 |
| iso-Butan | 0,274 |
| n-Butene | 0 |
| iso-Buten | 0,001 |
| Butadiene | 0,010 |
| Wasserstoff, | 0,058 |
| Kohlenmonoxid, | 0,693 |
| Kohlendioxid | 1,97 |
| Acrolein | 0,009 |
| Ethen | 0,037 |
| alle vorstehenden Gehalte sind wasserfrei gerechnet | |
| Wasser | 1,78 (bezogen auf alles) |

Durch indirekten Wärmetausch wurde das (Haupt)Restgas auf 40˚C erwärmt (um unerwünschte Kondensation auszuschließen), mittels eines Membranverdichters (großtechnisch ein mittels Elektromotor angetriebener Turboverdichter, z.B. vom Typ 12MH4B, der Fa. Mannesmann DEMAG, DE) auf 2,70 bar verdichtet und etwa 78 Vol.-% als Haupt-$C_3$-Kreisgas zur Beschickung des ersten Dehydrierreaktors in die Reaktionsstufe 1 rückgeführt.
Etwa 22 Vol.-% des verdichteten (Haupt)Restgases,wurden unter Entspannung auf 1,6 bar abgezweigt und in einer Edelstahl-Druck-Waschkolonne (Werkstoff 1.4571, Innendurchmesser 30 mm, Füllung: 2 m Rhombopak 9M) bei ca. 1,6 bar und ca. 51 ˚C mit dem weiter unten beschriebenen Gesamtstrom an geringst beladenem Absorptionsmittel im Gegenstrom gewaschen (das gerinstg beladene Absorptionsmittel wurde oben aufgegeben und das Gas unten in die Kolonne geführt).

In einer weiteren Edelstahl-Druckkolonne (Werkstoff 1.4571, Innendurchmesser 30 mm, Füllung: 3 m Rhombopak 9M, Doppelmantel mit Öl temperiert = Strippkolonne) wurde dieser gewaschene Gasstrom bei ca. 1,5 bar und ca. 119 bis 125 °C genutzt, um aus dem beladenen Absorptionsmittelstrom, , welcher aus dem Sumpf der Absorptionskolonne kontinuierlich abgezogen wurde (ca. 3,5 l/h, ca. 113°C), leichtsiedende Komponenten auszustrippen (z.B. so wie in der DE-A 10336386 beschrieben). Der dabei resultierende mit Leichtsiedern (leichter flüchtig als Acrylsäure) beladene Gasstrom (beladenes Strippgas) wurde in einer auf 170 °C temperierten Doppelmantelleitung ( Durchmesser 15 mm, Aussenmantel Edelstahlwellschlauch) aufgeheizt und am Eingang des Quenchgefässes mit dem heissen Produktgassgemischstrom 3 vereinigt.

Am unteren Ende der Edelstahl-Druck-Waschkolonne wurde der Absorptionsmittelstrom gering beladen stand geregelt in den bereits erwähnten Pufferbehälter (Glas, 5 Liter Volumen) abgelassen (in diesen Behälter wird, wie bereits beschrieben, auch das im Rotationsverdampfer anfallende Absorptionsmittelkondensat geleitet).

Vom Pufferbehälter wurde mittels einer Membranpumpe ein grösserer Teilstrom von 2,5 l/h an gering beladenem Absorptionsmittel zur Absorptionskolonne geführt und dort wie bereits beschrieben als Bestandteil des Absorptionsmittelhauptstroms unmittelbar unterhalb des Fangbodens für die Sauerwasserausschleusung im Segment 4 aufgegeben. Der kleinere Teilstrom von 460 ml/h an gering beladenem Absorptionsmittel wurde, wie ebenfalls bereits beschrieben, mittels einer weiteren Membranpumpe in das Glasrührgefäß der Sauerwasserextraktionsstufe geleitet.

Aus dem Sumpfumlauf (ca. 100 l/h, Druckluftmembranpumpe) der Strippkolonne wurden ca. 3,5 kg Sumpfflüssigkeit über einen Edelstahl-Gewebefilter und ein Regelventil stand geregelt abgezogen und zum Zweck der Reindestillation der Vakuumdestillationseinheit zugeführt.

Die Vakuumdestillationeinheit bestand aus einer verspiegelten und vakuumisolierten Glaskolonne (Reinkolonne) mit 50 mm Innendurchmesser und 4000 mm Länge. Mittels Zwangsentspannungssumpfumlauf (ca. 250 l/h, Peripheralradkreiselpumpe) wurde eine Sumpftemperatur von 191 °C aufrechterhalten (p = 4 bar). Der Absolutdruck im Sumpf betrug ca. 230 mbar, der Kopfdruck lag bei 100 mbar. Zum Zweck der Polymerisationsinhibierung wurden oberhalb des Sumpfspiegels 52 Nl/h Luft zugeführt..

Zwischen dem Sumpf der Vakuumdestillationskolonne und dem Zufluss des produktbeladenen Stroms aus dem Sumpfumlauf der Strippkolonne in die Vakuumdestillationskolonne waren zunächst 6 Glockenböden (Bodenabstand: 5 cm) und darüber (über dem Zufluss) weitere 15 Glockenböden (Bodenabstand: 5 cm) angebracht, oberhalb deren die Möglichkeit einer Probenahme mittels einer Miniatur-Membrandosierpumpe bestand.

Oberhalb dieser Probenahmestelle waren 10 Siebböden (je Boden 6 äquidistante Löcher mit Durchmesser 6,5 mm) angebracht (Bodenabstand: 5 cm), die sich nach oben bis zu einem Fangboden erstreckten, von dem kontinuierlich ca. 364 g/h gereinigte Acrylsäure als Zielpodukt ausgeschleust und nach Abkühlung in einem Vorratsbehälter gelagert wurden.

Nebenkomponentengehalte des Zielproduktes waren:

| | |
|---|---|
| Acrylsäure (Reinheit, ohne Inhibitor-gehalt) | 99,54 Gew.-% |
| alle nachfolgenden Nebenkomponen-tenanteile sind hier als auf die enthal-tene Acrylsäure bezogene Ge-wichtsmengen angegeben | |
| Essigsäure | 0,186 % |
| Propionsäure | 269 ppm |
| Maleinsäureanhydrid | 406 ppm |
| Formaldehyd | 344 ppm |
| Benzaldehyd | 186 ppm |
| Methacrylsäure | 1597 ppm |
| Wasser | 342 ppm |

Auf den obersten der unterhalb des Zielproduktabzugs befindlichen Siebböden wurde eine weitere Menge (732 ml/h) an vom Fangboden ausgetragener Acrylsäure unter Beibehalt ihrer Entnahmetemperatur von ca. 75 °C in die Destillationskolonne rückgeführt.

Oberhalb des Zielproduktaustrags befanden sich weitere 10 Siebböden (je Boden 6 Löcher mit Durchmesser 5,5 mm, Bodenabstand: 5 cm), die es gestatteten, noch vorhanden Leichtsieder zum Kolonnenkopf hin anzureichern. Oberhalb dieser Siebböden war ein weiterer Fangboden angebracht, um das Leichtsiederkondensat, welches durch durch indirekte Kühlung bedingte Kondensation im Kolonnenkopf (26 ˚C, 100 mbar Absolutdruck) anfällt, aufzufangen. Die Temperatur auf dem Fangboden betrug 73 ˚C. Das Leichtsiederkondensat enthielt:

| | |
|---|---|
| Acrylsäure | 98,42 Gew.-% |
| Essigsäure | 1,02 Gew.-% |
| Wasser | 0,427 Gew.-% |
| Methacrolein | 0,012 Gew.-% |
| Methacrylsäure | 0,021 Gew.-% |
| Diphyl | 0,009 Gew.-% |
| Propionsäure | 0,024 Gew.-% |
| Furan-2-aldehyd | 0,010 Gew.-% |
| Allylacrylat | 0,009 Gew.-% |
| Acrolein | 0,002 Gew.-% |

Von dem vom Fangboden entnommenen Leichtsiederkondensatgesamtstrom wurde ein Hauptstrom von 570 ml/h unterhalb des Leichtsiederkondensatfangbodens als Rücklauf wieder in die Destillationskolonne eingespeist. Der dabei verbleibende Leichtsiederkondensatreststrom von 190 ml/h wurde auf 40˚C abgekühlt und oberhalb des 2.Segments der Acrylsäureabsorptionskolonne selbiger zugeführt. Zur inhibierenden Wandbenetzung wurde im obersten Bereich des Reinkolonnenkopfs ein mit 0,5 Gew.-% Phenothiazin stabilisierter Zielproduktstrom von 51 ml/h mit der Temperatur 25˚C über eine 4-Loch-Vollstrahldüse eingedüst.

Der am Kopf der Reinkolonne mittels einer Membranvakuumpumpe abgezogene Gasstrom bestand in der Hauptsache aus Inertgasen und Leichtsiedern. In einer auf 8 ˚C gekühlten Kühlfalle konnten aus ihm noch 4,6 g/h kondensierbare Leichtsieder- Restkomponenten abgetrennt wurden. Dieses flüssig abgeschiedene Restkomponentenkondensat enthielt:

| | |
|---|---|
| Acrylsäure | 89,65 Gew.-% |
| Essigsäure | 2,45 Gew.-% |
| Wasser | 7,24 Gew.-% |
| Methacrolein | 0,197 Gew.-% |
| Methacrylsäure | 0,029 Gew.-% |
| Diphyl | 0,059 Gew.-% |
| Propionsäure | 0,020 Gew.-% |
| Furan-3-aldehyd | 0,021 Gew.-% |
| Allylacrylat | 0,007 Gew.-% |
| Acrolein | 0,037 Gew.-% |

Der abzüglich des Restkomponentenkondensats verbliebene "Inertgas"strom wies folgende Zusammensetzung auf:

| | Vol.-% |
|---|---|
| Stickstoff | Rest zu 100% |
| Sauerstoff | 1,20 |
| Propan | 18,8 |
| Propen | 0,08 |
| Methan | 0,004 |

(fortgesetzt)

|  | Vol.-% |
| --- | --- |
| Ethan | 0,052 |
| n-Butan | 0,069 |
| iso-Butan | 0,146 |
| n-Butene | 0 |
| iso-Buten | 0,0 |
| Butadiene | 0,010 |
| Kohlenmonoxid | 0,372 |
| Kohlendioxid | 1,88 |
| Ethen | 0,022 |

Er wurde oberhalb des Druckentkopplungsventils und unterhalb der darüber befindlichen Rhomopak-Packung in das 2. Segment der Absorptionskolonne rückgeführt.

Am Sumpf der Reinkolonne wurde standgeregelt das von Acrysäure weitgehend befreite Absorptionsmittel abgezogen und als geringstbeladener Absorptionsmittelgesamtstrom zu der bereits beschriebenen Edelstahl-Druck-Waschkolonneolonne geleitet, wobei die Druckerhöhung der Sumpfumlaufpumpe der Reinkolonne zum Austrag aus dem Vakuum genutzt wurde.

Das geringstbeladene Absorptionsmittel enthielt:

|  | Gew.-% |
| --- | --- |
| Essigsäure | 0,012 |
| Furan-2-aldehyd | 0,0000 |
| Propionsäure | 0,0000 |
| Benzaldehyd | 0,097 |
| Acrylsäure | 0,056 |
| Methacrylsäure | 0,017 |
| Diphyl | 77,5 |
| DMP | 20,0 |
| Benzoesäure | 0,642 |
| Di-Acrylsäure | 0,910 |
| Wasser | 0,0283 |

Zur Minderung von Eduktverlusten sowie zur Erreichung einer hohen Ausbeute wurden alle mit Edukt oder Zielprodukt beladenen Analysengasströme in einem Glasbehälter (0,5 Liter) zusammengeführt und mittels einer Kleinmembranpumpe der ersten Verdichterstufe des Nebenkomponentenauslaß zugeführt und vor dem dortigen Verdichter mit dem abgekühlten Produktgasgemisch 1 der Dehydrierstufe vereinigt und anschließend verdichtet. Bezogen auf umgesetztes Propan wurde so eine Ausbeute an Acrylsäure von 78.9 mol-% erzielt.

Bezogen auf das im Produktgasgemisch 3 enthaltene Propan und Propylen, wurde sowohl für das Propan als auch für das Propylen eine Rückführrate von > 98 mol-% in die erste Reaktionsstufe erzielt. Bei einer Abtrennung der Acrylsäure aus dem Produktgasgemisch 3, bei der man das Produktgasgemisch 3 nach gegebenenfalls erfolgter direkter und/oder indirekter Kühlung in einer trennwirksame Einbauten enthaltenden Kolonne aufsteigend unter Seitenabzug einer rohen Acrylsäure fraktionierend kondensiert und/oder mit Wasser und/oder wässriger Lösung absorbiert, wie es z.B. die WO 2004/035514 und die DE-A 10243625 beispielhaft beschreiben, betragen vorgenannte Rückführraten in der Regel > 99,9 mol-% und mehr. Dies ist ursächlich darauf zurückzuführen, dass wässrige Medien Propan und Propylen im wesentlichen nicht absorbieren und das so z.B. am Kolonnenkopf entweichende Oxidationskreisgas (Restgas) nicht umgesetztes Propan und Propylen im wesentlichen quantitativ enthält. Die Weiterreinigung der entnommenen rohen Acrylsäure erfolgt zweckmäßig mittels Suspensionskristallisation und nachfolgender Waschkolonnenabtrennung des Suspensionskristallisats wie in der Beschreibung dieser Schrift beschrieben.

Die als Zielprodukt gewonnene Roh-Acrylsäure kann wie in der Schrift EP-A 616998 (unter Beachtung der Lehre der EP-A 912486) oder wie in der Schrift DE-A 19606877 (die Mutterlaugenrückführung kann in die Absorptions- und/oder in die Reinkolonne hinein erfolgen) oder wie in der Schrift EP-A 648732 beschrieben kristallisativ oder rektifikativ zu Reinacrylsäure weitergereinigt werden, die dann zur Herstellung von Wasser superabsorbierenden Polymerisaten in an sich bekannter Weise radikalisch polymerisiert werden kann. Sowohl die gewonnene Roh-Acrylsäure als auch die gewonnene Reinacrylsäure eignen sich in hervorragender Weise zu Herstellung von Estern der Acrylsäure, z.B. zur Herstellung von Alkylacrylaten.

Erfindungsgemäß wesentlich ist, dass das erfindungsgemäße Verfahren auch dann geeignet ist, wenn die zweite Reaktionsstufe in einem Rohr(bündel)reaktor durchgeführt wird, und die Kontaktrohrbeschickung in der zweiten Reaktionsstufe von oben nach unten in Strömungsrichtung des Reaktionsgasgemischs 2 nicht wie folgt gestaltet ist:

Abschnitt A: 50 cm lange Vorschüttung aus Steatitringen (Steatit C220 der Fa. CeramTec) der Geometrie 7 mm x 7 mm x 4 mm (Außendurchmesser x Länge x Innendurchmesser).

Abschnitt B: 100 cm lange Katalysatorbeschickung mit homogenem Gemisch aus 30 Gew.-% Steatitringen (Steatit C220 der Fa. CeramTec) der Geo- metrie 5 mm x 3 mm x 2 mm (Außendurchmesser x Länge x Innen- durchmesser) und 70 Gew.-% Vollkatalysator aus Abschnitt C.

Abschnitt C: 170 cm lange Katalysatorbeschickung mit ringförmigem Vollkatalysa- tor (5 mm x 3 mm x 2 mm = Außendurchmesser x Länge x Innen- durchmesser) gemäß Beispiel 1 der DE-A 10046957)

Abschnitt D: 50 cm lange Nachschüttung aus Steatitringen (Steatit C220 der Fa. CeramTec) der Geometrie 7 mm x 7 mm x 4 mm (Außendurchmesser x Länge x Innendurchmesser).

Erfindungsgemäß wesentlich ist ferner, dass das erfindungsgemäße Verfahren auch dann geeignet ist, wenn die zweite Reaktionsstufe in einem Rohr(bündel)reaktor durchgeführt wird, und über den Reaktor betrachtet Salzschmelze und Reaktionsgasgemisch 2 nicht im Gegenstrom geführt werden.

Vorstehendes gilt insbesondere dann, wenn die Kontaktrohrbeschickung nicht wie vorstehend beschrieben gestaltet ist.

Das erfindungsgemäße Verfahren ist auch dann geeignet, wenn zwischen der zweiten und der dritten Reaktionsstufe eine Sauerstoffzwischeneinspeisung vorgenommen wird.

Das erfindungsgemäße Verfahren ist auch dann geeignet, wenn die dritte Reaktionsstufe in einem Rohr(bündel) reaktor durchgeführt wird, und die Kontaktrohrbeschickung von oben nach unten in Strömungsrichtung des Reaktionsgasgemischs 3 nicht wie folgt gestaltet ist:

Abschnitt A: 20 cm lange Vorschüttung aus Steatitringen (Steatit C220 der Fa. CeramTec) der Geometrie 7 mm x 7 mm x 4 mm (Außendurchmesser x Länge x Innendurchmesser)

Abschnitt B: 100 cm lange Katalysatorbeschickung aus homogenem oder hetero- genem Gemisch aus 30 Gew.-% an Steatitringen (Steatit C220 der Fa. CeramTec) der Geometrie 7 mm x 3 mm x 4 mm (Außendurch- messer x Länge x Innendurchmesser) und 70 Gew.-% Schalenkata- lysator aus Abschnitt C.

Abschnitt C: 200 cm lange Katalysatorbeschickung mit ringförmigem (7 mm x 3 mm x 4 mm = Außendurchmesser x Länge x Innendurchmesser) Schalenkatalysator gemäß Herstellungsbeispiel 5 der DE- A 10046928 (oder ein (z.B. entsprechend hergestellter) Schalenkata- lysator mit der Aktivmasse $Mo_{1,2}V_{2,8}W_{1,2}Cu_{2,4}O_x$ oder $Mo_{12}V_{3,5}W_{1,3}Cu_{2,4}O_x$).

Abschnitt D: 50 cm lange Nachschüttung aus Steatitringen (Steatit C220 der Fa. CeramTec) der Geometrie 7 mm x 7 mm x 4 mm (Außendurchmesser x Länge x Innendurchmesser).

Erfindungsgemäß wesentlich ist ferner, dass das erfindungsgemäße Verfahren auch dann geeignet ist, wenn die dritte Reaktionsstufe in einem Rohr(bündel)reaktor durchgeführt wird, und über den Reaktor betrachtet Salzschmelze und Reaktionsgasgemisch 3 nicht im Gleichstrom geführt werden.

Das erfindungsgemäße Verfahren ist auch dann geeignet, wenn die zweite und die dritte Reaktionsstufe in einem Rohr(bündel)reaktor durchgeführt wird, und die Kontaktrohrbeschickung von oben nach unten in Strömungsrichtung der Reaktionsgasgemische in beiden Reaktionsstufen nicht so wie vorstehend beschrieben gestaltet ist.

Das erfindungsgemäße Verfahren ist auch dann geeignet, wenn die zweite Reaktionsstufe und die dritte Reaktionsstufe in Rohrbündelreaktoren durchgeführt wird, in denen der Wärmeträger (die Salzschmelze) mäanderförmig fließt.

Das erfindungsgemäße Verfahren ist auch dann geeignet, wenn in der zweiten Reaktionsstufe weder ein Vollkatalysator gemäß Beispiel 1 der DE-A 10046957, noch ein Katalysator gemäß den Beispielen der DE-A 10351269, noch gemäß den Beispielen oder Vergleichsbeispielen der DE-A 10344149 verwendet wird.

Das erfindungsgemäße Verfahren ist auch dann geeignet, wenn in der dritten Reaktionsstufe weder ein Schalenkatalysator gemäß Herstellungsbeispiel 5 der DE-A 10046928 noch ein (z.B. entsprechend hergestellter) Schalenkatalysator mit einer der Aktivmassen $Mo_{12}V_{2,8}W_{1,2}Cu_{2,4}O_x$, $Mo_{12}V_{3,5}W_{1,3}Cu_{2,4}O_x$, noch ein Katalysator gemäß den Beispielen der DE-A 10351269, DE-A 10360057 sowie der DE-A 10360058 verwendet wird.

Insbesondere ist das erfindungsgemäße Verfahren auch dann geeignet, wenn die Abtrennung der Acrylsäure aus dem Produktgasgemisch 3 nicht mit einem Absorptionsmittel erfolgt, das 57,4 Gew.-% Diphenylether, 20,7 Gew.-% Diphenyl und 20 Gew.-% o-Dimethylphthalat enthält.

Das erfindungsgemäße Verfahren ist auch dann geeignet, wenn es auch keine Kombination der vorstehend negativen Merkmale aufweist (insbesondere keine Kombination aller negativen Merkmale).

**Patentansprüche**

1.  Verfahren zur Herstellung von Acrylsäure durch heterogen katalysierte partielle Gasphasenoxidation von Propylen, bei dem man

    a) in einer ersten Reaktionsstufe Propan im Beisein und/oder unter Ausschluß von Sauerstoff einer homogenen und/oder einer heterogen katalysierten Dehydrierung und/oder Oxidehydrierung unterwirft, wobei ein Propan und Propylen enthaltendes Produktgasgemisch 1 erhalten wird, und

    b) aus in der ersten Reaktionsstufe gebildetem Produktgasgemisch 1, von den darin enthaltenen, von Propan und Propylen verschiedenen Bestandteilen gegebenenfalls eine Teilmenge abtrennt und/oder in andere Verbindungen wandelt, wobei aus dem Produktgasgemisch 1 ein Produktgasgemisch 1' erzeugt wird, und

    c) Produktgasgemisch 1 und/oder Produktgasgemisch 1' als Bestandteil eines Reaktionsgasausgangsgemischs 2, das molekularen Sauerstoff und Propylen in einem molaren Verhältnis $O_2:C_3H_6 \geq 1$ enthält, in einer mit einem Katalysatorfestbett 2, dessen Katalysatoren als Aktivmasse wenigstens ein die Elemente Mo, Fe und Bi enthaltendes Multimetalloxid aufweisen, beschickten zweiten Reaktionsstufe einer heterogen katalysierten partiellen Gasphasenoxidation von im Produktgasgemisch 1 und/oder Produktgasgemisch 1' enthaltenem Propylen zu Acrolein unterwirft, wobei ein Produktgasgemisch 2 erhalten wird, und

    d) die Temperatur des die zweite Reaktionsstufe verlassenden Produktgasgemischs 2 durch indirekte und/oder direkte Kühlung gegebenenfalls verringert und dem Produktgasgemisch 2 gegebenenfalls molekularen Sauerstoff und/oder Inertgas zugibt, und

    e) danach als Acrolein, molekularen Sauerstoff und wenigstens ein Inertgas enthaltendes Reaktionsgasausgangsgemisch 3, das molekularen Sauerstoff und Acrolein in einem molaren Verhältnis $O_2:C_3H_4O \geq 0,5$ enthält, in einer mit einem Katalysatorfestbett 3, dessen Katalysatoren als Aktivmasse wenigstens ein die Elemente Mo und V enthaltendes Multimetalloxid aufweisen, beschickten dritten Reaktionsstufe einer heterogen katalysierten partiellen Gasphasenoxidation von im Reaktionsgasausgangsgemisch 3 enthaltenem Acrolein zu Acrylsäure unterwirft, wobei ein Produktgasgemisch 3 erhalten wird, und

    f) aus dem Produktgasgemisch 3 in einer Trennzone A Acrylsäure abtrennt und wenigstens das im Produktgasgemisch 3 enthaltene nicht umgesetzte Propan und Propylen zu jeweils wenigstens 80 mol-%, bezogen auf die im Produktgasgemisch 3 enthaltene jeweilige Menge, in wenigstens die erste der drei Reaktionsstufen zurückführt, **dadurch gekennzeichnet, dass**

    - der Umsatz $U^P$ des Propylens in der zweiten Reaktionsstufe, bezogen auf einmaligen Durchgang durch selbige, $\leq 99,5$ mol-%, und
    - der Umsatz $U^A$ des Acroleins in der dritten Reaktionsstufe, bezogen auf einmaligen Durchgang durch selbige $\geq 96$ mol-% beträgt, und das Verfahren wenigstens einen Auslass für von Propan und Propen verschiedene Bestandteile aufweist.

2.  Verfahren nach Anspruch 1, **dadurch gekennzeichnet, dass** $U^P \leq 99,0$ mol-%.

3.  Verfahren nach Anspruch 1, **dadurch gekennzeichnet, dass** $U^P \leq 98,5$ mol-%.

**4.** Verfahren nach Anspruch 1, **dadurch gekennzeichnet, dass** $U^P \leq 97{,}5$ mol-%.

**5.** Verfahren nach Anspruch 1, **dadurch gekennzeichnet, dass** $U^P \leq 97{,}0$ mol-%.

**6.** Verfahren nach Anspruch 1, **dadurch gekennzeichnet, dass** $U^P \leq 96{,}5$ mol-%.

**7.** Verfahren nach Anspruch 1, **dadurch gekennzeichnet, dass** $U^P \leq 96{,}0$ mol-%.

**8.** Verfahren nach Anspruch 1, **dadurch gekennzeichnet, dass** $U^P \leq 95{,}5$ mol-%.

**9.** Verfahren nach Anspruch 1, **dadurch gekennzeichnet, dass** $U^P \leq 95{,}0$ mol-%.

**10.** Verfahren nach einem der Ansprüche 1 bis 9, **dadurch gekennzeichnet**, das $U^A \geq 97$ mol-%.

**11.** Verfahren nach einem der Ansprüche 1 bis 9, **dadurch gekennzeichnet, dass** $U^A \geq 98$ mol-%.

**12.** Verfahren nach einem der Ansprüche 1 bis 9, **dadurch gekennzeichnet, dass** $U^A \geq 99$ mol-%.

**13.** Verfahren nach einem der Ansprüche 1 bis 9, **dadurch gekennzeichnet, dass** $U^A \geq 99{,}5$ mol-%.

**14.** Verfahren nach Anspruch 1, **dadurch gekennzeichnet, dass** $U^P$ 80 bis 98 mol-% und $U^A$ 99 bis 99,9 mol-% beträgt.

**15.** Verfahren nach einem der Ansprüche 1 bis 14, **dadurch gekennzeichnet, dass** das im Produktgasgemisch 3 enthaltene nicht umgesetzte Propan und Propylen zu jeweils wenigstens 85 mol-% in wenigstens die erste Reaktionsstufe zurückgeführt wird.

**16.** Verfahren nach einem der Ansprüche 1 bis 14, **dadurch gekennzeichnet, dass** das im Produktgasgemisch 3 enthaltene nicht umgesetzte Propan und Propylen zu jeweils wenigstens 90 mol-% in wenigstens die erste Reaktionsstufe zurückgeführt wird.

**17.** Verfahren nach einem der Ansprüche 1 bis 14, **dadurch gekennzeichnet, dass** das im Produktgasgemisch 3 enthaltene nicht umgesetzte Propan und Propylen zu jeweils wenigstens 95 mol-% in wenigstens die erste Reaktionsstufe zurückgeführt wird.

**18.** Verfahren nach einem der Ansprüche 1 bis 17, **dadurch gekennzeichnet, dass** wenigstens einer der drei Reaktionsstufen Frischpropan zugeführt wird.

**19.** Verfahren nach einem der Ansprüche 1 bis 17, **dadurch gekennzeichnet, dass** der Reaktionsstufe 2, oder der Reaktionsstufe 3 oder beiden Reaktionsstufen Frischpropan zugeführt wird.

**20.** Verfahren nach einem der Ansprüche 1 bis 19, **dadurch gekennzeichnet, dass** das Reaktionsgasausgangsgemisch 2 $\geq 7$ Vol-% Propylen enthält.

**21.** Verfahren nach einem der Ansprüche 1 bis 20, **dadurch gekennzeichnet, dass** das molare Verhältnis $V_1$ von im Reaktionsgasausgangsgemisch 2 enthaltenem Propan zu im Reaktionsgasausgangsgemisch 2 enthaltenem Propylen 1 bis 4 beträgt.

**22.** Verfahren nach Anspruch 21, **dadurch gekennzeichnet, dass** $V_1$ 1 bis 3 beträgt.

**23.** Verfahren nach Anspruch 21, **dadurch gekennzeichnet, dass** $V_1$ 1 bis 2,5 beträgt.

**24.** Verfahren nach einem der Ansprüche 1 bis 23, **dadurch gekennzeichnet, dass** das Reaktionsgasausgangsgemisch 3 $\geq 5{,}5$ Vol.-% Acrolein enthält.

**25.** Verfahren nach einem der Ansprüche 1 bis 24, **dadurch gekennzeichnet, dass** das Reaktionsgasausgangsgemisch 3 $\geq 6$ Vol.-% Acrolein enthält.

**26.** Verfahren nach einem der Ansprüche 1 bis 25, **dadurch gekennzeichnet, dass** das molare Verhältnis $V_5$ von im

Reaktionsgasausgangsgemisch 3 enthaltenem Propan zu in ihm enthaltenem Acrolein 1 bis 4 beträgt.

27. Verfahren nach Anspruch 26, **dadurch gekennzeichnet, dass** $V_5$ 1,5 bis 3,5 beträgt.

28. Verfahren nach Anspruch 26, **dadurch gekennzeichnet, dass** $V_5$ 1,5 bis 3 beträgt.

29. Verfahren nach einem der Ansprüche 1 bis 28, **dadurch gekennzeichnet, dass** das Reaktionsgasausgangsgemisch 3 enthält:

> 6 bis 8 Vol.-% Acrolein,
> 3 bis 9 Vol.-% molekularer Sauerstoff,
> 10 bis 20 Vol.-% Propan,
> 50 bis 20 Vol.-% molekularer Stickstoff und
> 7 bis 13 Vol.-% Wasserdampf.

30. Verfahren nach einem der Ansprüche 1 bis 29, **dadurch gekennzeichnet, dass** die Belastung des Katalysatorfestbetts 2 mit Propylen ≥ 140 Nl/l · h beträgt.

31. Verfahren nach einem der Ansprüche 1 bis 30, **dadurch gekennzeichnet, dass** die Belastung des Katalysatorfestbetts 2 mit Acrolein ≥ 120 Nl/l · h beträgt.

32. Verfahren nach einem der Ansprüche 1 bis 31, **dadurch gekennzeichnet, dass** die Reaktionsstufe 1 eine autotherme heterogen katalysierte Dehydrierung ist.

33. Verfahren nach einem der Ansprüche 1 bis 32, **dadurch gekennzeichnet, dass**, zwischen den Reaktionsstufen 2 und 3 keine Sauerstoffzwischeneinspeisung erfolgt.

34. Verfahren nach einem der Ansprüche 1 bis 33, **dadurch gekennzeichnet, dass** die Reaktionsstufen 2 und 3 in einem gemeinsamen Reaktor durchgeführt werden.

35. Verfahren nach einem der Ansprüche 1 bis 34, **dadurch gekennzeichnet, dass** sich ein Verfahren anschließt, bei dem man das Produktgasgemisch 3 nach gegebenenfalls erfolgter direkter und/oder indirekter Kühlung in einer trennwirksame Einbauten enthaltenden Kolonne aufsteigend unter Seitenabzug einer rohen Acrylsäure fraktionierend kondensiert und/oder mit Wasser und/oder wässriger Lösung absorbiert.

36. Verfahren nach Anspruch 35, **dadurch gekennzeichnet, dass** sich ein Verfahren anschließt, bei dem die rohe Acrylsäure einer Suspensionskristallisation unter Bildung von Acrylsäuresuspensionskristallisat und verbleibender Mutterlauge unterworfen wird.

37. Verfahren nach Anspruch 36, **dadurch gekennzeichnet, dass** sich ein Verfahren anschließt, bei dem das Acrylsäuresuspensionskristallisat mittels einer Waschkolonne von verbliebener Mutterlauge abgetrennt wird.

38. Verfahren nach Anspruch 37, **dadurch gekennzeichnet, dass** die Waschkolonne eine solche mit erzwungenem Transport des Kristallbetts ist.

39. Verfahren nach Anspruch 37 oder 38, **dadurch gekennzeichnet, dass** die Waschkolonne eine hydraulische Waschkolonne ist.

40. Verfahren nach einem der Ansprüche 37 bis 39, **dadurch gekennzeichnet, dass** als Waschflüssigkeit die Schmelze von in der Waschkolonne vorab abgetrennten Acrylsäurekristallen verwendet wird.

41. Verfahren nach einem der Ansprüche 37 bis 40, **dadurch gekennzeichnet, dass** sich ein Verfahren anschließt, bei dem das abgetrennte Acrylsäureauspensionskristallisat aufgeschmolzen und in Polymerisate radikalisch einpolymerisiert wird.

**Claims**

1. A process for preparing acrylic acid by heterogeneously catalyzed partial gas phase oxidation of propylene, by

    a) in a first reaction stage, subjecting propane to a homogeneous and/or a heterogeneously catalyzed dehydrogenation and/or oxydehydrogenation in the presence of and/or with exclusion of oxygen to obtain a product gas mixture 1 comprising propane and propylene, and

    b) optionally removing and/or converting to other compounds a portion of the constituents, other than propane and propylene, present in the product gas mixture 1 formed in the first reaction stage to obtain a product gas mixture 1' from product gas mixture 1, and

    c) subjecting product gas mixture 1 and/or product gas mixture 1', as a constituent of a starting reaction gas mixture 2 which comprises molecular oxygen and propylene in a molar $O_2:C_3H_6$ ratio of $\geq 1$, to a heterogeneously catalyzed partial gas phase oxidation of propylene present in product gas mixture 1 and/or product gas mixture 1' to acrolein in a second reaction stage charged with a fixed catalyst bed 2 whose catalysts have at least one multimetal oxide comprising the elements Mo, Fe and Bi as an active composition to obtain a product gas mixture 2, and

    d) optionally lowering the temperature of the product gas mixture 2 leaving the second reaction stage by indirect and/or direct cooling and optionally adding molecular oxygen and/or inert gas to product gas mixture 2, and

    e) then subjecting it, as a starting reaction gas mixture 3 which comprises acrolein, molecular oxygen and at least one inert gas and comprises molecular oxygen and acrolein in a molar $O_2:C_3H_4O$ ratio of $\geq 0.5$, to a heterogeneously catalyzed partial gas phase oxidation of acrolein present in starting reaction gas mixture 3 to acrylic acid in a third reaction stage charged with a fixed catalyst bed 3 whose catalysts have at least one multimetal oxide comprising the elements Mo and V as an active composition to obtain a product gas mixture 3, and

    f) removing acrylic acid in a separating zone A from product gas mixture 3 and recycling at least the unconverted propane and propylene present in product gas mixture 3 to an extent of in each case at least 80 mol% based on the particular amount present in product gas mixture 3 into at least the first of the three reaction stages, wherein

    - the conversion $C^P$ of propylene in the second reaction stage, based on single pass through it, ist $\leq 99.5$ mol%, and
    - the conversion $C^A$ of acrolein in the third reaction stage, based on single pass through it, is $\geq 96$ mol%, and the process has at least one discharge for constituents other than propane and propene.

2. The process according to claim 1, wherein $C^P$ is $\leq 99.0$ mol%.

3. The process according to claim 1, wherein $C^P$ is $\leq 98.5$ mol%.

4. The process according to claim 1, wherein $C^P$ is $\leq 97.5$ mol%.

5. The process according to claim 1, wherein $C^P$ is $\leq 97.0$ mol %.

6. The process according to claim 1, wherein $C^P \leq 96.5$ mol %.

7. The process according to claim 1, wherein $C^P$ is $\leq 96.0$ mol%.

8. The process according to claim 1, wherein $C^P$ is $\leq 95.5$ mol%.

9. The process according to claim 1, wherein $C^P$ is $\leq 95.0$ mol%.

10. The process according to any of claims 1 to 9, wherein $C^A$ is $\geq 97$ mol%.

11. The process according to any of claims 1 to 9, wherein $C^A$ is $\geq 98$ mol%.

12. The process according to any of claims 1 to 9, wherein $C^A$ is $\geq 99$ mol%.

13. The process according to any of claims 1 to 9, wherein $C^A$ is $\geq 99.5$ mol %.

14. The process according to claim 1, wherein $C^p$ is from 80 to 98 mol% and $C^A$ is from 99 to 99.9 mol%.

**15.** The process according to any of claims 1 to 14, wherein the unconverted propane and propylene present in product gas mixture 3 are recycled to an extent of in each case at least 85 mol% into at least the first reaction stage.

**16.** The process according to any of claims 1 to 14, wherein the unconverted propane and propylene present in product gas mixture 3 are recycled to an extent of in each case at least 90 mol% into at least the first reaction stage.

**17.** The process according to any of claims 1 to 14, wherein the unconverted propane and propylene present in product gas mixture 3 are recycled to an extent of in each case at least 95 mol% into at least the first reaction stage.

**18.** The process according to any of claims 1 to 17, wherein fresh propane is fed to at least one of the three reaction stages.

**19.** The process according to any of claims 1 to 17, wherein fresh propane is fed to reaction stage 2, or to reaction stage 3, or to both reaction stages.

**20.** The process according to any of claims 1 to 19, wherein starting reaction gas mixture 2 comprises $\geq 7\%$ by volume of propylene.

**21.** The process according to any of claims 1 to 20, wherein the molar ratio $V_1$ of propane present in starting reaction gas mixture 2 to propylene present in starting reaction gas mixture 2 is from 1 to 4.

**22.** The process according to claim 21, wherein $V_1$ is from 1 to 3.

**23.** The process according to claim 21, wherein $V_1$ is from 1 to 2.5.

**24.** The process according to any of claims 1 to 23, wherein starting reaction gas mixture 3 comprises $\geq 5.5\%$ by volume of acrolein.

**25.** The process according to any of claims 1 to 24, wherein starting reaction gas mixture 3 comprises $\geq 6\%$ by volume of acrolein.

**26.** The process according to any of claims 1 to 25, wherein the molar ratio $V_5$ of propane present in starting reaction gas mixture 3 to acrolein present therein is from 1 to 4.

**27.** The process according to claim 26, wherein $V_5$ is from 1.5 to 3.5.

**28.** The process according to claim 26, wherein $V_5$ is from 1.5 to 3.

**29.** The process according to any of claims 1 to 28, wherein starting reaction gas mixture 3 comprises:

from 6 to 8% by volume of acrolein,
from 3 to 9% by volume of molecular oxygen,
from 10 to 20% by volume of propane,
from 50 to 20% by volume of molecular nitrogen and
from 7 to 13% by volume of steam.

**30.** The process according to any of claims 1 to 29, wherein the hourly space velocity of propylene on fixed catalyst bed 2 is $\geq 140$ 1 (STP)/l · h.

**31.** The process according to any of claims 1 to 30, wherein the hourly space velocity of acrolein on fixed catalyst bed 2 is $\geq 120$ 1 (STP)/l · h.

**32.** The process according to any of claims 1 to 31, wherein reaction stage 1 is an autothermal heterogeneously catalyzed dehydrogenation.

**33.** The process according to any of claims 1 to 32, wherein there is no intermediate oxygen feeding between reaction stages 2 and 3.

**34.** The process according to any of claims 1 to 33, wherein reaction stages 2 and 3 are carried out in a combined reactor.

35. The process according to any of claims 1 to 34, which is followed by a process in which product gas mixture 3, after optional direct and/or indirect cooling, is fractionally condensed rising in a column comprising separating internals with side draw removal of crude acrylic acid and/or absorbed with water and/or aqueous solution.

36. The process according to claim 35, which is followed by a process in which the crude acrylic acid is subjected to a suspension crystallization to form acrylic acid suspension crystals and remaining mother liquor.

37. The process according to claim 36, which is followed by a process in which the acrylic acid suspension crystals are removed from remaining mother liquor by means of a wash column.

38. The process according to claim 37, wherein the wash column is one with forced transport of the crystal bed.

39. The process according to claim 37 or 38, wherein the wash column is a hydraulic wash column.

40. The process according to any of claims 37 to 39, wherein the washing liquid used is the melt of acrylic acid crystals which have been removed beforehand in the wash column.

41. The process according to any of claims 37 to 40, which is followed by a process in which the removed acrylic acid suspension crystals are melted and free-radically polymerized into polymers.

**Revendications**

1. Procédé de fabrication d'acide acrylique par oxydation partielle en phase gazeuse sous catalyse hétérogène de propylène, selon lequel

a) lors d'une première étape de réaction, du propane est soumis à une déshydrogénation et/ou oxydéshydrogénation sous catalyse homogène et/ou hétérogène en présence d'oxygène et/ou en éliminant l'oxygène, un mélange gazeux de produits 1 contenant du propane et du propylène étant obtenu, et

b) une partie des constituants différents du propane et du propylène contenus dans le mélange gazeux de produits 1 formé lors de la première étape de réaction est éventuellement séparée et/ou transformée en autres composés, un mélange gazeux de produits l'étant formé à partir du mélange gazeux de produits 1, et

c) le mélange gazeux de produits 1 et/ou le mélange gazeux de produits l'est soumis en tant que constituant d'un mélange réactionnel gazeux initial 2, qui contient de l'oxygène moléculaire et du propylène en un rapport molaire $O_2:C_3H_6 \geq 1$, à une oxydation partielle en phase gazeuse sous catalyse hétérogène du propylène contenu dans le mélange gazeux de produits 1 et/ou le mélange gazeux de produits l'en acroléine dans une deuxième étape de réaction chargée avec un lit catalytique fixe 2, dont les catalyseurs comportent en tant que masse active au moins un oxyde de plusieurs métaux contenant les éléments Mo, Fe et Bi, un mélange gazeux de produits 2 étant obtenu, et

d) la température du mélange gazeux de produits 2 quittant la deuxième étape de réaction est éventuellement réduite par refroidissement indirect et/ou direct et de l'oxygène moléculaire et/ou un gaz inerte est éventuellement ajouté au mélange gazeux de produits 2, puis

e) en tant que mélange réactionnel gazeux initial 3 contenant de l'acroléine, de l'oxygène moléculaire et au moins un gaz inerte, qui contient de l'oxygène moléculaire et de l'acroléine en un rapport molaire $O2:C_3H_4O \geq 0,5$, il est soumis à une oxydation partielle en phase gazeuse sous catalyse hétérogène de l'acroléine contenue dans le mélange réactionnel gazeux initial 3 en acide acrylique dans une troisième étape de réaction chargée avec un lit catalytique fixe 3, dont les catalyseurs comportent en tant que masse active au moins un oxyde de plusieurs métaux contenant les éléments Mo et V, un mélange gazeux de produits 3 étant obtenu, et

f) l'acide acrylique est séparé du mélange gazeux de produits 3 dans une zone de séparation A et au moins le propane et le propylène non réagis contenus dans le mélange gazeux de produits 3 sont recyclés au moins dans la première des trois étapes de réaction, chacun à hauteur d'au moins 80 % en moles, par rapport à la quantité respective contenue dans le mélange gazeux de produits 3, **caractérisé en ce que**

- la conversion $U^P$ du propylène dans la deuxième étape de réaction, par rapport à un passage unique dans celle-ci, est $\leq 99,5$ % en moles et
- la conversion $U^A$ de l'acroléine dans la troisième étape de réaction, par rapport à un passage unique dans celle-ci, est $\geq 96$ % en moles, et le procédé comprend au moins une sortie pour les constituants différents du propane et du propène.

**2.** Procédé selon la revendication 1, **caractérisé en ce que** $U^P \leq 99{,}0$ % en moles.

**3.** Procédé selon la revendication 1, **caractérisé en ce que** $U^P \leq 98{,}5$ % en moles.

**4.** Procédé selon la revendication 1, **caractérisé en ce que** $U^p \leq 97{,}5$ % en moles.

**5.** Procédé selon la revendication 1, **caractérisé en ce que** $U^p \leq 97{,}0$ % en moles.

**6.** Procédé selon la revendication 1, **caractérisé en ce que** $U^P \leq 96{,}5$ % en moles.

**7.** Procédé selon la revendication 1, **caractérisé en ce que** $U^P \leq 96{,}0$ % en moles.

**8.** Procédé selon la revendication 1, **caractérisé en ce que** $U^P \leq 95{,}5$ % en moles.

**9.** Procédé selon la revendication 1, **caractérisé en ce que** $U^P \leq 95{,}0$ % en moles.

**10.** Procédé selon l'une quelconque des revendications 1 à 9, **caractérisé en ce que** $U^A \geq 97$ % en moles.

**11.** Procédé selon l'une quelconque des revendications 1 à 9, **caractérisé en ce que** $U^A \geq 98$ % en moles.

**12.** Procédé selon l'une quelconque des revendications 1 à 9, **caractérisé en ce que** $U^A \geq 99$ % en moles.

**13.** Procédé selon l'une quelconque des revendications 1 à 9, **caractérisé en ce que** $U^A \geq 99{,}5$ % en moles.

**14.** Procédé selon la revendication 1, **caractérisé en ce que** $U^P$ est de 80 à 98 % en moles et $U^A$ est de 99 à 99,9 % en moles.

**15.** Procédé selon l'une quelconque des revendications 1 à 14, **caractérisé en ce que** le propane et le propylène non réagis contenus dans le mélange gazeux de produits 3 sont recyclés chacun à hauteur d'au moins 85 % en moles au moins dans la première étape de réaction.

**16.** Procédé selon l'une quelconque des revendications 1 à 14, **caractérisé en ce que** le propane et le propylène non réagis contenus dans le mélange gazeux de produits 3 sont recyclés chacun à hauteur d'au moins 90 % en moles au moins dans la première étape de réaction.

**17.** Procédé selon l'une quelconque des revendications 1 à 14, **caractérisé en ce que** le propane et le propylène non réagis contenus dans le mélange gazeux de produits 3 sont recyclés chacun à hauteur d'au moins 95 % en moles au moins dans la première étape de réaction.

**18.** Procédé selon l'une quelconque des revendications 1 à 17, **caractérisé en ce que** du propane frais est introduit dans au moins une des trois étapes de réaction.

**19.** Procédé selon l'une quelconque des revendications 1 à 17, **caractérisé en ce que** du propane frais est introduit dans l'étape de réaction 2 ou dans l'étape de réaction 3 ou dans les deux étapes de réaction.

**20.** Procédé selon l'une quelconque des revendications 1 à 19, **caractérisé en ce que** le mélange réactionnel gazeux initial 2 contient $\geq 7$ % en volume de propylène.

**21.** Procédé selon l'une quelconque des revendications 1 à 20, **caractérisé en ce que** le rapport molaire $V_1$ entre le propane contenu dans le mélange réactionnel gazeux initial 2 et le propylène contenu dans le mélange réactionnel gazeux initial 2 est de 1 à 4.

**22.** Procédé selon la revendication 21, **caractérisé en ce que** $V_1$ est de 1 à 3.

**23.** Procédé selon la revendication 21, **caractérisé en ce que** $V_1$ est de 1 à 2,5.

**24.** Procédé selon l'une quelconque des revendications 1 à 23, **caractérisé en ce que** le mélange réactionnel gazeux initial 3 contient $\geq 5{,}5$ % en volume d'acroléine.

**25.** Procédé selon l'une quelconque des revendications 1 à 24, **caractérisé en ce que** le mélange réactionnel gazeux initial 3 contient ≥ 6 % en volume d'acroléine.

**26.** Procédé selon l'une quelconque des revendications 1 à 25, **caractérisé en ce que** le rapport molaire $V_5$ entre le propane contenu dans le mélange réactionnel gazeux initial 3 et l'acroléine contenue dans celui-ci est de 1 à 4.

**27.** Procédé selon la revendication 26, **caractérisé en ce que** $V_5$ est de 1,5 à 3,5.

**28.** Procédé selon la revendication 26, **caractérisé en ce que** $V_5$ est de 1,5 à 3.

**29.** Procédé selon l'une quelconque des revendications 1 à 28, **caractérisé en ce que** le mélange réactionnel gazeux initial 3 contient :

> 6 à 8 % en volume d'acroléine,
> 3 à 9 % en volume d'oxygène moléculaire,
> 10 à 20 % en volume de propane,
> 50 à 20 % en volume d'azote moléculaire et
> 7 à 13 % en volume de vapeur d'eau.

**30.** Procédé selon l'une quelconque des revendications 1 à 29, **caractérisé en ce que** le chargement du lit catalytique fixe 2 avec le propylène est ≥ 140 Nl/l·h.

**31.** Procédé selon l'une quelconque des revendications 1 à 30, **caractérisé en ce que** le chargement du lit catalytique fixe 2 avec l'acroléine est ≥ 120 Nl/l·h.

**32.** Procédé selon l'une quelconque des revendications 1 à 31, **caractérisé en ce que** l'étape de réaction 1 est une déshydrogénation autotherme sous catalyse hétérogène.

**33.** Procédé selon l'une quelconque des revendications 1 à 32, **caractérisé en ce qu'**aucune introduction intermédiaire d'oxygène n'a lieu entre les étapes de réaction 2 et 3.

**34.** Procédé selon l'une quelconque des revendications 1 à 33, **caractérisé en ce que** les étapes de réaction 2 et 3 sont réalisées dans un réacteur commun.

**35.** Procédé selon l'une quelconque des revendications 1 à 34, **caractérisé en ce qu'**un procédé s'ensuit, selon lequel le mélange gazeux de produits 3, après un éventuel refroidissement direct et/ou indirect, est soumis à une condensation fractionnée dans une colonne contenant des composants de séparation de manière ascendante avec sortie latérale d'un acide acrylique brut et/ou absorbé avec de l'eau et/ou une solution aqueuse.

**36.** Procédé selon la revendication 35, **caractérisé en ce qu'**un procédé s'ensuit, selon lequel l'acide acrylique brut est soumis à une cristallisation en suspension avec formation d'un cristallisat de la suspension d'acide acrylique et d'une liqueur mère restante.

**37.** Procédé selon la revendication 36, **caractérisé en ce qu'**un procédé s'ensuit, selon lequel le cristallisat de la suspension d'acide acrylique est séparé de la liqueur mère restante au moyen d'une colonne de lavage.

**38.** Procédé selon la revendication 37, **caractérisé en ce que** la colonne de lavage est une colonne de lavage à transport forcé du lit de cristaux.

**39.** Procédé selon la revendication 37 ou 38, **caractérisé en ce que** la colonne de lavage est une colonne de lavage hydraulique.

**40.** Procédé selon l'une quelconque des revendications 37 à 39, **caractérisé en ce que** la masse fondue des cristaux d'acide acrylique séparés auparavant dans la colonne de lavage est utilisée en tant que liquide de lavage.

**41.** Procédé selon l'une quelconque des revendications 37 à 40, **caractérisé en ce qu'**un procédé s'ensuit, selon lequel le cristallisat de la suspension d'acide acrylique séparé est fondu et polymérisé par voie radicalaire en polymères.

**EP 1 765 759 B1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 990636 A
- US 5198578 A
- EP 1015410 A
- EP 1484303 A
- EP 1484308 A
- EP 1484309 A
- US 20040242826 A
- EP 1388533 A
- EP 1388532 A
- DE 10235847 A
- EP 792867 A
- WO 9801415 A
- EP 1015411 A
- WO 9950219 A
- WO 0053560 A
- WO 0209839 A
- WO 03041833 A
- DE 10223058 A
- DE 10243625 A
- DE 10336386 A
- EP 854129 A
- US 4317926 A
- DE 19837520 A
- DE 19606877 A
- DE 190501325 A
- DE 10247240 A
- DE 19740253 A
- EP 695736 A
- EP 982287 A
- EP 1041062 A
- EP 117146 A
- DE 4308087 A
- DE 4335172 A
- DE 4436243 A
- DE 19924532 A
- DE 10332758 A
- DE 19924533 A
- EP 982289 A
- DE 10115277 A
- DE 19740252 A
- DE 19627847 A
- EP 920408 A
- EP 1068174 A
- EP 1066239 A
- EP 1066240 A
- WO 0053561 A
- DE 10053086 A
- EP 982288 A

- WO 2004063138 A
- WO 2004035514 A
- WO 0177056 A
- WO 03041832 A
- WO 02055469 A
- WO 03078378 A
- EP 1180508 A
- DE 10313209 A1
- EP 925272 A
- DE 10131297 A
- WO 03029177 A
- DE 102004021764 A
- DE 102004021706 A
- DE 10351269 A
- DE 102004025445 A
- WO 04085369 A
- DE 10245585 A
- WO 0196270 A
- DE 102004032129 A
- WO 03076370 A
- WO 0196271 A
- WO 030118804 A
- US 3161670 A
- DE 3313573 A
- WO 04031106 A
- DE 10316039 A
- DE 19508558 A
- WO 0311804 A
- DE 19837519 A
- DE 19837517 A
- WO 9736849 A
- EP 1106598 A
- EP 274681 A
- DE 1020040217063 A
- DE 19937107 A
- DE 19837518 A
- DE 10246119 A
- US 3798283 A
- CN 1105352 A
- DE 19622331 A
- US 4788371 A
- CN 1073893 A
- DE 19753817 A
- US 3862256 A
- US 3887631 A
- DE 19530454 A
- US 4341664 A
- US 5086032 A

- US 4255284 A
- EP 938463 A
- EP 167109 A
- DE 19838312 A
- EP 0700893 A
- EP 0700714 A
- WO 03011804 A
- DE 10219879 A
- EP 731077 A
- DE 10211275 A
- WO 9946039 A
- EP 0705136 A
- WO 9929420 A
- US 4220091 A
- US 5430220 A
- US 5877369 A
- EP 0117146 A
- DE 19937196 A
- DE 19937105 A
- WO 0251547 A
- WO 0251540 A
- DE 102005002127 A
- US 4886928 A
- US 5430209 A
- US 5530171 A
- US 5527979 A
- US 5563314 A
- DE 10235419 A
- WO 04007405 A
- WO 0196170 A
- EP 253409 A
- DE 4431957 A
- DE 4431949 A
- DE 10325488 A
- DE 10325487 A
- DE 10353954 A
- DE 10344149 A
- DE 10350812 A
- DE 10350822 A
- DE 19955176 A
- DE 19948523 A
- DE 10101695 A
- DE 19948248 A
- DE 19955168 A
- EP 700714 A
- DE 10046957 A
- DE 10063162 A
- DE 3338380 C
- DE 19902562 A
- EP 15565 A
- DE 2380765 C
- EP 807465 A
- EP 279374 A
- DE 3300044 A
- EP 575897 A
- US 4438217 A
- DE 19855913 A
- WO 9824746 A
- DE 19746210 A
- JP 3294239 A
- EP 293224 A
- DE 4023239 A
- DE 2909671 A
- EP 293859 A
- EP 714700 A
- DE 10046928 A
- DE 19815281 A
- DE 4335973 A
- EP 668104 A
- DE 19736105 A
- DE 19740493 A
- DE 19528646 A
- EP 468290 A
- DE 19910506 A
- EP 1159244 A
- WO 04085363 A
- WO 04085362 A
- DE 19910508
- EP 911313 A
- EP 979813 A
- DE 2830765 A
- DE 19910508 A
- EP 1159246 A
- WO 04085365 A
- WO 04085370 A
- WO 0408536 A
- EP 1159248 A
- WO 04085367 A
- DE 19948523
- DE 19910506
- DE 19948241
- DE 2830765 C
- DE 2513405 C
- US 3147084 A
- DE 2201528 A
- EP 383224 A
- DE 2903218 A
- EP 700893 A
- EP 468290 B
- DE S2201528 A
- EP 382098 A
- WO 0136364 A
- DE 10261186 A
- EP 0214187 W
- EP 0214188 W
- EP 0214189 W
- EP 873783 A
- EP 1270065 A
- DE 4442346 A
- DE 10028582 A
- DE 10360057 A
- DE 10360058 A
- DE 10313210 A
- DE 10313213 A

- DE 10313212 A
- DE 10313211 A
- DE 10313208 A
- DE 10313209 A

- DE 10313214 A
- EP 616998 A
- EP 912486 A
- EP 648732 A

**In der Beschreibung aufgeführte Nicht-Patentliteratur**

- *Applied Catalysis,* 1991, vol. 70 (2), 175-187
- *Catalysis Today,* 1992, vol. 13, 673-678
- *Catalysis Letters,* 1994, vol. 23, 103-106
- **Zhang.** *Gaodeng Xuexiao Huaxue Xuebao,* 1993, vol. 14, 566
- **Z. Huang.** *Shiyou Huagong,* 1992, vol. 21, 592
- *J. of Catalysis,* 1997, vol. 167, 560-569
- *J. of Catalysis,* 1997, vol. 167, 550-559
- *Topics in Catalysis,* 1996, vol. 3, 265-275

- *Catalysis Letters,* 1991, vol. 10, 181-192
- *Ind. Eng. Chem. Res.,* 1996, vol. 35, 14-18
- *Applied Catalysis A: General,* 1993, vol. 100, 111-130
- *J. of Catalysis,* 1994, vol. 148, 56-67
- New Developments in Selective Oxidation II. Elsevier Science B.V, 1994, 305-313
- 3rd World Congress on Oxidation Catalysis. Elsevier Science B.V, 1997, 375ff